# EUROPEAN PATENT APPLICATION

(11) **EP 1 717 320 A1**
(43) Date of publication of application: **02.11.2006**
(21) Application number: 05103409.8
(22) Date of filing: 26.04.2005
(51) Int. Cl.: C12Q 1/68

(54) **Identification of human gene sequences of cancer antigens expressed in metastatic carcinoma and involved in metastasis formation, and their use in cancer diagnosis, prognosis and therapy**

(71) Applicant: TopoTarget Germany AG, 60596 Frankfurt am Main (DE)
(72) Inventor: Mink, Sigrun, Dr., 76135 Karlsruhe (DE); Mengwasser, Jörg, Dr., 10117 Berlin (DE); Martin, Elke, Dr., 76187 Karlsruhe (DE); Simgen, Birgit, Dr., 76185 Karlsruhe (DE); Raab, Monika, Dr., 63549 Ronneburg (DE); Schwarz, Sylvia, Dr., 66994 Dahn (DE); Hentsch, Bernd, Dr., 60322 Frankfurt am Main (DE)
(74) Representative: Kalhammer, Georg

(57) **Abstract**

The present invention relates to methods using newly identified cancer related polynucleotides and the polypeptides encoded by these polynucleotides. The invention further relates to the use of such "cancer antigens" for diagnosing cancer and cancer metastases. The invention relates to the use of these cancer antigens employing expression vectors, host cells, antibodies directed to such cancer antigens, and recombinant methods and synthetic methods for producing the same. Also provided are diagnostic and prognostic methods for detecting, treating, or preventing cancer, for suppressing tumor progression and minimal residual tumor disease, and therapeutic methods for treating such disorders. The invention further relates to screening methods for identifying agonists and antagonists of the cancer antigens of the invention. The present invention further relates to inhibiting the production and function of the polynucleotides and polypeptides of the present invention.

SEQUENCE LISTING

<110> G2M Cancer Drugs AG

<120> Identification of human gene sequences of cancer antigens
expressed in metastatic carcinoma and involved in metastasis
formation, and their use in cancer diagnosis, prognosis and
therapy

<130> G2M AG

<160> 18

<170> PatentIn version 3.2

<210> 1
<211> 2428
<212> DNA
<213> Homo sapiens

<400> 1
cgtccagttt gagtctaggt tggagttgga accgtggaga tgcggaagga aaccccaccc 60

cccctagtgc ccccggcggc ccgggagtgg aatcttcccc caaatgcgcc cgcctgcatg 120

gaacggcagt tggaggctgc gcggtaccgg tccgatgggg cgcttctcct cggggcctcc 180

agcctgagtg ggcgctgctg ggccggctcc ctctggcttt ttaaggaccc ctgtgccgcc 240

cccaacgaag gcttctgctc cgccggagtc caaacggagg ctggagtggc tgacctcact 300

tgggttgggg agagaggtat tctagtggcc tccgattcag gtgctgttga attgtgggaa 360

ctagatgaga atgagacact tattgtcagc aagttctgca agtatgagca tgatgacatt 420

gtgtctacag tcagtgtctt gagctctggc acacaagctg tcagtggtag caaagacatc 480

tgcatcaagg tttgggacct tgctcagcag gtggtactga gttcataccg agctcatgct 540

gctcaggtca cttgtgttgc tgcctctcct cacaaggact ctgtgtttct ttcatgcagc 600

gaggacaata gaattttact ctgggatacc cgctgtccca agccagcatc acagattggc 660

tgcagtgcgc ctggctacct tcctacctcg ctggcttggc atcctcagca aagtgaagtc 720

tttgtctttg gtgatgagaa tgggacagtc tcccttgtgg acaccaagag tacaagctgt 780

gtcctgagct cagctgtaca ctcccagtgt gtcactgggc tggtgttctc cccacacagt 840

gttcccttcc tggcctctct cagtgaagac tgctcacttg ctgtgctgga ctcaagcctt 900

tctgagttgt ttagaagcca agcccacaga gactttgtga gagatgcgac ttggtccccg 960

ctcaatcact ccctgcttac cacagtgggc tgggaccatc aggtcgtcca ccacgttgtg 1020

cccacagaac ctctcccagc ccctggacct gcaagtgtta ctgagtagat tggatttaag 1080

acaaaaagca agtcccccat gagtgtccac ttctttgccc tgccctctca gcttgtgaga 1140

caacacagga gccttctata gtatgttgat atgctagatc tgtgccgtta ataggcatcg 1200

tctctcagcc tgagggaggc tggattctgg gttcctgtag tcacagggag gaaaagcttt 1260

cttaaaaatg gacatgtatg tgcgtgtgag tgtgtgtgta gatttatagt ttttggtagt 1320

ggcaggaata aaaaaaatcc atcctacatc ttccctaagc actgcctctc tctcaccccc 1380

caaaacaagt tgacgaaagg gttttatgta gctgtctatg aggaattggc cgtgtctggg 1440

tgggttatgg gatgtgggca tccctgggtt cttggaagca gctcttatgc tactcataga 1500
gatgggattg actttatttt tttatagtgc ttaattcacc attatgagaa atgcttccag 1560
tcacaaaaat gcagcccagc tcactctgag gaagaagcag gacttggtac ggttttacac 1620
aactccttac cattaaactg aatcagaaat ccattttctg gctgaataaa aagtttggct 1680
tgcctgtgta atgcccactc ccttccccct ggctccctag tgatgggaca tatatgagag 1740
agaagtgttt ttctatcata gacaccatag ggaaagtttt ggggatgaag gagagcttaa 1800
aggtgtttca attaagttag aaaactgaca caggctgttg agaattcttt gccacttttc 1860
ccaccccaaa acagcatggg gcctgacatc ttctgccctg gtcccctttc tcttgatgtg 1920
gaaagtctga atgcagtatt tatagacttc taaggtttta aaatccagta tcaagaagaa 1980
aatcagaaat actggttggt gaaataaaga gtttaggcat tgttggcctg tcttttttga 2040
agcatgtgtg ttatgtgtag ttagatatat ttcacttatg tgagtcatca tggtgttggt 2100
cttgtagccc attatttttc ctgtgcttcc ccagcttccc aaagtagcta gttagaactt 2160
aaggtaaata tttattcttg ggttggtgga gtggatattg ccagttagga gtcatggatc 2220
aattactgat tatattgaaa gtaaatataa tcaattatgt acttttgagc tttgcaggtt 2280
caatttaggt aaaaatcaca ttatgaaact gggaaagtct gaaggaatat gggcaaaata 2340
tttctcagta aagcttccat gcttcaccct tgacatgatt acccttgagt aaaacatggg 2400
aatttgtaaa aaaaaaaaaa aaaaaaaa 2428

<210> 2
<211> 2219
<212> DNA
<213> Homo sapiens

<400> 2
ggcaggtgtt gaggggctcc cggtccggct gccgccgctc ccccgctccg gacccggggc 60
tccccctagc gccgctgagg agccgcctct gcggctccag gagggcgcag gagcgggact 120
gagagcgcct ggaggctcga gcggagggta attcatttgc acacctgtta gcaagaaaca 180
gaagttgaag gactggaaca agtgaactag gaaagaggga acgccaatcc aaggatagaa 240
ggacaaggac agaatcacca gcactggctg aaggcctcct gtttcctgcg ctttctcctt 300
ttcctgtgaa atctccgagg agaagaaaga atgatggaca gtttatcctt tcactgccac 360
aaggcctgtt tacttggcag taggtcctta agttccttgc ttttttgctg ctgtttggtg 420
actggaagag gcaccagaga ctctcactct ggggaggttt gctggcatgg gtaatctcat 480
taaggtgcta accagggaca tagaccacaa tgcagcacat tttttcttgg actttgaaag 540
taccttaaca tggggaatct tcttaaagtt ttgacatgca cagaccttga gcaggggcca 600
aatttttttcc ttgattttga aaatgcccag cctacagagt ctgagaagga atttatatat 660
caggtgaatg tagtattaaa agatgcagaa ggcatcttgg aggacttgca gtcatacaga 720
ggagctggcc acgaaatacg agaggcaatc cagcatccag cagatgagaa gttgcaagag 780
aaggcatggg gtgcagttgt tccactagta ggcaaattaa agaaatttta cgaattttct 840

cagaggttag aagcagcatt aagaggtctt ctgggagcct taacaagtac cccatattct 900
cccacccagc atctagagcg agagcaggct cttgctaaac agtttgcaga aattcttcat 960
ttcacactcc ggtttgatga actcaagatg acaaatcctg ccatacagaa tgatttcagc 1020
tattatagaa gaacattgag tcgtatgagg attaacaatg taccggcaga aggagaaaat 1080
gaagtaaata atgaattggc aaatcgaatg tctttgtttt atgctgaggc aactccaatg 1140
ctgaaaacct tgagtgatgc cacaacaaaa tttgtatcag agaataaaaa tttaccaata 1200
gaaaatacca cagattgttt aagcacaatg gctagtgtat gcagagtcat gctggaaaca 1260
ccggaataca gaagcagatt tacaaatgaa gagacagtgt cattctgctt gagggtaatg 1320
gtgggtgtca taatactcta tgaccacgta catccagtgg gagcatttgc taaaacttcc 1380
aaaattgata tgaaaggttg tatcaaagtt cttaaggacc aacctcctaa tagtgtggaa 1440
ggtcttctaa atgctctcag gtacacaaca aaacatttga atgatgagac tacctccaag 1500
caaattaaat ccatgctgca ataacaattc tggaataagc acctgctgta gacagaagac 1560
agtattctgc aatgactgag aatgcagttt tttagtgatt gcaattacta tctcatttat 1620
tcttgctttt atttctttcc tctgttcctc ttccctcttt tttaatcatg ttcttaagac 1680
ttcttttctg tgccaaaatc agtaaagtta cactctgaag ggatatcatc ctttcaaacg 1740
ggccatctaa ggcagctaat tatgcattgc attggggtct ctactgagaa aaattctgtg 1800
acttgaacta aatattttta aatgtggatt ttttttgaaa ctaatattta atattgcttc 1860
tcctgcatgg caaaactgcc tattctgcta tttaaaaacc ctcaatgact ttattttcta 1920
ctgccgcctt tttcatgtgc aaccaaaatg aaaatgttta aattaactgt gttgtacaaa 1980
tggtacccaa cacaaacttt ttttaaatta gtaatacttt tgtttaaagt tttaagtttg 2040
cattttgact tttttttgtaa ggatgtatgt tgtgtgttta acctttatta actaacgtta 2100
aaagctgtga tgtgtgcgta gaatattacg tatgcatgtt catgtctaaa gaatggctgt 2160
tgatgataaa ataaaaatca gctttcattt ttctaaaaaa aaaaaaaaaa aaaaaaaaa 2219

<210> 3
<211> 3816
<212> DNA
<213> Homo sapiens

<400> 3
   ggggtcgcgc cgagccgagc cgagccgagc ggagccggcg gagcctctgg aatcacccgg 60
   gtcgctgttc ctgaggtggt caaggtggac agggggcggt ggtgatggcg cagtttgaca 120
   ctgaatacca gcgcctagag gcctcctata gtgattcacc cccaggggag gaggacctgt 180
   tggtgcacgt cgccgagggg agcaagtcac cttggcaccg tattgaaaac cttgacctct 240
   tcttctctcg agtttataat ctgcaccaga agaatggctt cacatgtatg ctcatcgggg 300
   agatctttga gctcatgcag ttcctctttg tggttgcctt cactaccttc ctggtcagct 360
   gcgtggacta tgacatccta tttgccaaca agatggtgaa ccacagtctt caccctactg 420
   aacccgtcaa ggtcactctg ccagacgcct ttttgcctgc tcaagtctgt agtgccagga 480
   ttcaggaaaa tggctccctt atcaccatcc tggtcattgc tggtgtcttc tggatccacc 540
   ggcttatcaa gttcatctat aacatttgct gctactggga gatccactcc ttctacctgc 600
   acgctctgcg catccctatg tctgcccttc cgtattgcac gtggcaagaa gtgcaggccc 660
   ggatcgtgca gacgcagaag gagcaccaga tctgcatcca caaacgtgag ctgacagaac 720
   tggacatcta ccaccgcatc ctccgtttcc agaactacat ggtggcactg gttaacaaat 780
   ccctcctgcc tctgcgcttc cgcctgcctg gcctcgggga agctgtcttc ttcacccgtg 840
   gtctcaagta caactttgag ctgatcctct tctggggacc tggctctctg tttctcaatg 900
   aatggagcct caaggccgag tacaaacgtg gggggcaacg gctagagctg gcccagcgcc 960
   tcagcaaccg catcctgtgg attggcatcg ctaacttcct gccgtgcccc ctcatcctca 1020
   tatggcaaat cctctatgcc ttcttcagct atgctgaggt gctgaagcgg gagccggggg 1080
   ccctgggagc acgctgctgg tcactctatg gccgctgcta cctccgccac ttcaacgagc 1140
   tggagcacga gctgcagtcc cgcctcaacc gtggctacaa gcccgcctcc aagtacatga 1200
   attgcttctt gtcacctctt ttgacactgc tggccaagaa tggagccttc ttcgctggct 1260
   ccatcctggc tgtgcttatt gccctcacca tttatgacga agatgtgttg gctgtggaac 1320
   atgtgctgac caccgtcaca ctcctggggg tcaccgtgac cgtgtgcagg tcctttatcc 1380
   cggaccagca catggtgttc tgccctgagc agctgctccg cgtgatcctc gctcacatcc 1440
   actacatgcc tgaccactgg cagggtaatg cccaccgctc gcagacccgg gacgagtttg 1500
   cccagctctt ccagtacaag gcagtgttca ttttggaaga gttgttgagc cccattgtca 1560
   cacccctcat cctcatcttc tgcctgcgcc cacgggccct ggagattata gacttcttcc 1620
   gaaacttcac cgtggaggtc gttggtgtgg gagatacctg ctcctttgct cagatggatg 1680
   ttcgccagca tggtcatccc cagtggctat ctgctgggca gacagaggcc tcagtgtacc 1740
   agcaagctga ggatggaaag acagagttgt cactcatgca ctttgccatc accaaccctg 1800
   gctggcagcc accacgtgag agcacagcct tcctaggctt cctcaaggag caggttcagc 1860
   gggatggagc agctgctagc ctcgcccaag gggtctgctc ccctgaaaat gccctcttta 1920
   cgtctatcca gtccttacaa tctgagtctg agcccctgag ccttatcgca aatgtggtag 1980
   ctggctcatc ctgccggggc cctccactgc ccagagacct gcagggctcc aggcacaggg 2040
   ctgaagtcgc ctctgccctg cgctccttct ccccgctgca acccgggcag gcgcccacag 2100
   gccgggctca cagcaccatg acaggctctg gggtggatgc caggacagcc agctccggga 2160
   gcagcgtgtg ggaaggacag ctgcagagcc tggtgctgtc agaatatgca tccacagaga 2220
   tgagcctgca tgccctctat atgcaccagc tccacaagca gcaggcccag gctgaacctg 2280
   agcggcatgt atggcaccgc cgggagagtg atgagagtgg agaaagcgcc cctgatgaag 2340
   gggagagggg cgcccgggcc ccccagtcta tccctcgctc tgctagctat ccctgtgtag 2400
   caccccggcc tggagctcct gagaccaccg ccctgcatgg gggcttccag aggcgctacg 2460
   gtggcatcac agatcctggc acagtgccca gggttccctc tcatttctct cggctgcctc 2520
   ttggagggtg ggcagaagat gggcagtcgg catcaaggca ccctgagccc gtgcccgaag 2580
   agggctcgga ggatgagcta ccccctcagg tgcacaaggt atagacaagg ctgagcaggg 2640
   ttcctgtggc ccaggatgga ggccaccgct gccctgccat cccgtctgcc tgccatggga 2700
   cggctcctct gagtgttccc tggccccatg tgtgtggtgt ttgtgtgtct gtgcctggcc 2760
   aagggaggtg ccaacactgg gcttgccaca gccccaggag aggaatttgg ggcctaggaa 2820
   ccgagggcac acgggactct agcctcatcc ccaggacccc cttggctcag agtgtggtgc 2880
   tagaaactgg tccccagccc agccccagta ctgccacctt tacacctacc cctgcaagtc 2940
   cccagagggc tgcccacgat agaagctgcc aagcagggag aacctgtgcc aactgtggag 3000
   tggggaggtt gggcctggac cctcaacccc tgcaaccttc cctagccccc tcaatagatg 3060
   agcaggtcag gctgtggccc ttacctcacc cgcagttctc gcccagtgct gcagccggct 3120
   cacctctctc cgcttcttgc acatcactgg cctgtgtgtg ctgcttgctc ctgttctgtt 3180
   cgcttgctcc cgttccgttc ggcttttgct ttgcgttagg gtgaagaccc tagcgtccag 3240
   ctcccctcaa cgctatattt tgacactaaa aaagaaggtt tctaaattgt aggagcagga 3300
   tggaaatact ttgctgccct tgccatcttt taggatgggc ccccaggaga ctgaggtctt 3360
   cctgggccct cattgctgct tatcgtaccc cccatcacct gcacatggga cagaccgggc 3420
   tggagggtga ccttggctgt gtacgtccca gcaaaagagc tctggcccgc atctcgctgt 3480
   gccctgaagg gggatgaagg gcgatgcctc gcccgaggct ttgggctgct gcactgcatg 3540
   ctgggactgc tcctactctc tgtcccaccc ctcacccagc tgtggtccgg ctttgggaga 3600
   gtggtgaatt gcgctgcccg aactcggagc ggagcagggt agggaccgtg tacagcttga 3660
   taacccttaa taaaaaggga gtttgaccag aaaaaaaaaa aaaaaaaaaa aaaaaaaaaa 3720
   aaaaaaaaaa aaaaaaaaaa aaaaaaaaaa aaaaaagaaa aaaaaaaaaa aaaaaagaaa 3780
   aaaaaaaaaa aaaagaaaaa aaaaaaaaaa aaacct 3816

<210> 4
<211> 4280
<212> DNA
<213> Homo sapiens

<400> 4
gaattcaaga aatgttgccc tggttcacct gatattgaca aactggacgt tgccacaatg 60

acagagtatt taaattttga gaagagtagt tcagtctctc gatatggagc ctctcaagtt 120

gaagatatgg ggaatataat tttagcaatg atttcagagc cttataatca caggttttca 180

gatccagaga gagtgaatta caagtttgaa agtggaactt gcagcaagat ggaacttatt 240

gatgataaca ccgtagtcag ggcacgaggt ttaccatggc agtcttcaga tcaagatatt 300

gcaagattct tcaaaggact caatattgcc aagggaggtg cagcactttg tctgaatgct 360

cagggtcgaa ggaacggaga agctctggtt aggtttgtaa gtgaggagca ccgagaccta 420

gcactacaga ggcacaaaca tcacatgggg acccggtata ttgaggttta caaagcaaca 480

ggtgaagatt tccttaaaat tgctggtggt acttccaatg aggtagccca gtttctctcc 540

aaggaaaatc aagtcattgt tcgcatgcgg gggctccctt tcacggccac agctgaagaa 600

gtggtggcct tctttggaca gcattgccct attactgggg gaaaggaagg catcctcttt 660
gtcacctacc cagatggtag gccaacaggg gacgcttttg tcctctttgc ctgtgaggaa 720
tatgcacaga atgcgttgag gaagcataaa gacttgttgg gtaaaagata cattgaactc 780
ttcaggagca cagcagctga agttcagcag gtgctgaatc gattctcctc ggcccctctc 840
attccacttc caacccctcc cattattcca gtactacctc agcaatttgt gccccctaca 900
aatgttagag actgtatacg ccttcgaggt cttccctatg cagccacaat tgaggacatc 960
ctggatttcc tgggggagtt cgccacagat attcgtactc atggggttca catggttttg 1020
aatcaccagg gccgcccatc aggagatgcc tttatccaga tgaagtctgc ggacagagca 1080
tttatggctg cacagaagtg tcataaaaaa aaacatgaag gacagatatg ttgaagtctt 1140
tcagtgttca gctgaggaga tgaactttgt gttaatgggg ggcactttaa atcgaaatgg 1200
cttatcccca ccgccatgta agttaccatg cctgtctcct ccctcctaca catttccagc 1260
tcctgctgca gttattccta cagaagctgc catttaccag ccctctgtga ttttgaatcc 1320
acgagcactg cagccctcca cagcgtacta cccagcaggc actcagctct tcatgaacta 1380
cacagcgtac tatcccagcc ccccaggttc gcctaatagt cttggctact tccctacagc 1440
tgctaatctt agcggtgtcc ctccacagcc tggcacggtg gtcagaatgc agggcctggc 1500
ctacaatact ggagttaagg aaattcttaa cttcttccaa ggttaccagt gtttgaaaga 1560
tgtatggtga tcttgaaacc tccagacaca agaaaacttc tagcaaattc aggggaagtt 1620
tgtctacact caggctgcag tattttcagc aaacttgatt ggacaaacgg gcctgtgcct 1680
tatcttttgg tggagtgaaa aagtttgagc tagtgaagcc aaatcgtaac ttacagcaag 1740
cagcatgcag catacctggc tctttgctga ttgcaaatag gcatttaaaa tgtgaatttg 1800
gaatcagatg tctccattac ttccagttaa agtggcatca taggtgtttc ctaagtttta 1860
agtcttggat aaaaactcca ccagtgtcta ccatctccac catgaactct gttaaggaag 1920
cttcattttt gtatattccc gctcttttct cttcatttcc ctgtcttctg cataatcatg 1980
ccttcttgct aagtaattca agcataagat cttggaataa taaaatcaca atcttaggag 2040
aaagaataaa attgttattt tcccagtctc ttggccatga tgatatctta tgattaaaaa 2100
caaattaaat tttaaaacac ctgaaaaaaa aaaaaaaaaa gaattcaaga aatgttgccc 2160
tggttcacct gatattgaca aactggacgt tgccacaatg acagagtatt taaattttga 2220
gaagagtagt tcagtctctc gatatggagc ctctcaagtt gaagatatgg ggaatataat 2280
tttagcaatg atttcagagc cttataatca caggttttca gatccagaga gagtgaatta 2340
caagtttgaa agtggaactt gcagcaagat ggaacttatt gatgataaca ccgtagtcag 2400
ggcacgaggt ttaccatggc agtcttcaga tcaagatatt gcaagattct tcaaaggact 2460
caatattgcc aagggaggtg cagcactttg tctgaatgct cagggtcgaa ggaacggaga 2520
agctctggtt aggtttgtaa gtgaggagca ccgagaccta gcactacaga ggcacaaaca 2580
tcacatgggg acccggtata ttgaggttta caaagcaaca ggtgaagatt tccttaaaat 2640

tgctggtggt acttccaatg aggtagccca gtttctctcc aaggaaaatc aagtcattgt 2700
tcgcatgcgg gggctccctt tcacggccac agctgaagaa gtggtggcct tctttggaca 2760
gcattgccct attactgggg gaaaggaagg catcctcttt gtcacctacc cagatggtag 2820
gccaacaggg gacgcttttg tcctctttgc ctgtgaggaa tatgcacaga atgcgttgag 2880
gaagcataaa gacttgttgg gtaaaagata cattgaactc ttcaggagca cagcagctga 2940
agttcagcag gtgctgaatc gattctcctc ggcccctctc attccacttc caacccctcc 3000
cattattcca gtactacctc agcaatttgt gcccctacaa aatgttagag actgtatacg 3060
ccttcgaggt cttccctatg cagccacaat tgaggacatc ctggatttcc tgggggagtt 3120
cgccacagat attcgtactc atggggttca catggttttg aatcaccagg gccgcccatc 3180
aggagatgcc tttatccaga tgaagtctgc ggacagagca tttatggctg cacagaagtg 3240
tcataaaaaa aaacatgaag gacagatatg ttgaagtctt tcagtgttca gctgaggaga 3300
tgaactttgt gttaatgggg ggcactttaa atcgaaatgg cttatcccca ccgccatgta 3360
agttaccatg cctgtctcct ccctcctaca catttccagc tcctgctgca gttattccta 3420
cagaagctgc catttaccag ccctctgtga ttttgaatcc acgagcactg cagccctcca 3480
cagcgtacta cccagcaggc actcagctct tcatgaacta cacagcgtac tatcccagcc 3540
ccccaggttc gcctaatagt cttggctact tccctacagc tgctaatctt agcggtgtcc 3600
ctccacagcc tggcacggtg gtcagaatgc agggcctggc ctacaatact ggagttaagg 3660
aaattcttaa cttcttccaa ggttaccagt gtttgaaaga tgtatggtga tcttgaaacc 3720
tccagacaca agaaaacttc tagcaaattc aggggaagtt tgtctacact caggctgcag 3780
tattttcagc aaacttgatt ggacaaacgg gcctgtgcct tatcttttgg tggagtgaaa 3840
aagtttgagc tagtgaagcc aaatcgtaac ttacagcaag cagcatgcag catacctggc 3900
tctttgctga ttgcaaatag gcatttaaaa tgtgaatttg gaatcagatg tctccattac 3960
ttccagttaa agtggcatca taggtgtttc ctaagtttta agtcttggat aaaaactcca 4020
ccagtgtcta ccatctccac catgaactct gttaaggaag cttcattttt gtatattccc 4080
gctcttttct cttcatttcc ctgtcttctg cataatcatg ccttcttgct aagtaattca 4140
agcataagat cttggaataa taaaatcaca atcttaggag aaagaataaa attgttattt 4200
tcccagtctc ttggccatga tgatatctta tgattaaaaa caaattaaat tttaaaacac 4260
ctgaaaaaaa aaaaaaaaaa 4280

<210> 5
<211> 1384
<212> DNA
<213> Homo sapiens

<400> 5
accgttcttt taactgcgca ggcgcgccgg aagcacctag agagcggcgc gtgcgcagcg 60
ggagtcgaag cggagatccc ggggtcgcgc gagagccgca agcggagttg gtgggcgcta 120
tgctatcacc cgaggcagag cgagtgctgc ggtaccttgt agaagtggag gagctcgccg 180

aggaggtgct ggcggacaag cggcagattg tggacctgga cactaaaagg aatcagaatc 240
gagagggcct gagggccctg cagaaggatc tcagcctctc tgaagatgtg atggtttgct 300
tcgggaacat gtttatcaag atgcctcacc ctgagacaaa ggaaatgatt gaaaaagatc 360
aagatcatct ggataaagaa atagaaaaac tgcggaagca acttaaagtg aaggtcaacc 420
gcctttttga ggcccaaggc aaaccggagc tgaagggttt taacttgaac cccctcaacc 480
aggatgagct taaagctctc aaggtcatct tgaaaggatg agactcaaga accaagatgg 540
gggaccagca accccccagg gtcatggagg acccaggacc ctccaacctt gacacctgta 600
aggacaggat ctgccctgta aggggccagc cgtcaggaat ctggccatga aaacctcttt 660
gtagtgcttg gctactctgt gatggcagga gggaaccttc agcctgtctg gctgctggac 720
ctggacacca gggctcggtg gacacaagat ctattgacgg gccttggtag ccaccagtgg 780
gtgtgtgggg cagtggctgt gggggtgtaa gaatgactgc aacaggcact tcccaacaat 840
ggcctgctgt tcacatggac cctgagcaag gaaggaggga gggaggggca gagtggagtg 900
tcattccagc attcctctca gaagggagag aggttttcag gctggtgcca tgcgattgga 960
ataaagcagg aggctcatgg gtggttgctg aatgaagaac agaatcttgg tgctttgtgg 1020
ctcaccacag ccatctgtgg ggcaggcaca cacacctccc gccagctcca attttgcact 1080
ttttccctgc ttgattccaa gagtaggtgc tgcctagcag cccttcgtgg ccactcttta 1140
ctcaggaggg ccttgcagag tcctgcacca ggcctgggtg agtggatgcg cctcttacca 1200
tatgacacgt gtcaagatgc ccttccgccc cctctgaaag tggggcccgg ccagcactgc 1260
tcgttactgt ctgccttcag tggtctgagg tcccagtatg aactgccgtg aagtcaaaac 1320
tcttatgtgt tcattaaggg ctcaataaat gttagctgaa tgaatgaata gcaaaaaaaa 1380
aaaa 1384

<210> 6
<211> 4159
<212> DNA
<213> Homo sapiens

<400> 6
   gtgcgccctt gcttcgtgcc ctcaacccgc atggcggagc cgctggcgcg ccgcggagag 60
   gccgggcgag tcgggcggtt tcggcgcccg cgctgagccg cggaggaggg gcggaggacg 120
   cccctgcagc cggtgcgtct gccctcagtg aggcggggcg cgcggcggac gcccccgggc 180
   aggggcggga gtggtggagg cgccggcggt tggcactgac aggggcggtg agcgagccgc 240
   tccggtctcc gggcgaggct tggccttccg agcagagacg gcgggaagcg gcggcggcag 300
   cggcggccct agggccggct ggtgaggcga tggcggcgcc ggccccgggg gctggggcag 360
   cctcgggcgg cgctggctgt agcggcggcg gcgcgggcgc gggcgcgggc tcgggctctg 420
   gggccgcggg ggccgggggc cggctgccca gccgggtgct ggagttggtg ttctcttacc 480
   tggagctgtc cgagctgcgg agctgcgccc tggtgtgcaa gcactggtac cgctgcctgc 540
   acggcgatga gaacagcgag gtgtggcgga gcctgtgcgc ccgcagcctg gcagaagagg 600
   ctctgcgcac ggacatcctg tgcaacctgc ccagctacaa ggccaagata cgtgcttttc 660
   aacatgcctt cagcactaat gactgctcca ggaatgtcta cattaagaag aatggcttta 720
   ctttacatcg aaaccccatt gctcagagca ctgatggtgc aaggaccaag attggtttca 780
   gtgagggccg ccatgcatgg gaagtgtggt gggagggccc tctgggcact gtggcagtga 840
   ttggaattgc cacaaaacgg gcccccatgc agtgccaagg ttatgtggca ttgctgggca 900
   gtgatgacca gagctggggc tggaatctgg tggacaataa tctactacat aatggagaag 960
   tcaatggcag ttttccacag tgcaacaacg caccaaaata tcagatagga gaaagaattc 1020
   gagtcatctt ggacatggaa gataagactt tagcttttga acgtggatat gagttcctgg 1080
   gggttgcttt tagaggactt ccaaaggtct gcttataccc agcagtttct gctgtatatg 1140
   gcaacacaga agtgactttg gtttaccttg gaaacctttt ggacggatga cagtggcttt 1200
   cttgtgatga cagacagaat ggaggagaga tctgcttatg ggaagtagaa ccatgaagtg 1260
   actgtcacac atgcatgtcc aagaaacatc ctgaaaacac atgaagtcgt aaactggaga 1320
   agcagctcta cagcagagat tatcttcgtg tttcctcttt ctactgggcc agaaaaatcc 1380
   tcagggttgc agttggttga gtgggcagtt gacatatgca tgttgcaccc gatgttgtct 1440
   ctaagttagc aatgtgttat ttccagcttt aaaggtgaga ttgtagagat gctgtcaaag 1500
   ggataaggaa atagcaagat ttttaagtag tgtgtttgtg aagactgatc ccattttaca 1560
   actgcctgtt ctttctccag tccttttttt tccagccagc ttgactatta gaaaagtatg 1620
   aaactggttg ggttttattt aatattttta atatattgag aagcatggtc tgcctggact 1680
   gcacttctct aaaagtgaga tataaaattg tgcagctatt ttaaaagttg tatataatat 1740
   gtgtgtaaaa aaaaaaaact gtaaaaaaga aaggacaaac aggttgtttt gttctagttc 1800
   taatttctta aaaaccacta catggttaca aaattggaat aacatttggg gacaactggg 1860
   ttaactacaa agaagaggat tttaagagga gatgtgttgt attgactcat tttgtattat 1920
   ttttggctta cagttcccat agctgttaga gtctggtttg tttttgtttt tactctcaaa 1980
   atcatagtaa agatctctca gtctcctggc taaagattga aggaaggcaa atctatttct 2040
   aattatacat atatcagtaa ggatgatctc aacataatag taatgtgtat cttttggtat 2100
   ccagttttat ttttggcctt ctaagaaagt gtctcataac acagaacatt gccatttgct 2160
   cttgtaggcc tcaaatatga aagctattag tcatagagcc taggaaaaaa agaattgatt 2220
   aatggtcctt ttattttgta accttataaa tgctgtagat attatcaaaa aaattttaat 2280
   ttcatattgt ttacatcatg caactaatct aagcctcaaa ctcgttattg gggctataaa 2340
   gaaaacgttt acttacccag ctgaaacagg ttaagaatat tcttaatctc attatagata 2400
   attgccccca tgggacttga aatacaacac cttgtgctga aaacttcagg ttggcaatat 2460
   ttgaaggttt cgttgtagaa gagtttaaca ttaactccta ttttgactta caaatcttgt 2520
   ttctcatcac taaaatgctt ttgaattaat aatccaaccc acatgagctg agagtttttc 2580
   ttttgttaga aaagaaacag acatctttct gtatgaaagt ataaattgta tggttttaga 2640
   tacataagaa ttgacaaaag cgagcgaaat ctttgtactt ctgagttctt gctgtatgta 2700
   tgttttgttt taaatctgat tagggacacc cagcagctgg ccgggattct tggattgctc 2760
   cttgggagtt aagattgtca atactcctgt gaagcaaggg atttcagcca tagaacaaag 2820
   atttattgtt gccacctgaa aagtttacaa gtatttattg tgtatttgat acattgcttg 2880
   aaaagatgaa atctgttaaa gattcttttc gatgtccagg ttaagaagaa acctccttgt 2940
   attgagtgaa attatatgtt aaatgtatta gagaatgtag gtggtataga aattgatttt 3000
   tcttggtgta gaacaactca gttcggcaaa gtttaaaatt tgattaaaca agagaagtgg 3060
   ttcaggttga agatggactt gttaggaagt gatcaagtcc tttaagtact tgtttctttt 3120
   tcaggttgtg atgtggccat tccgaatttt gttgagagtt tggtttataa ttgtctcttt 3180
   tgtcttgtta gtaaacattc atttgcaaca gttttgaagg tgctgagtgg aaaaccgaaa 3240
   cacatggtta ttgcgtattg gacctagaat gaaataattg cctcaatatt taacaacaag 3300
   ccattcttat ctcaaagatt taaattcccg aatgtcccat tcgcaaatca tatgcaattg 3360
   aagtgagcag catgagcatc tgggtcatga gggccttcat ttacgtaaat ttgtcactaa 3420
   aacccagtag tagctctaca aaatcttaaa ctgctgcagt gctcaaggag atggaatatc 3480
   tttgtcattg gtgctgagga gagcatttcg gtagaagaca gttgcgcctg aagattgagt 3540
   gtaaatcatt caaaccagtg gttctcagtg ttggctgtat acactttgta gtcactttgg 3600
   aatgttggaa gacacatcga tgcttgggtt ccgtatgcca agattctgat gttggtctgg 3660
   aatatgagct ggtcataagg atttttaaaa actttctggt catttcaata tgctgccaag 3720
   gttgagaacc actgttgtaa aattcacctt gagttttctc atctgcaaaa tagaaaaaaa 3780
   aaaatccttg ctccctccct tcactacctc acaaggatat tgagggtaaa ggagaaaata 3840
   atgggaaagt gcttgtgccg tggatgaaaa gtgctattaa aagtcaaagg agtgttctgt 3900
   ttcaattcat agtatgatca gggaaagtgt aactgagtat actttgttga cttgggaaac 3960
   ctggagcact ttctttggtt ggttaacgaa gcatgcagat gtggaagcag acgttactat 4020
   tatccctact atggtcttct gtcatactga gacaggctgt tttaattacc tggttttaca 4080
   taggaaagaa gaaatattaa ggcttaaagt ttgtaatgat caatggctca taattcatta 4140
   aatcttttca tacaaggaa 4159

<210> 7
<211> 4021
<212> DNA
<213> Homo sapiens

<400> 7
ggtagccgcc ccgccccgcg gggcgccacg ggcgggtctt ggcagcgccc actgagccag 60
ccgggccgca ggtgccgccc ccgatacacg gtgtcccgcc caagctgatc cgcgtctgcg 120
gtcggtcggt gcgtgcgtgc gcctcgtcgg tccgcgtgtc tggccgagag cccccttcct 180
ctgcggccat gactccgccg ccgccgccgc cccctccccc gggccctgac cccgcggccg 240
accccgccgc ggacccctgc ccctggcccg gatcactggt cgtcctcttc ggggctacgg 300
cgggtgcgct gggacgggac ctgggctcgg acgagaccga cttaatcctc ctagtttggc 360

aagtggttga gccgcggagc cgccaggtgg ggacgctgca caaatcgctg gttcgtgccg 420
aggcggccgc actgagtacg cagtgccgcg aggcgagcgg cctgagcgcc gacagcctgg 480
cgcgggcaga gccgctggac aaggtgctgc agcagttctc acagctggtg aacggggatg 540
tggctttgct gggcgggggc ccctacatgc tctgcactga tgggcagcag ctattgcgac 600
aggtcctgca ccccgaggcc tccaggaaga acctggtgct ccccgacatg ttcttctcct 660
tctatgacct ccgaagagaa ttccatatgc agcatccaag cacctgccct gccagggacc 720
tcactgtggc caccatggca cagggtttag gactggagac agatgccaca gaggatgact 780
ttggggtctg ggaagtcaag acaatggtag ctgttatcct ccatctactc aaagagccca 840
gcagtcaatt gttttcgaag cccgaggtga taaagcagaa atacgagacg gggccttgca 900
gcaaggctga tgtggtggac agtgagactg tggtacgggc tcgtgggttg ccgtggcagt 960
catcagacca ggacgtggct cgcttcttca aagggctcaa cgtggccagg ggtggtgtag 1020
cactctgcct caacgcccag ggccgcagaa atggcgaggc cctcatccgc tttgtggaca 1080
gcgagcagcg ggacctagcg ctgcagagac acaagcacca catgggcgtc cgctatattg 1140
aggtgtataa agcgacaggg gaggagtttg taaagattgc agggggcaca tcactagagg 1200
tggctcgttt cttgtcacgg gaagaccaag tgatcctgcg gctgcgggga ctgcccttct 1260
cggctgggcc aacggacgtg cttggcttcc tggggccaga gtgcccagtg actgggggta 1320
ccgaggggct gctctttgtg cgccatcctg atggccggcc gactggtgat gccttcgccc 1380
tctttgcttg tgaggagctg gcacaggctg cactgcgcag gcacaagggc atgctgggta 1440
agcgatacat tgaactcttc cggagcactg cagccgaagt gcagcaggtc ttgaaccgct 1500
atgcatccgg cccactcctt cctacactga ctgccccact gctgcccatc cccttcccac 1560
tggcacctgg gactgggagg gactgtgtac gcctccgagg cctgccctac acggccacca 1620
ttgaagacat cctgagcttt ctgggggagg cagcagctga cattcggccc cacggtgtac 1680
acatggtgct caaccagcag ggccggccat cgggcgatgc cttcattcag atgacatcag 1740
cagagcgagc cctagctgct gctcagcgtt gccataagaa ggtgatgaag gagcgctacg 1800
tggaggtggt cccctgttcc acagaggaga tgagccgagt gctgatgggg ggcaccttgg 1860
gccgcagtgg catgtcccct ccaccctgca agctgccctg cctctcacca cctacctaca 1920
ccaccttcca agccacccca acgctcattc ccacggagac ggcagctcta tacccctctt 1980
cagcactgct cccagctgcc agggtgcctg ctgcccccac ccctgttgcc tactatccag 2040
ggccagccac tcaactctac ctgaactaca cagcctacta cccaagcccc ccagtctccc 2100
ccaccactgt gggctacctc actacaccca ctgctgccct ggcctctgct cccacctcag 2160
tgttgtccca gtcaggagcc ttggtccgca tgcagggtgt cccatacacg gctggtatga 2220
aggatctgct cagcgtcttc caggcctacc agctacccgc tgatgactac accagtctga 2280
tgcctgttgg tgacccacct cgcactgtgt tacaagcccc caaggaatgg gtgtgtttgt 2340
aggagagaaa gccaggaggt aagagccagc tgatatcctc ggcgaacatg tctctcctga 2400

gtccagaaga ccagcaccct caacctggta gcttctttct ggcttgtcaa agctctcaga 2460
aggtacctag aggagcccaa gccccagctc catcctccac ttattctgcc tgtttccccc 2520
aaagacaatg gctggaccct gcatgcaggg ctgggggtgg aatggggcta accagctcct 2580
gatggcctga gccaggcatc ttgactggca cctggagagc ccttaagtct gtcctggctg 2640
tggcccatgc cgacagatat cgtggggctg acaggtccac ggcaggcttg ctttctttta 2700
taaaatggaa gctctggtac cttcaatgta tgactcctgg gagaatcaag ggtccatctg 2760
agcctctgag taaagatccc aatgttctac ctctccctgt ccctcttgta ggggataggg 2820
aggcagagag agccagcccc taccctcaga gtatctggac ctcagagacc atgttgtgcc 2880
aggggtggtc ccacctaaag atgctagccc ctctccaggt gggcataagg agtaacagat 2940
ggcaaaacca caaactattt tgatggactg tgctgcagta tcaccagaag acattagggg 3000
gcagtaggcc cccacacaaa accttcaggc ttgaatttta aaggggagga ctttctgcca 3060
acttttcttg tatgccttgg gaaagccagt tgccctgaac ccagcagaca ccatggaatg 3120
tcctttgcac gcattaaatg gtacagaact gaagcctcgg aagcaatttg gaactcgatc 3180
ttctcttcct taaatgaaaa gttattgacc aaatggactt tttaaaagac acaggaccct 3240
taactttgcc ccaaagtgag gggctccaca ccaaccccag gcggaggaac actcagacag 3300
attaaggata ctgttgacct gtcactgttt attatttcag cactaaaact gaggagcctc 3360
aactgctggc tcttcttccc tttgtatttg tgtaaggagc actgcactcc cataaaaggt 3420
tttaaaatac aaaatgtaca agaacacaca attccaagtg ctgtaaacat aactgagaac 3480
cagttccttt actaaacatc cattttataa aacacaaggt ttcaatttga gcccatctga 3540
gccttaaaga tccattctga ataccaaaaa cagggcttca cagccaggcc cagaagaggt 3600
ctggtgataa tggctggccc tgggtgggga tagtttacac ccgggcagca gcaccacaca 3660
tgaacccaaa gacatgttct ttttaaagct gttttcagcc atgtttctct gtgcatctcc 3720
agtaagcaga aggctaccca ttccattcct caacccaaga gctagcacag ttagagtagg 3780
agggggtgcg tactagcacg tgcccagttg ctcagtgctg ctagtagaaa ttgatttgca 3840
tagtccaatg gatgtgtgct ttaacaccac tatgttgcac aaaaatttaa gtctttatct 3900
acaaagccaa aaaatattga ctcttaacac caaagctttt acaaagctga tataaaactg 3960
cttacatagt atacaaagct ctattttaaa atttaatgtt tattttaaat aggaaagcat 4020
t 4021

<210> 8
<211> 1810
<212> DNA
<213> Homo sapiens

<400> 8
gaaggccctg ccgggcggcg gcggcggcga cagcgtgcga gccatggtcg cgctggagaa 60
ccccgagtgc ggcccggagg cggcggaggg caccccgggc gggcggcggc tgctgcccct 120
tcccagctgc ctgcctgccc tagccagctc ccaggtgaag agactctcgg cttccaggcg 180

gaaacagcac ttcatcaacc aggcagtgcg gaactcagac ctcgtgccca aggccaaggg 240
gcggaagagc ctccagcgcc tggagaacac ccagtacctc ctgaccctgc tggagacaga 300
cgggggcctg cctggcctgg aggatgggga cttggcaccc cctgcatcac caggcatctt 360
tgccgaggcc tgcaacaacg ccacctatgt ggaggtctgg aacgatttca tgaaccgctc 420
cggggaggag caggagcggg ttcttcgcta cctggaggat gagggcagga gcaaggcgcg 480
gaggaggggc cctggccgtg gggaggaccg gaggagagag gaccccgcct atacaccccg 540
cgagtgcttc cagcgcatca gccggcgtct gcgagccgtc ctcaagcgca gccgcatccc 600
catggaaacg ctggagacct gggaggagcg gctgcttcgg ttcttctccg tgtcccccca 660
ggccgtgtac acagcaatgc tagacaacag cttcgagagg cttctgctgc acgctgtctg 720
ccagtacatg gacctcatct cggccagtgc tgacctggag gggaagcggc agatgaaggt 780
cagtaatcgg cacctggatt tcctgccgcc ggggctgctc ctgtccgcct acctggagca 840
gcacagctga tggcggcccc gcggagaccc cgctgccacc tcgcccagcc atcaagccct 900
ccgatacctt cggctaaaat atctttcata tttttagaat ttgtcctcgg aaaccttttt 960
cgcttggggt ggtctctctc actctgcccc ctcctcacgc agctcttggc agtcaacaga 1020
cgctggcggc tggggctgcc catgccatcc cagctccaag cttcccactc cgggacttgt 1080
gtttgggtgg ggagacctga cctgggcatg ttcctgtttc ttcatcgttg agcttttctg 1140
gcccggtctg aagctcaagt gaggaggggg aggctgggtt tttatcactt ttaatgaatt 1200
tggtgtgatt tgttgtagat ttttaaattt cccttttgga gagaaaaacc aaaaaaactc 1260
gccccactgg taaaacatgg gtcttggtcc cagcccctgc tcagcccctc ccagttttta 1320
gcttgaatga gggtggggtc tctgggaccc tgcccctcat gccagaagca tcttgtgttg 1380
tatatgtgtg cgcgcgtgtg ccctgagacc caggacagaa gccacggtcc taagagccgg 1440
ttttatcctc gtcattctgc gtgtcctccc ccacgccacc tgtgtcgggg ctcagggtct 1500
cctgctttat atgagccccc ttcctttcct cccctccttt atgctggggg tccaggactt 1560
ccagccagaa gcctctgccc ttgcactacc ttgtctgtca ccccatcccg tgtcccctcg 1620
tcccccagcc tgactcctgc ctgatagctc ctgtgtcccc atgctggtcc tcctggccca 1680
ggctgcagga gccaggctgg ggggcctccg cacccccttg ctgcgtgtgg gtaattgtgt 1740
tttgggggaa agtggggaat ttaataaatt tctggtgctc tggcaaaaaa aaaaaaaaaa 1800
aaaaaaaaaa 1810

<210> 9
<211> 3417
<212> DNA
<213> Homo sapiens

<400> 9
cgaggctggc atagcggctg ccgacccgcc ttcgttcctc caccccctgc acgggactgc 60
tgggcccgcc ccgccccgcc tgcaggtgaa gcggccgcag ccgccgagta ggtgcgtggg 120
gatgatctca ctcgcgcgct ccgcgccagg aggaggagga gcgggagcgg atccaacttc 180

cgggtagtgg agccgcaagc caccggcatc ttgctttttc ttccccctcc tcctgtgtgc 240
cccgcgccgc tccctctttc ccttttattc ccggccccac ccgccaaaat gaacagctcg 300
gacgaagaga agcagctgca gctcattacc agtctgaagg agcaagcaat aggcgaatat 360
gaagacctta gagcagagaa ccagaaaaca aaggagaagt gtgacaaaat taggcaagaa 420
cgagatgaag ccgttaaaaa actggaagaa tttcagaaaa tttctcacat ggtcatagag 480
gaagttaatt tcatgcagaa ccatcttgaa atagagaaga cttgtcgaga aagtgctgaa 540
gctttggcaa caaagctaaa taaagaaaat aaaacgttga aaagaatcag catgttgtac 600
atggccaagc tgggaccaga tgtaataact gaagagataa acattgatga tgaagattcg 660
actacagaca cagacggtgc cgccgagact tgtgtctcag tacagtgtca gaagcaaatt 720
aaagaacttc gagatcaaat tgtatctgtt caggaggaaa agaagatttt agccattgag 780
ctggaaaatc tcaagagcaa actcgtagaa gtaattgaag aagtaaataa agttaaacaa 840
gaaaagactg ttttaaattc agaagttctt gaacagagaa aagtcttaga aaaatgcaat 900
agagtgtcca tgttagctgt agaagagtat gaggagatgc aagtaaacct ggagctggag 960
aaggaccttc gaaagaaagc agagtcattt gcacaagaga tgttcattga gcaaaacaag 1020
ctaaagagac aaagccacct tctgctgcag agctccatcc ctgatcagca gcttttgaaa 1080
gctttagacg aaaatgcaaa actcacccag caacttgaag aagagagaat tcagcatcaa 1140
caaaaggtca aagaattaga agagcaacta gaaaatgaaa cactccacaa agaaatacac 1200
aacctcaaac agcaactgga gcttctagag gaagataaaa aggaattgga attgaaatat 1260
cagaattctg aagagaaagc cagaaattta aagcactctg ttgatgaact ccagaaacga 1320
gtgaaccagt ctgagaattc agtacctcca ccacctcctc ctccaccacc acttccccct 1380
ccacctccca atcctatccg atccctcatg tccatgatcc ggaaacgatc ccaccccagt 1440
ggcagtggtg ctaagaaaga aaaggcaact caaccagaaa caactgaaga agtcacagat 1500
ctaaagaggc aagcagttga agagatgatg gatagaatta aaaagggagt tcatcttaga 1560
cccgttaatc agacagccag accgaagaca aagccagaat cttcgaaagg ctgcgaaagt 1620
gcagtggatg aactaaaagg aatactggcc tcccagtagc attggatgca ggaaaaaata 1680
cattgacggt gaaaaacaag ccgaaccagt tgtagtttta gatcctgttt ctacacatga 1740
accccaaacc aaagaccagg ttgctgaaaa agatccaact caacacaagg aggatgaagg 1800
cgaaattcaa ccagaaaaca aagaagacag cattgaaaac gtgagagaga cagacagctc 1860
caactgctga tccataaacc agaagcctga tacgtttgga agtccttttc aataagcaca 1920
tgattagtgt tgttatattg gcaagggctg tagacattct gctctggtca ctgtattcag 1980
aatacaggtt cttttctggt gtcacttttg taagtagcaa ctataaacat aagtaagctg 2040
tttagcaaaa cacacattcc tagtaggttt tggttttttg atctttataa agatgaggtt 2100
tttttcctag ttactgtatt aagtatgact tcttttagaa ggttacaaaa aaattcagat 2160
gttgatacct ttttaggaaa tgtgcatacc actcatcaaa tggaatgctg aaagtttgag 2220
gtgcttgtat ataatcggat aaacaaaact gatcaaccca atgtgatttt aaaagccccc 2280

aaagaagctt ctgttttggg tctgatcctc ttgatggaga aactgcagca gcatggaaat 2340
tgttgggtac tgtggcatac aagttatttt ctacagtaga ctgagataaa ctgaaaactc 2400
aggagctggc atcaaactcg tagtcccata gtcagtgtta attacacaca ttgttaacta 2460
ttggatgaaa aatacatgct attgattgtg tccaaagcct cccgaggacc tccgtgggga 2520
tgctctggta gcctgaatac agaactgagg tgaaagtcca aaccttgaat tttacagtag 2580
taagttggta aaccatgtgc tctgtgctat gagttaatta tgttttccca aatactaatg 2640
tggcacaagt accatatttt atcagagttc ttatgtacag tatggtgaag ataagtgaca 2700
agcacacatt tttcttgctt cactgctgtt ctatattaca caggtttgtt gttgtttttt 2760
ttaaaaaaga aattaagcag tagttagtct ctaaaaatac aatgtttcag gctaccacag 2820
tgaataaata gaaatgtaat cagggattaa aaaaaaaact tatgcagctt ttcaaagttg 2880
attgtttcaa aattggtgtt tatttaaaat aagtggtaat gtacttgaat gcacttttta 2940
tgacaatgat tcagtaatgg taattttact attaaagaaa gtgaaaggtt tagttttgtt 3000
agcatggctc agcatgtagc tgtcaggtgt tttcacctaa agggcaaaag aaaatgatag 3060
taataattgc agtagttgta ttgtattgta tttttgcacg tgtggtaagc ataggcttga 3120
agaggtgggt aggcaggtac atgtacttcc taaattttga gataattatc tttctgtaag 3180
ttcgttatgc ttgactgttt ccatgttctc ccaataatga ttttatagtt acttatcact 3240
ttactcatgg agaattaaaa cgtaatgttt ttcaactgta tctttcttta actggataat 3300
actgctatat gatatgctta ctacagactg cattaattca cgaaacgaat tctgttatgc 3360
tgtaatttga actctcctca ccacaactta ttaaaaaggc accaatagtt tcccatt 3417

<210> 10
<211> 342
<212> PRT
<213> Homo sapiens

<400> 10

Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ala Leu Leu Leu Gly Ala Ser Ser
        35                  40                  45

Leu Ser Gly Arg Cys Trp Ala Gly Ser Leu Trp Leu Phe Lys Asp Pro
    50                  55                  60

Cys Ala Ala Pro Asn Glu Gly Phe Cys Ser Ala Gly Val Gln Thr Glu
65                  70                  75                  80

Ala Gly Val Ala Asp Leu Thr Trp Val Gly Glu Arg Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Ser Val Leu Ser Ser Gly Thr Gln Ala Val Ser Gly Ser
    130                 135                 140

Lys Asp Ile Cys Ile Lys Val Trp Asp Leu Ala Gln Gln Val Val Leu
145                 150                 155                 160

Ser Ser Tyr Arg Ala His Ala Ala Gln Val Thr Cys Val Ala Ala Ser
                165                 170                 175

Pro His Lys Asp Ser Val Phe Leu Ser Cys Ser Glu Asp Asn Arg Ile
            180                 185                 190

Leu Leu Trp Asp Thr Arg Cys Pro Lys Pro Ala Ser Gln Ile Gly Cys
        195                 200                 205

Ser Ala Pro Gly Tyr Leu Pro Thr Ser Leu Ala Trp His Pro Gln Gln
    210                 215                 220

Ser Glu Val Phe Val Phe Gly Asp Glu Asn Gly Thr Val Ser Leu Val
225                 230                 235                 240

Asp Thr Lys Ser Thr Ser Cys Val Leu Ser Ser Ala Val His Ser Gln
                245                 250                 255

Cys Val Thr Gly Leu Val Phe Ser Pro His Ser Val Pro Phe Leu Ala
            260                 265                 270

Ser Leu Ser Glu Asp Cys Ser Leu Ala Val Leu Asp Ser Ser Leu Ser
        275                 280                 285

Glu Leu Phe Arg Ser Gln Ala His Arg Asp Phe Val Arg Asp Ala Thr
    290                 295                 300

Trp Ser Pro Leu Asn His Ser Leu Leu Thr Thr Val Gly Trp Asp His
305                 310                 315                 320

Gln Val Val His His Val Val Pro Thr Glu Pro Leu Pro Ala Pro Gly
                325                 330                 335

Pro Ala Ser Val Thr Glu
            340

<210> 11
<211> 324
<212> PRT

<213>  Homo sapiens

<400>  11

Met Gly Asn Leu Leu Lys Val Leu Thr Cys Thr Asp Leu Glu Gln Gly
1               5                   10                  15

Pro Asn Phe Phe Leu Asp Phe Glu Asn Ala Gln Pro Thr Glu Ser Glu
            20                  25                  30

Lys Glu Ile Tyr Asn Gln Val Asn Val Val Leu Lys Asp Ala Glu Gly
        35                  40                  45

Ile Leu Glu Asp Leu Gln Ser Tyr Arg Gly Ala Gly His Glu Ile Arg
    50                  55                  60

Glu Ala Ile Gln His Pro Ala Asp Glu Lys Leu Gln Glu Lys Ala Trp
65                  70                  75                  80

Gly Ala Val Val Pro Leu Val Gly Lys Leu Lys Lys Phe Tyr Glu Phe
                85                  90                  95

Ser Gln Arg Leu Glu Ala Ala Leu Arg Gly Leu Leu Gly Ala Leu Thr
            100                 105                 110

Ser Thr Pro Tyr Ser Pro Thr Gln His Leu Glu Arg Glu Gln Ala Leu
        115                 120                 125

Ala Lys Gln Phe Ala Glu Ile Leu His Phe Thr Leu Arg Phe Asp Glu
    130                 135                 140

Leu Lys Met Thr Asn Pro Ala Ile Gln Asn Asp Phe Ser Tyr Tyr Arg
145                 150                 155                 160

Arg Thr Leu Ser Arg Met Arg Ile Asn Asn Val Pro Ala Glu Gly Glu
                165                 170                 175

Asn Glu Val Asn Asn Glu Leu Ala Asn Arg Met Ser Leu Phe Tyr Ala
            180                 185                 190

Glu Ala Thr Pro Met Leu Lys Thr Leu Ser Asp Ala Thr Thr Lys Phe
        195                 200                 205

Val Ser Glu Asn Lys Asn Leu Pro Ile Glu Asn Thr Thr Asp Cys Leu
    210                 215                 220

Ser Thr Met Ala Ser Val Cys Arg Val Met Leu Glu Thr Pro Glu Tyr
225                 230                 235                 240

Arg Ser Arg Phe Thr Asn Glu Glu Thr Val Ser Phe Cys Leu Arg Val
                245                 250                 255

Met Val Gly Val Ile Ile Leu Tyr Asp His Val His Pro Val Gly Ala

            260                 265                 270

Phe Ala Lys Thr Ser Lys Ile Asp Met Lys Gly Cys Ile Lys Val Leu
        275                 280                 285

Lys Asp Gln Pro Pro Asn Ser Val Glu Gly Leu Leu Asn Ala Leu Arg
    290                 295                 300

Tyr Thr Thr Lys His Leu Asn Asp Glu Thr Thr Ser Lys Gln Ile Lys
305                 310                 315                 320

Ser Met Leu Gln

<210> 12
<211> 839
<212> PRT
<213> Homo sapiens

<400> 12

Met Ala Gln Phe Asp Thr Glu Tyr Gln Arg Leu Glu Ala Ser Tyr Ser
1               5                   10                  15

Asp Ser Pro Pro Gly Glu Glu Asp Leu Leu Val His Val Ala Glu Gly
            20                  25                  30

Ser Lys Ser Pro Trp His Arg Ile Glu Asn Leu Asp Leu Phe Phe Ser
        35                  40                  45

Arg Val Tyr Asn Leu His Gln Lys Asn Gly Phe Thr Cys Met Leu Ile
    50                  55                  60

Gly Glu Ile Phe Glu Leu Met Gln Phe Leu Phe Val Val Ala Phe Thr
65                  70                  75                  80

Thr Phe Leu Val Ser Cys Val Asp Tyr Asp Ile Leu Phe Ala Asn Lys
                85                  90                  95

Met Val Asn His Ser Leu His Pro Thr Glu Pro Val Lys Val Thr Leu
            100                 105                 110

Pro Asp Ala Phe Leu Pro Ala Gln Val Cys Ser Ala Arg Ile Gln Glu
        115                 120                 125

Asn Gly Ser Leu Ile Thr Ile Leu Val Ile Ala Gly Val Phe Trp Ile
    130                 135                 140

His Arg Leu Ile Lys Phe Ile Tyr Asn Ile Cys Cys Tyr Trp Glu Ile
145                 150                 155                 160

His Ser Phe Tyr Leu His Ala Leu Arg Ile Pro Met Ser Ala Leu Pro
                165                 170                 175

Tyr Cys Thr Trp Gln Glu Val Gln Ala Arg Ile Val Gln Thr Gln Lys
            180                 185                 190

Glu His Gln Ile Cys Ile His Lys Arg Glu Leu Thr Glu Leu Asp Ile
        195                 200                 205

Tyr His Arg Ile Leu Arg Phe Gln Asn Tyr Met Val Ala Leu Val Asn
    210                 215                 220

Lys Ser Leu Leu Pro Leu Arg Phe Arg Leu Pro Gly Leu Gly Glu Ala
225                 230                 235                 240

Val Phe Phe Thr Arg Gly Leu Lys Tyr Asn Phe Glu Leu Ile Leu Phe
                245                 250                 255

Trp Gly Pro Gly Ser Leu Phe Leu Asn Glu Trp Ser Leu Lys Ala Glu
            260                 265                 270

Tyr Lys Arg Gly Gly Gln Arg Leu Glu Leu Ala Gln Arg Leu Ser Asn
        275                 280                 285

Arg Ile Leu Trp Ile Gly Ile Ala Asn Phe Leu Pro Cys Pro Leu Ile
    290                 295                 300

Leu Ile Trp Gln Ile Leu Tyr Ala Phe Phe Ser Tyr Ala Glu Val Leu
305                 310                 315                 320

Lys Arg Glu Pro Gly Ala Leu Gly Ala Arg Cys Trp Ser Leu Tyr Gly
                325                 330                 335

Arg Cys Tyr Leu Arg His Phe Asn Glu Leu Glu His Glu Leu Gln Ser
            340                 345                 350

Arg Leu Asn Arg Gly Tyr Lys Pro Ala Ser Lys Tyr Met Asn Cys Phe
        355                 360                 365

Leu Ser Pro Leu Leu Thr Leu Leu Ala Lys Asn Gly Ala Phe Phe Ala
    370                 375                 380

Gly Ser Ile Leu Ala Val Leu Ile Ala Leu Thr Ile Tyr Asp Glu Asp
385                 390                 395                 400

Val Leu Ala Val Glu His Val Leu Thr Thr Val Thr Leu Leu Gly Val
                405                 410                 415

Thr Val Thr Val Cys Arg Ser Phe Ile Pro Asp Gln His Met Val Phe
            420                 425                 430

Cys Pro Glu Gln Leu Leu Arg Val Ile Leu Ala His Ile His Tyr Met
        435                 440                 445

Pro Asp His Trp Gln Gly Asn Ala His Arg Ser Gln Thr Arg Asp Glu
    450                 455                 460

Phe Ala Gln Leu Phe Gln Tyr Lys Ala Val Phe Ile Leu Glu Glu Leu
465                 470                 475                 480

Leu Ser Pro Ile Val Thr Pro Leu Ile Leu Ile Phe Cys Leu Arg Pro
                485                 490                 495

Arg Ala Leu Glu Ile Ile Asp Phe Phe Arg Asn Phe Thr Val Glu Val
            500                 505                 510

Val Gly Val Gly Asp Thr Cys Ser Phe Ala Gln Met Asp Val Arg Gln
        515                 520                 525

His Gly His Pro Gln Trp Leu Ser Ala Gly Gln Thr Glu Ala Ser Val
    530                 535                 540

Tyr Gln Gln Ala Glu Asp Gly Lys Thr Glu Leu Ser Leu Met His Phe
545                 550                 555                 560

Ala Ile Thr Asn Pro Gly Trp Gln Pro Pro Arg Glu Ser Thr Ala Phe
                565                 570                 575

Leu Gly Phe Leu Lys Glu Gln Val Gln Arg Asp Gly Ala Ala Ala Ser
            580                 585                 590

Leu Ala Gln Gly Gly Leu Leu Pro Glu Asn Ala Leu Phe Thr Ser Ile
        595                 600                 605

Gln Ser Leu Gln Ser Glu Ser Glu Pro Leu Ser Leu Ile Ala Asn Val
    610                 615                 620

Val Ala Gly Ser Ser Cys Arg Gly Pro Pro Leu Pro Arg Asp Leu Gln
625                 630                 635                 640

Gly Ser Arg His Arg Ala Glu Val Ala Ser Ala Leu Arg Ser Phe Ser
                645                 650                 655

Pro Leu Gln Pro Gly Gln Ala Pro Thr Gly Arg Ala His Ser Thr Met
            660                 665                 670

Thr Gly Ser Gly Val Asp Ala Arg Thr Ala Ser Ser Gly Ser Ser Val
        675                 680                 685

Trp Glu Gly Gln Leu Gln Ser Leu Val Leu Ser Glu Tyr Ala Ser Thr
    690                 695                 700

Glu Met Ser Leu His Ala Leu Tyr Met His Gln Leu His Lys Gln Gln
705                 710                 715                 720

Ala Gln Ala Glu Pro Glu Arg His Val Trp His Arg Arg Glu Ser Asp

                725                 730                 735

Glu Ser Gly Glu Ser Ala Pro Asp Glu Gly Gly Glu Gly Ala Arg Ala
            740                 745                 750

Pro Gln Ser Ile Pro Arg Ser Ala Ser Tyr Pro Cys Val Ala Pro Arg
        755                 760                 765

Pro Gly Ala Pro Glu Thr Thr Ala Leu His Gly Gly Phe Gln Arg Arg
    770                 775                 780

Tyr Gly Gly Ile Thr Asp Pro Gly Thr Val Pro Arg Val Pro Ser His
785                 790                 795                 800

Phe Ser Arg Leu Pro Leu Gly Gly Trp Ala Glu Asp Gly Gln Ser Ala
                805                 810                 815

Ser Arg His Pro Glu Pro Val Pro Glu Glu Gly Ser Glu Asp Glu Leu
            820                 825                 830

Pro Pro Gln Val His Lys Val
        835

<210> 13
<211> 358
<212> PRT
<213> Homo sapiens

<400> 13

Met Thr Glu Tyr Leu Asn Phe Glu Lys Ser Ser Ser Val Ser Arg Tyr
1               5                   10                  15

Gly Ala Ser Gln Val Glu Asp Met Gly Asn Ile Ile Leu Ala Met Ile
            20                  25                  30

Ser Glu Pro Tyr Asn His Arg Phe Ser Asp Pro Glu Arg Val Asn Tyr
        35                  40                  45

Lys Phe Glu Ser Gly Thr Cys Ser Lys Met Glu Leu Ile Asp Asp Asn
    50                  55                  60

Thr Val Val Arg Ala Arg Gly Leu Pro Trp Gln Ser Ser Asp Gln Asp
65                  70                  75                  80

Ile Ala Arg Phe Phe Lys Gly Leu Asn Ile Ala Lys Gly Gly Ala Ala
                85                  90                  95

Leu Cys Leu Asn Ala Gln Gly Arg Arg Asn Gly Glu Ala Leu Val Arg
            100                 105                 110

Phe Val Ser Glu Glu His Arg Asp Leu Ala Leu Gln Arg His Lys His
        115                 120                 125

His Met Gly Thr Arg Tyr Ile Glu Val Tyr Lys Ala Thr Gly Glu Asp
    130                 135                 140

Phe Leu Lys Ile Ala Gly Gly Thr Ser Asn Glu Val Ala Gln Phe Leu
145                 150                 155                 160

Ser Lys Glu Asn Gln Val Ile Val Arg Met Arg Gly Leu Pro Phe Thr
                165                 170                 175

Ala Thr Ala Glu Glu Val Val Ala Phe Phe Gly Gln His Cys Pro Ile
            180                 185                 190

Thr Gly Gly Lys Glu Gly Ile Leu Phe Val Thr Tyr Pro Asp Gly Arg
        195                 200                 205

Pro Thr Gly Asp Ala Phe Val Leu Phe Ala Cys Glu Glu Tyr Ala Gln
    210                 215                 220

Asn Ala Leu Arg Lys His Lys Asp Leu Leu Gly Lys Arg Tyr Ile Glu
225                 230                 235                 240

Leu Phe Arg Ser Thr Ala Ala Glu Val Gln Gln Val Leu Asn Arg Phe
                245                 250                 255

Ser Ser Ala Pro Leu Ile Pro Leu Pro Thr Pro Pro Ile Ile Pro Val
            260                 265                 270

Leu Pro Gln Gln Phe Val Pro Pro Thr Asn Val Arg Asp Cys Ile Arg
        275                 280                 285

Leu Arg Gly Leu Pro Tyr Ala Ala Thr Ile Glu Asp Ile Leu Asp Phe
    290                 295                 300

Leu Gly Glu Phe Ala Thr Asp Ile Arg Thr His Gly Val His Met Val
305                 310                 315                 320

Leu Asn His Gln Gly Arg Pro Ser Gly Asp Ala Phe Ile Gln Met Lys
                325                 330                 335

Ser Ala Asp Arg Ala Phe Met Ala Ala Gln Lys Cys His Lys Lys Lys
            340                 345                 350

His Glu Gly Gln Ile Cys
        355

<210>  14
<211>  133
<212>  PRT
<213>  Homo sapiens

<400>  14

Met Leu Ser Pro Glu Ala Glu Arg Val Leu Arg Tyr Leu Val Glu Val

1               5                   10                  15

Glu Glu Leu Ala Glu Glu Val Leu Ala Asp Lys Arg Gln Ile Val Asp
            20                  25                  30

Leu Asp Thr Lys Arg Asn Gln Asn Arg Glu Gly Leu Arg Ala Leu Gln
        35                  40                  45

Lys Asp Leu Ser Leu Ser Glu Asp Val Met Val Cys Phe Gly Asn Met
    50                  55                  60

Phe Ile Lys Met Pro His Pro Glu Thr Lys Glu Met Ile Glu Lys Asp
65                  70                  75                  80

Gln Asp His Leu Asp Lys Glu Ile Glu Lys Leu Arg Lys Gln Leu Lys
                85                  90                  95

Val Lys Val Asn Arg Leu Phe Glu Ala Gln Gly Lys Pro Glu Leu Lys
            100                 105                 110

Gly Phe Asn Leu Asn Pro Leu Asn Gln Asp Glu Leu Lys Ala Leu Lys
        115                 120                 125

Val Ile Leu Lys Gly
    130

<210>  15
<211>  286
<212>  PRT
<213>  Homo sapiens

<400>  15

Met Ala Ala Pro Ala Pro Gly Ala Gly Ala Ala Ser Gly Gly Ala Gly
1               5                   10                  15

Cys Ser Gly Gly Gly Ala Gly Ala Gly Ala Gly Ser Gly Ser Gly Ala
            20                  25                  30

Ala Gly Ala Gly Gly Arg Leu Pro Ser Arg Val Leu Glu Leu Val Phe
        35                  40                  45

Ser Tyr Leu Glu Leu Ser Glu Leu Arg Ser Cys Ala Leu Val Cys Lys
    50                  55                  60

His Trp Tyr Arg Cys Leu His Gly Asp Glu Asn Ser Glu Val Trp Arg
65                  70                  75                  80

Ser Leu Cys Ala Arg Ser Leu Ala Glu Glu Ala Leu Arg Thr Asp Ile
                85                  90                  95

Leu Cys Asn Leu Pro Ser Tyr Lys Ala Lys Ile Arg Ala Phe Gln His
            100                 105                 110

Ala Phe Ser Thr Asn Asp Cys Ser Arg Asn Val Tyr Ile Lys Lys Asn
        115                 120                 125

Gly Phe Thr Leu His Arg Asn Pro Ile Ala Gln Ser Thr Asp Gly Ala
    130                 135                 140

Arg Thr Lys Ile Gly Phe Ser Glu Gly Arg His Ala Trp Glu Val Trp
145                 150                 155                 160

Trp Glu Gly Pro Leu Gly Thr Val Ala Val Ile Gly Ile Ala Thr Lys
                165                 170                 175

Arg Ala Pro Met Gln Cys Gln Gly Tyr Val Ala Leu Leu Gly Ser Asp
            180                 185                 190

Asp Gln Ser Trp Gly Trp Asn Leu Val Asp Asn Asn Leu Leu His Asn
        195                 200                 205

Gly Glu Val Asn Gly Ser Phe Pro Gln Cys Asn Asn Ala Pro Lys Tyr
    210                 215                 220

Gln Ile Gly Glu Arg Ile Arg Val Ile Leu Asp Met Glu Asp Lys Thr
225                 230                 235                 240

Leu Ala Phe Glu Arg Gly Tyr Glu Phe Leu Gly Val Ala Phe Arg Gly
                245                 250                 255

Leu Pro Lys Val Cys Leu Tyr Pro Ala Val Ser Ala Val Tyr Gly Asn
            260                 265                 270

Thr Glu Val Thr Leu Val Tyr Leu Gly Lys Pro Leu Asp Gly
        275                 280                 285

<210>  16
<211>  717
<212>  PRT
<213>  Homo sapiens

<400>  16

Met Thr Pro Pro Pro Pro Pro Pro Pro Pro Pro Gly Pro Asp Pro Ala
1               5                   10                  15

Ala Asp Pro Ala Ala Asp Pro Cys Pro Trp Pro Gly Ser Leu Val Val
            20                  25                  30

Leu Phe Gly Ala Thr Ala Gly Ala Leu Gly Arg Asp Leu Gly Ser Asp
        35                  40                  45

Glu Thr Asp Leu Ile Leu Leu Val Trp Gln Val Val Glu Pro Arg Ser
    50                  55                  60

Arg Gln Val Gly Thr Leu His Lys Ser Leu Val Arg Ala Glu Ala Ala

65                  70                  75                  80

Ala Leu Ser Thr Gln Cys Arg Glu Ala Ser Gly Leu Ser Ala Asp Ser
                85                  90                  95

Leu Ala Arg Ala Glu Pro Leu Asp Lys Val Leu Gln Gln Phe Ser Gln
            100                 105                 110

Leu Val Asn Gly Asp Val Ala Leu Leu Gly Gly Gly Pro Tyr Met Leu
        115                 120                 125

Cys Thr Asp Gly Gln Gln Leu Leu Arg Gln Val Leu His Pro Glu Ala
    130                 135                 140

Ser Arg Lys Asn Leu Val Leu Pro Asp Met Phe Phe Ser Phe Tyr Asp
145                 150                 155                 160

Leu Arg Arg Glu Phe His Met Gln His Pro Ser Thr Cys Pro Ala Arg
                165                 170                 175

Asp Leu Thr Val Ala Thr Met Ala Gln Gly Leu Gly Leu Glu Thr Asp
            180                 185                 190

Ala Thr Glu Asp Asp Phe Gly Val Trp Glu Val Lys Thr Met Val Ala
        195                 200                 205

Val Ile Leu His Leu Leu Lys Glu Pro Ser Ser Gln Leu Phe Ser Lys
    210                 215                 220

Pro Glu Val Ile Lys Gln Lys Tyr Glu Thr Gly Pro Cys Ser Lys Ala
225                 230                 235                 240

Asp Val Val Asp Ser Glu Thr Val Val Arg Ala Arg Gly Leu Pro Trp
                245                 250                 255

Gln Ser Ser Asp Gln Asp Val Ala Arg Phe Phe Lys Gly Leu Asn Val
            260                 265                 270

Ala Arg Gly Gly Val Ala Leu Cys Leu Asn Ala Gln Gly Arg Arg Asn
        275                 280                 285

Gly Glu Ala Leu Ile Arg Phe Val Asp Ser Glu Gln Arg Asp Leu Ala
    290                 295                 300

Leu Gln Arg His Lys His His Met Gly Val Arg Tyr Ile Glu Val Tyr
305                 310                 315                 320

Lys Ala Thr Gly Glu Glu Phe Val Lys Ile Ala Gly Gly Thr Ser Leu
                325                 330                 335

Glu Val Ala Arg Phe Leu Ser Arg Glu Asp Gln Val Ile Leu Arg Leu
            340                 345                 350

Arg Gly Leu Pro Phe Ser Ala Gly Pro Thr Asp Val Leu Gly Phe Leu
        355                 360                 365

Gly Pro Glu Cys Pro Val Thr Gly Gly Thr Glu Gly Leu Leu Phe Val
    370                 375                 380

Arg His Pro Asp Gly Arg Pro Thr Gly Asp Ala Phe Ala Leu Phe Ala
385                 390                 395                 400

Cys Glu Glu Leu Ala Gln Ala Ala Leu Arg Arg His Lys Gly Met Leu
                405                 410                 415

Gly Lys Arg Tyr Ile Glu Leu Phe Arg Ser Thr Ala Ala Glu Val Gln
            420                 425                 430

Gln Val Leu Asn Arg Tyr Ala Ser Gly Pro Leu Leu Pro Thr Leu Thr
        435                 440                 445

Ala Pro Leu Leu Pro Ile Pro Phe Pro Leu Ala Pro Gly Thr Gly Arg
    450                 455                 460

Asp Cys Val Arg Leu Arg Gly Leu Pro Tyr Thr Ala Thr Ile Glu Asp
465                 470                 475                 480

Ile Leu Ser Phe Leu Gly Glu Ala Ala Ala Asp Ile Arg Pro His Gly
                485                 490                 495

Val His Met Val Leu Asn Gln Gln Gly Arg Pro Ser Gly Asp Ala Phe
            500                 505                 510

Ile Gln Met Thr Ser Ala Glu Arg Ala Leu Ala Ala Ala Gln Arg Cys
        515                 520                 525

His Lys Lys Val Met Lys Glu Arg Tyr Val Glu Val Val Pro Cys Ser
    530                 535                 540

Thr Glu Glu Met Ser Arg Val Leu Met Gly Gly Thr Leu Gly Arg Ser
545                 550                 555                 560

Gly Met Ser Pro Pro Pro Cys Lys Leu Pro Cys Leu Ser Pro Pro Thr
                565                 570                 575

Tyr Thr Thr Phe Gln Ala Thr Pro Thr Leu Ile Pro Thr Glu Thr Ala
            580                 585                 590

Ala Leu Tyr Pro Ser Ser Ala Leu Leu Pro Ala Ala Arg Val Pro Ala
        595                 600                 605

Ala Pro Thr Pro Val Ala Tyr Tyr Pro Gly Pro Ala Thr Gln Leu Tyr
    610                 615                 620

Leu Asn Tyr Thr Ala Tyr Tyr Pro Ser Pro Pro Val Ser Pro Thr Thr
625                 630                 635                 640

Val Gly Tyr Leu Thr Thr Pro Thr Ala Ala Leu Ala Ser Ala Pro Thr
                645                 650                 655

Ser Val Leu Ser Gln Ser Gly Ala Leu Val Arg Met Gln Gly Val Pro
            660                 665                 670

Tyr Thr Ala Gly Met Lys Asp Leu Leu Ser Val Phe Gln Ala Tyr Gln
        675                 680                 685

Leu Pro Ala Asp Asp Tyr Thr Ser Leu Met Pro Val Gly Asp Pro Pro
    690                 695                 700

Arg Thr Val Leu Gln Ala Pro Lys Glu Trp Val Cys Leu
705                 710                 715

<210>  17
<211>  166
<212>  PRT
<213>  Homo sapiens

<400>  17

Met Val Ala Asn Cys Gly Ala Ala Gly Thr Gly Gly Arg Arg Ser Cys
1               5                   10                  15

Ala Ala Ser Ser Val Lys Arg Ser Ala Ser Arg Arg Lys His Asn Ala
            20                  25                  30

Val Arg Asn Ser Asp Val Lys Ala Lys Gly Arg Lys Ser Arg Asn Thr
        35                  40                  45

Tyr Thr Thr Asp Gly Gly Gly Asp Gly Asp Ala Ala Ser Gly Ala Ala
    50                  55                  60

Cys Asn Asn Ala Thr Tyr Val Val Trp Asn Asp Met Asn Arg Ser Gly
65                  70                  75                  80

Arg Val Arg Tyr Asp Gly Arg Ser Lys Ala Arg Arg Arg Gly Gly Arg
                85                  90                  95

Gly Asp Arg Arg Arg Asp Ala Tyr Thr Arg Cys Arg Ser Arg Arg Arg
            100                 105                 110

Ala Val Lys Arg Ser Arg Met Thr Thr Trp Arg Arg Ser Val Ser Ala
        115                 120                 125

Val Tyr Thr Ala Met Asp Asn Ser Arg His Ala Val Cys Tyr Met Asp
    130                 135                 140

Ser Ala Ser Ala Asp Gly Lys Arg Met Lys Val Ser Asn Arg His Asp

145                 150                 155                 160

Gly Ser Ala Tyr His Ser
                165

<210>  18
<211>  456
<212>  PRT
<213>  Homo sapiens

<400>  18

Met Asn Ser Ser Asp Glu Glu Lys Gln Leu Gln Leu Ile Thr Ser Leu
1               5                   10                  15

Lys Glu Gln Ala Ile Gly Glu Tyr Glu Asp Leu Arg Ala Glu Asn Gln
            20                  25                  30

Lys Thr Lys Glu Lys Cys Asp Lys Ile Arg Gln Glu Arg Asp Glu Ala
        35                  40                  45

Val Lys Lys Leu Glu Glu Phe Gln Lys Ile Ser His Met Val Ile Glu
    50                  55                  60

Glu Val Asn Phe Met Gln Asn His Leu Glu Ile Glu Lys Thr Cys Arg
65                  70                  75                  80

Glu Ser Ala Glu Ala Leu Ala Thr Lys Leu Asn Lys Glu Asn Lys Thr
                85                  90                  95

Leu Lys Arg Ile Ser Met Leu Tyr Met Ala Lys Leu Gly Pro Asp Val
            100                 105                 110

Ile Thr Glu Glu Ile Asn Ile Asp Asp Glu Asp Ser Thr Thr Asp Thr
        115                 120                 125

Asp Gly Ala Ala Glu Thr Cys Val Ser Val Gln Cys Gln Lys Gln Ile
    130                 135                 140

Lys Glu Leu Arg Asp Gln Ile Val Ser Val Gln Glu Glu Lys Lys Ile
145                 150                 155                 160

Leu Ala Ile Glu Leu Glu Asn Leu Lys Ser Lys Leu Val Glu Val Ile
                165                 170                 175

Glu Glu Val Asn Lys Val Lys Gln Glu Lys Thr Val Leu Asn Ser Glu
            180                 185                 190

Val Leu Glu Gln Arg Lys Val Leu Glu Lys Cys Asn Arg Val Ser Met
        195                 200                 205

Leu Ala Val Glu Glu Tyr Glu Glu Met Gln Val Asn Leu Glu Leu Glu
    210                 215                 220

Lys Asp Leu Arg Lys Lys Ala Glu Ser Phe Ala Gln Glu Met Phe Ile
225                 230                 235                 240

Glu Gln Asn Lys Leu Lys Arg Gln Ser His Leu Leu Leu Gln Ser Ser
                245                 250                 255

Ile Pro Asp Gln Gln Leu Leu Lys Ala Leu Asp Glu Asn Ala Lys Leu
            260                 265                 270

Thr Gln Gln Leu Glu Glu Glu Arg Ile Gln His Gln Gln Lys Val Lys
        275                 280                 285

Glu Leu Glu Glu Gln Leu Glu Asn Glu Thr Leu His Lys Glu Ile His
    290                 295                 300

Asn Leu Lys Gln Gln Leu Glu Leu Leu Glu Glu Asp Lys Lys Glu Leu
305                 310                 315                 320

Glu Leu Lys Tyr Gln Asn Ser Glu Glu Lys Ala Arg Asn Leu Lys His
                325                 330                 335

Ser Val Asp Glu Leu Gln Lys Arg Val Asn Gln Ser Glu Asn Ser Val
            340                 345                 350

Pro Pro Pro Pro Pro Pro Pro Pro Pro Leu Pro Pro Pro Pro Pro Asn
        355                 360                 365

Pro Ile Arg Ser Leu Met Ser Met Ile Arg Lys Arg Ser His Pro Ser
    370                 375                 380

Gly Ser Gly Ala Lys Lys Glu Lys Ala Thr Gln Pro Glu Thr Thr Glu
385                 390                 395                 400

Glu Val Thr Asp Leu Lys Arg Gln Ala Val Glu Glu Met Met Asp Arg
                405                 410                 415

Ile Lys Lys Gly Val His Leu Arg Pro Val Asn Gln Thr Ala Arg Pro
            420                 425                 430

Lys Thr Lys Pro Glu Ser Ser Lys Gly Cys Glu Ser Ala Val Asp Glu
        435                 440                 445

Leu Lys Gly Ile Leu Ala Ser Gln
    450                 455

## Description

### Background of the invention

It has been widely accepted that carcinogenesis is a multistep process involving genetic and epigenetic changes that dysregulate molecular control of cell proliferation and differentiation (Balmain, 2003, Nat. Genet. 33, 238-244). The genetic changes can include activation of proto-oncogenes and/or the inactivation of tumor suppressor genes that can initiate tumorigenesis. Tumorprogression and Metastasis are also multi-stage processes by which tumor cells leave the site of a primary tumor, enter blood and lymph vessels, migrate to distant parts of the body and form novel foci of tumor growth. Metastasis is a major cause of mortality for cancer patients. Many studies on cancer metastasis have been conducted and several molecules participating in tumor cell invasion and metastasis have been identified and characterized. Among these molecules, some facilitate invasion and metastasis, e.g. laminin receptor, metalloproteinases, and CD44 ( Hojilla, 2003, Br. J. Cancer 89, 1817-1821; Marhaba, 2004, J. Mol. Histol. 35, 211-231).
Despite use of a number of histochemical, genetic, and immunological markers, clinicians still have a difficult time predicting which tumors will progress and will finally metastasize to other organs, or whether a patient has already developed early metastasis. Some patients are in need of adjuvant therapy to prevent recurrence and metastasis and others are not. Distinguishing between these subpopulations of patients is not straightforward. There is therefore a need for new markers for distinguishing between tumors of differing metastatic potential and for new molecular targets and new therapeutic treatment options. In addition, such markers could be useful to monitor a potential anti-tumor response of a patient's body upon treatment with an anti-cancer drug.

Modern drug development typically involves the elucidation of the molecular mechanism underlying a disease or a condition, the identification of candidate target molecules and the evaluation of said target molecules. It is obvious that the identification of a candidate target molecule is essential to such process. With the sequencing of the human genome and publishing of respective sequence data, in principle, all of the coding nucleic acids of man are available. However, a serious limitation to this data is that typically no annotation of the function of said sequence is given. Furthermore" the mere knowledge of a coding nucleic acid sequence is not sufficient to predict the polypeptide's function in vivo.

In order to utilize such aforementioned new markers, it is required to identify the molecular basis of these markers based on their gene nucleotide and protein sequences. To define the profile of such genes whose expression is up-regulated during progression from a non metastasizing to metastatic cancer competence, initially rat tumor progression models were used for the identification of the markers presented in this invention. Here, instead of starting directly from human tumor material, it was chosen to analyze precisely defined clonal rodent tumor cell lines in a first differential gene sequence expression analysis. The utilization of such well characterized tumor cell lines offers the advantage that they often exhibit a reproducible metastatic or nonmetastatic phenotype that can be retested at any stage of the analysis. Moreover, tumor cell lines are accessible to genetic manipulation and functional tests in experimental animals. Rat tumor cells have the advantage of being able to be passaged in syngeneic animals, whereas human tumor cells have to be passaged in the rather artificial setting of an immunodeficient host. Furthermore, the cross species homology between rodent and human sequences creates the opportunity for the subsequent isolation of human homologues of such candidate tumor progression genes, hereafter referred to as "cancer antigens", and evaluation of their expression in primary human tumor material.
For the above mentioned intended molecular comparison of gene expression differences, two rat carcinoma models were used. The first model represents a rat pancreatic adenocarcinoma model which comprises several clones that differ in their metastatic potential *in vivo* and have been derived from a common primary tumor (Matzku, 1983, Cancer Research 49, 1294-1299). For example, BSp73-1AS cells form primary tumors that do not metastasize, whereas BSp73-ASML cells are highly metastatic and, after s.c. injection into host animals, disseminate via the lymphatic system to finally colonize the lungs. The second system, the rat mammary adenocarcinoma cell system 13762NF (Neri, 1981, Int. J. Cancer 28, 731-738), is composed of a number of cell lines derived from a parental mammary tumor and its corresponding spontaneous lung and lymph node metastases. For example, the cell line MTPa has been reported to be nonmetastatic *in vivo* in syngeneic animals, whereas the related MTLY cells are highly metastatic, giving rise to multiple metastases in the lymph nodes and lungs (Neri, 1981, Int J. Cancer 28, 731-738).
These systems guarantee a high reproducibility of the cellular metastatic potentials and provide a reproducible and easy access to cellular material. Thus, a high standard of quality and quantity of the critical starting material is warranted. The metastatic and the non-metastatic material is highly related, a relationship which cannot be reached using human primary or secondary tumors or human tumor derived cell lines as frequently employed in other studies.

In order to identify gene sequences - cancer antigens - in these systems which are stronger expressed in cells displaying high metastatic potential in comparison to related cells with a lower metastatic potential, transcripts of the non-metastatic cell line were subtracted from those of the metastatic cells via the Subtractive Suppression Hybridization (SSH Analysis) (Nestl, 2001, Cancer Research 61, 1569-1577) technology. For this purpose, RNA was isolated from the metastatic (tester population) and non-metastatic cells (driver population), cDNA was then generated and digested to get smaller, suitably sized pieces of DNA. Tester cDNA was divided into two portions and each was ligated with a different adaptor. Each tester sample was then hybridized with an excess of driver cDNA. Only DNA fragments specifically present in the tester sample (derived from the metastatic cells) remained single stranded. The primary hybridization samples were then mixed and hybridized again. Now, only the remaining equalized and subtracted single strand tester cDNAs are able to reassociate and form hybrids with two different adaptors. Those fragments with two different adaptor ends could then be amplified by PCR and transferred into suitable vector systems for further analysis. Therefore, only the transcripts specifically expressed in metastatic cells are amplified whereas the amplification of transcripts present in both populations is suppressed (Diatschenko , 1996, Proc. Natl. Acad. Sci. 93, 6025-6030).

Using this analysis, 981 differentially expressed cDNA clones from these rat systems were isolated, which after analysis using sequence blast and clustering analysis bioinformatics tools equated to 229 individual rat sequences. Of those, 189 could subsequently be transferred to human sequences utilizing human gene sequence data banks and advanced bioinformatics analysis. Of these 189 gene sequences, 144 represented human proteins of known function, and 45 coded for human proteins of unknown function or hypothetical proteins.

To further characterize these sequences in respect to their biological connection to the process of tumor progression and metastasis formation, and to verify their suitability as cancer antigens or as metastasis markers, several additional analytical examinations were applied. Initially, all sequences of which a connection to metastasis formation or tumor progression has previously already been reported were sorted out. Secondly, the expression of the remaining gene sequences was analyzed in human tumor samples, and thirdly, the functional involvement of these sequences in cellular metastatic processes was analyzed by (i) overexpression of the gene sequences, and (ii) by RNA interference studies in suitable test systems. This analytical process revealed 9 previously not described new cancer antigens or metastasis markers which are useful as diagnostic tools or which may serve as new target structures to create new therapeutic treatment options for cancer patients, and which are one subject of this invention.

This invention relates to these sequences and their role in cellular process of increased metastasizing potential since their expression is found to be increased parallel to the increase in this metastasising potential. Thus, these gene sequences and the proteins encoded thereof may alone or in combination of two or more of these sequences contribute to the establishment of, or the progression to a more metastatic phenotype. With this respect, the pro-metastatic activities of a given sequence or the respectively encoded polypeptide may be enhanced when these activities are combined with the pro-metastatic activities of another sequence or polypeptide encoded thereof. Thus, the acquisition of pro-metastatic activities through enhanced expression of such individual sequences and polypeptides must therefore be regarded as part of a process in which a cell step wise acquires an increasing metastatic phenotype, whereas such a single step is defined by the acquisition of the upregulated expression of one of these sequences. This implies that these sequences are functionally linked to each other by each adding one step to the process of cellular metastatic potential, and these sequences should therefore be regarded as all being part of the same process, and therefore the same underlying invention which is presented herein.

A first aspect of the present invention is a method for diagnosing a disease or condition, or a susceptibility to a disease or condition, comprising the step of determining the expression, activity or mutations of at least one polynucleotide or expression product thereof in a biological sample from a (first) subject, wherein said at least one polynucleotide comprises
(i) a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9 and the corresponding RNA sequences,
(ii) a sequence complementary to any one of the sequences under (i), or
(iii) a variant sequence of any one of the sequences under (i) or (ii).

The subject from which the biological sample was obtained may be a patient having the disease or condition, or an individual not affected by the disease or condition. In the latter case, the subject may be an individual suspected of having the disease or condition. Usually, the subject is a human.

The biological sample may be derived from or contain a body liquid obtained from said subject, for example blood or cerebrospinal fluid. In a preferred embodiment, the biological sample contains tissue material obtained through biopsy. The tissue may be a tissue affected by the disease or condition, e.g. a solid tumor. A tissue affected by the disease or condition is a tissue which differs from the corresponding tissue from a healthy individual. The difference may be a difference in morphology, histology, gene expression, response to treatment, protein composition etc.

Usually, the sample has been processed to be in a condition suitable for the method of determining the expression, activity or mutations as detailed infra. The processing may include dilution, concentration, homogenization, extraction, precipitation, fixation, washing and/or permeabilization, etc. The processing may also include reverse transcription and/or amplification of nucleic acids present in the sample.

The method of the invention may comprise only steps which are carried out *in vitro.* In that case, the step of obtaining the tissue material from the subject's body is not encompassed by the present invention. In another embodiment, the method further comprises the step of obtaining the biological sample from the subject's body.

The method comprises the step of determining the expression, activity or mutations of at least one polynucleotide or expression product thereof in a biological sample. The phrase "determining the expression" as used herein preferably means "determining the expression level". The expression or expression level correlates with the amount of polynucleotide or expression product thereof in the sample. The phrase "determining the expression of polynucleotide or expression product in the biological sample" includes or consists of determining the presence and/or amount of said at least one polynucleotide or expression product thereof. As used herein, the phrase "determining the mutations" means determining the presence or absence of one or more mutations in the nucleotide sequence of said at least one polynucleotide in said biological sample. It is preferred that mutations with respect to any one of the sequences SEQ ID NO:1 through 9 are determined.

The term "polynucleotide(s)" generally refers to any polyribonucleotide or polydeoxyribonucleotide that may be RNA or DNA. The polynucleotide may be single- or double-stranded. The polynucleotide in accordance with the diagnostic method of this invention may have a sequence as shown in any one of SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO:9. In addition, the polynucleotide may have a sequence which is a variant of these sequences. The variant may be a sequence having one or more additions, substitutions, and/or deletions of one or more nucleotides such as an allelic variant or single nucleotide polymorphisms of the above sequences. The variant may have an identity of at least 80%, preferably of at least 85%, more preferably of at least 90%, even more preferably of at least 95%, most preferably of at least 99% to any one of the sequences SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO:9. The percent identity or conservation may be determined by the algorithm of Wilbur and Lipman, Proc. Natl. Acad. Sci. USA 80; 726-730 (1983) which is embodied in the MegAlign program (DNA Star), using a k-tuple of 3 and a gap penalty of 3. Alternatively the algorithm of Myers and Miller, CABIOS (1989), which is embodied in the ALIGN program (version 2.0) or its equivalent, using a gap length penalty of 12 and a gap penalty of 3 where such parameters are required. All other parameters are set to their default positions. Access to ALIGN is readily available (see, e.g., http://www2.igh.cnrs.fr/bin/alignguess.cgi on the Internet).

The variant may be a polynucleotide which hybridizes to any one of the sequences SEQ ID NO:1 through 9, preferably under stringent conditions. A specific example of stringent hybridization conditions is incubation at 42°C for 16 hours in a solution comprising: 50% formamide, 5xSSC (150 mM NaCl, 15 mM trisodium citrate), 50 mM sodium phosphate (pH7.6), 5xDenhardt's solution, 10% dextran sulfate, and 20 µg/ml of denatured, sheared salmon sperm DNA, followed by washing the hybridization support in 0.1xSSC at about 65°C. Hybridization and wash conditions are well known and exemplified in Sambrook, et al., Molecular Cloning: A Laboratory Manual, Second Edition, Cold Spring Harbor, N. Y., (1989), particularly Chapter 11 therein. Alternative hybridization conditions are described infra with respect to solid supports.

In the variant 1 to 20, preferably 1 to 10, more preferably 1 to 5, most preferably 1, 2 or 3 nucleotides may be added, substituted or inserted with respect to any one of the sequences as shown in SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO:9. The variants further include fragments of SEQ ID NO:1 through 9. The fragments may comprise at least 100, preferably at least 500, more preferably at least 1000 contiguous nucleotides of any one of SEQ ID NO:1 through 9. Most preferably the fragment has a length such that less than 100, or less than 50, or less than 25 nucleotides are missing with respect to any one of SEQ ID NO:1 through 9.

Alternatively, the polynucleotide may have the corresponding RNA sequence. The sequence of the polynucleotide may also be complementary to any one of the above sequences.

Preferably, the polynucleotide in accordance the diagnostic method of this invention comprises a sequence encoding a polypeptide having a sequence selected from the group consisting of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO:18.

The expression product of said polynucleotide usually is a polypeptide encoded by any one of the above polynucleotides. Preferably, the polypeptide comprises a sequence selected from the group consisting of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO:18. The polypeptide may be a variant of any one of SEQ ID NO:10-18. For example, the amino acid sequence of the polypeptide may have an identity of at least 80%, preferably of at least 85%, more preferably of at least 90%, even more preferably of at least 95%, most preferably of at least 98% to any one of the sequences SEQ ID NO:10-18. The identity is to be understood as identity over the entire length of the polypeptide. The percent identity or conservation may be determined by the algorithm of Wilbur and Lipman, Proc. Natl. Acad. Sci. USA 80; 726-730 (1983) which is embodied in the MegAlign program (DNA Star), using a k-tuple of 3 and a gap penalty of 3. Alternatively the algorithm of Myers and Miller, CABIOS (1989), which is embodied in the ALIGN program (version 2.0) or its equivalent, using a gap length penalty of 12 and a gap penalty of 3 where such parameters are required. All other parameters are set to their default positions. Access to ALIGN is readily available (see, e.g., http://www2.igh.cnrs.fr/bin/align-guess.cgi on the Internet).

In the variant 1 to 10, preferably 1 to 5, more preferably 1 to 4, most preferably 1, 2 or 3 amino acids may be added, substituted or inserted with respect to any one of the sequences as shown in SEQ ID NO:10 through 18. The variants further include fragments of SEQ ID NO:10 through 18. The fragments may comprise at least 50, preferably at least 100, more preferably at least 500 contiguous amino acids of any one of SEQ ID NO:10 through 18. Most preferably the fragment has a length such that less than 50, or less than 30, or less than 15 amino acids are missing with respect to any one of SEQ ID NO:10 through 18.

In some embodiments, the variant polynucleotides and/or the polypeptides they encode retain at least one activity or function of the unmodified polynucleotide and/or the polypeptide, such as hybridization, antibody binding, etc.

In one embodiment, the method comprises the use of nucleic acid hybridization technology for determining the amount or presence of the polynucleotide in the sample, or for determining the mutations in the polynucleotide. Hybridization methods for nucleic acids are well known to those of ordinary skill in the art (see, e.g. Molecular Cloning: A Laboratory Manual, J. Sambrook, et al., eds., Second Edition, Cold Spring Harbor Laboratory Press, Cold Spring Harbor, N.Y., 1989, or Current Protocols in Molecular Biology, F. M. Ausubel, et al., eds., John Wiley & Sons, Inc., New York).

According to the invention, standard hybridization techniques of microarray technology may be utilized to assess polynucleotide expression. Microarray technology, which is also known as DNA chip technology, gene chip technology, and solid-phase nucleic acid array technology, is well known to the skilled person and is based on, but not limited to, obtaining an array of identified nucleic acid probes on a fixed support, labeling target molecules with reporter molecules (e.g., radioactive, chemiluminescent, or fluorescent tags), hybridizing target nucleic acids to the probes, and evaluating target-probe hybridization. A probe with a nucleic acid sequence that perfectly matches the target sequence will, in general, result in detection of a stronger reporter-molecule signal than will probes with less perfect matches. Many components and techniques utilized in nucleic acid microarray technology are presented in "The Chipping Forecast", Nature Genetics, Vol.21, January 1999.

According to the present invention, microarray supports may include but are not limited to glass, silica, aluminosilicates, borosilicates, plastics, metal oxides, nitrocellulose, or nylon. The use of a glass support is preferred. According to the invention, probes are selected from the group of polynucleotides including, but not limited to: DNA, genomic DNA, cDNA, and oligonucleotides; and may be natural or synthetic. Oligonucleotide probes preferably are 20 to 25-mer oligonucleotides and DNA/cDNA probes preferably are 500 to 5000 bases in length, although other lengths may be used. Appropriate probe length may be determined by the skilled person by known procedures. Probes may be purified to remove contaminants using standard methods known to those of ordinary skill in the art such as gel filtration or precipitation. Accordingly, the polynucleotide immobilized to the solid support is preferably an isolated polynucleotide. The term "isolated" polynucleotide refers to a polynucleotide that is substantially free from other nucleic acid sequences, such as and not limited to other chromosomal and extrachromosomal DNA and RNA. Isolated polynucleotides may be purified from a host cell. Conventional nucleic acid purification methods known to skilled artisans may be used to obtain isolated polynucleotides. The term also includes recombinant polynucleotides and chemically synthesized polynucleotides.

In one embodiment, probes are synthesized directly on the support in a predetermined grid pattern using methods such as light-directed chemical synthesis, photochemical deprotection, or delivery of nucleotide precursors to the support and subsequent probe production. In embodiments of the invention one or more control polynucleotides are attached to the support. Control polynucleotides may include but are not limited to cDNA of genes such as housekeeping genes or fragments thereof.

The solid support comprises at least one polynucleotide immobilized on or attached to its surface, wherein said polynucleotide hybridizes with a polynucleotide as described supra, preferably under stringent conditions. Suitable hybridization conditions are for example described in the manufacturer's instructions of "DIG Easy Hyb Granules" (Roche Diagnostics GmbH, Germany, Cat. No. 1796895). These instructions are incorporated herein by reference. The hybridization conditions described in the following protocol may be used:
- Hybridizations are carried out using DIG Easy Hyb buffer (Roche Diagnostics, Cat. No. 1796895).
- Ten microliters of hybridization solution with probe is placed on the microarray and a coverslip carefully applied.
- The slide is placed in a hybridization chamber and incubated for 16 h incubation at 42°C.
- The coverslips are removed in a container with 2 x SSC + 0.1 % SDS and the microarrays are washed for 15 min in 2 x SSC + 0.1 % SDS at 42°C followed by a 5 min wash in 0.1 x SSC + 0.1% SDS at 25°C followed by two short washes in 0.1 x SSC and 0.01 x SSC at 25°C, respectively.
- The microarrays are dried by centrifugation and can be stored at 4°C.

Preferably, the polynucleotide immobilized on the solid support has a sequence as shown in any one of SEQ ID NO:1 through 9; or a complement thereof; or a fragment thereof.

In one embodiment, preferred probes are sets of two or more of the nucleic acid molecules as defined. In a specific embodiment, at least 9 different isolated polynucleotides are immobilized on said solid support, and said 9 different isolated polynucleotides have the nucleotide sequences as shown in SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO:9, respectively, or the corresponding complementary sequences, or fragments thereof.

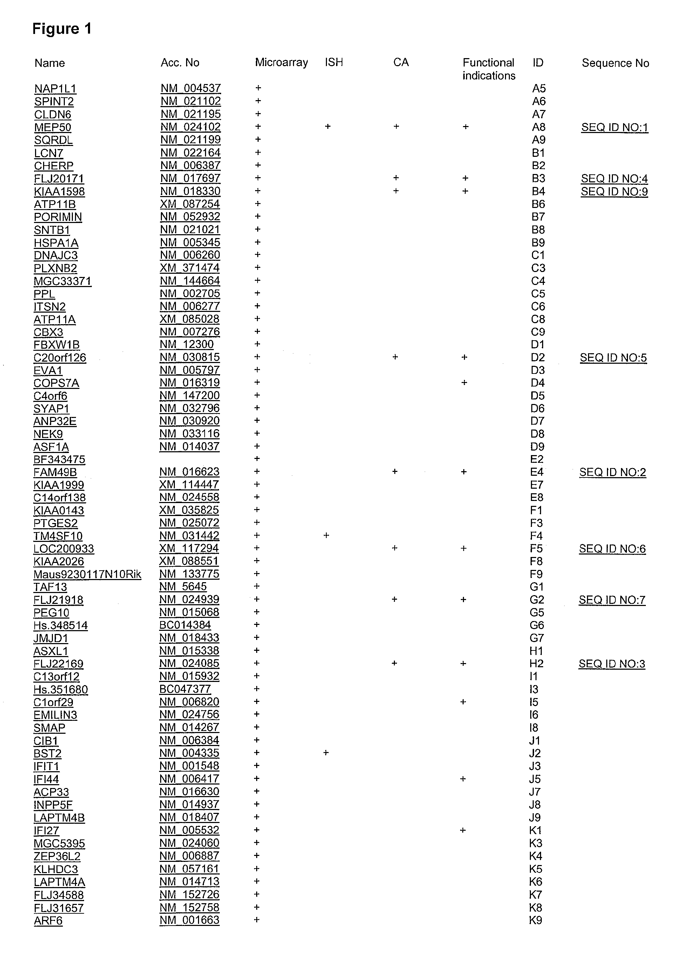

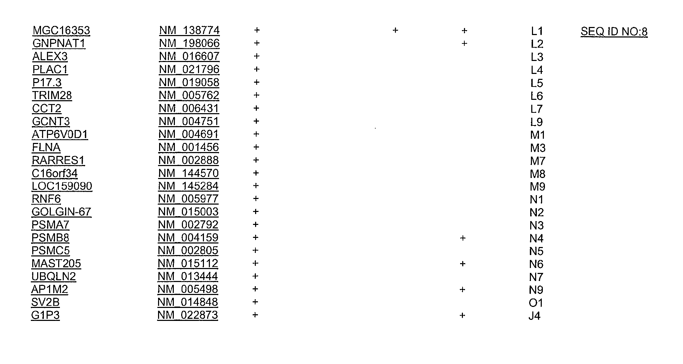

In another embodiment, at least 20 or at least 50 or at least 75 different isolated polynucleotides selected from the polynucleotides listed in Figure 1 are immobilized on said solid support. In a specific embodiment, at least 89 different isolated polynucleotides are immobilized on said solid support, and said at least 89 isolated polynucleotides have the nucleotide sequences as outlined in Figure 1. The nucleotide sequences of the polynucleotides as outlined in Figure 1 are defined by their name and/or accession number and are incorporated herein by reference.

In another embodiment, the method comprises utilizing an antibody directed against a polypeptide described hereinabove. Preferably, the polypeptide is selected from the group consisting of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO:18. The antibody may be polyclonal or monoclonal, with monoclonal antibodies being preferred. The antibody is preferably immunospecific for any one of the above polypeptides. The antibodies can be used to detect the polypeptide by any standard immunoassay technique including ELISA, immunoblotting (Western blotting), immunoprecipitation, BIACORE technology and the like, as will be appreciated by one of ordinary skill in the art.

The method of the invention usually further comprises the step of comparing said expression or activity determined as described supra and the expression or activity of said polynucleotide or expression product thereof in a second sample which was obtained from tissue which is not affected by said disease. For example, an increased expression or activity in said first sample compared to the expression or activity in said second sample may be diagnostic of the disease. The second sample may be derived from a second subject which is not affected by the disease. Alternatively, the second sample may be derived from the first subject, but from a different tissue than the first sample.

The disease may be a tumor disease or cancer. Preferably, the disease is any one of the following diseases and conditions: estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis, sarcomas, Kaposi's sarcoma, osteosarcoma, head and neck cancer, small cell lung carcinoma, non-small cell lung carcinoma, leukemias, lymphomas, or other blood cell cancers, ichtiosis, acne, acne vulgaris, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and obesity.

Most preferably, the disease is minimal residual disease or tumor metastasis.

The genes identified herein permit, inter alia, rapid screening of biological samples by nucleic acid microarray hybridization or protein expression technology to determine the expression of the specific genes and thereby to predict the outcome of the disease. Such screening is beneficial, for example, in selecting the course of treatment to provide to the patient, and to monitor the efficacy of a treatment.

Another aspect of this invention is a method for identifying compounds which modulate the expression or activity of any of the polynucleotides or expression products thereof as defined in any one of claims 1 to 3, comprising
(a) contacting a candidate compound with cells which express said polynucleotide or a polypeptide encoded thereby, or with cell membranes comprising said polypeptide, or respond to said polypeptide,
(b) determining the effect of said candidate compound on the expression, activity, cellular localization or structural condition of said polynucleotide or polypeptide, or determining a functional response of said cells.

The step of determining the effect may comprise comparing said expression, activity, cellular localization or structural condition of said polynucleotide or polypeptide with the expression, activity, cellular localization or structural condition of said polynucleotide or polypeptide in cells which were not contacted with the candidate compound. The method may further comprise comparing the viability of the cells which were contacted with the candidate compound and the viability of cells which were not contacted with the candidate compound.

The candidate compound may be selected if the expression of said polynucleotide or polypeptide in the cells which were contacted with the candidate compound is lower than in the cells which were not contacted with the candidate compound. In such case, the compound is capable of suppressing the expression of the polynucleotide or expression product thereof. One may further compare the viability of the cells which were contacted with the candidate compound and the viability of cells which were not contacted with the candidate compound.

The invention further concerns a compound identified by the above-described method, wherein said compound is a compound which antagonizes or agonizes any one of the polynuleotides or expression products thereof as defined in this application. Such compounds include but are not limited to antisense nucleic acid moleucles capable of suppressing the expression of any one of the polynucleotides or expression products thereof as defined herein.

Yet another aspect of the invention is a solid support on which at least one isolated polynucleotide is immobilized, wherein said isolated polynucleotide has
(i) a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, and fragments thereof;
(ii) a sequence complementary to any one of the sequences under (i); or
(iii) a sequence which is an allelic variant of any one of the sequences under (i) or (ii).

The solid support preferably has the form of a microarray or DNA chip. Other preferred embodiments of the solid support have been described hereinabove in connection with the diagnostic methods of the invention.

Yet another aspect of the invention is the use of a polynucleotide or polypeptide as defined herein for the diagnostic method, or of a compound identified by the screening method described above, in the manufacture of a medicament for the treatment or prevention of a disease associated with increased activity or expression of a polynucleotide or polypeptide as defined herein.

Diagnostic tools based on the newly identified cancer antigens are another subject of this invention, and include test systems to analyze expression of these sequences in tumors to predict the tumor's potential to progress and to develop metastasis. In addition, these tools can be used to examine a patient's body for the presence of micrometastases or minimal residual disease which may lead to improved decisions on further treatment modalities. In this respect, a test system applied could consist of cDNAs, comprising, e.g., the cancer antigen sequences, which are contained on a carrier system, such as being spotted on, e.g., glass slides (gene or cDNA chip) which subsequently would be analysed utilizing fluorescence labelled RNA samples - derived from patients - that are hybridized to these chips to investigate the expression patterns of several metastasis markers - including the cancer antigens - at the same time.

Therefore, the present invention relates to methods for the diagnosis or screening of a subject in need, e.g., a patient suffering from a disease, e.g., but not limited to cancer, which correlates with the expression of at least one of the cancer antigens of this invention, to test whether the subject displays an enhanced activity or expression of a polynucleotide or polypeptide. Such investigations could, e.g., give information about the presence of the metastatic potential of a patient's tumor cells, or whether a patient's body harbors minimal residual tumor disease. These investigations may comprise nucleic acid technologies, such as hybridisation methods using hybridisation samples derived from patient's normal or diseased tissues. Also, such processes may be useful to draw prognostic conclusions about about a patient's disease, or about a patient's response to a therapeutic treatment by monitoring of the clinical effectiveness of the treatment, and the correlation of the expression or activity of a cancer antigen (polynucleotide or polypeptide) of this invention. ,

Furthermore, since the genes or gene products coding for the cancer antigens of this invention could be causally involved in the progression of tumor diseases, these gene sequences or gene products encoded by those, may represent new target structures for the development of new drugs, including but not limited to anti-cancer drugs, and the subsequent therapeutic treatment of patients with these drugs.
Therefore, this invention also comprises methods for the treatment of a subject having the need to inhibit the activity or expression of a polynucleotide or polypeptide presented herein. Such treatment could comprise one or more of the following steps targeting the expression or function of a polynucleotide or polypeptide:
(a) administering to the subject a therapeutically effective amount of a compound which causes a decrease in the expression of a polynucleotide,
(b) administering to the subject a therapeutically effective amount of an antagonist to said polypeptide,
(c) administering to the subject a therapeutically effective amount of an agonist to said polypeptide,
(d) administering to the subject a nucleic acid molecule that inhibits the expression of the nucleotide sequence encoding said polypeptide,
(e) administering to the subject a polynucleotide or a nucleotide sequence complementary to said nucleotide sequence in a form so as to effect production of said thereof encoded polypeptide activity,
(f) administering to the subject a therapeutically effective amount of a polypeptide that competes with said polypeptide for its ligand, substrate, or receptor,
(g) administering to the subject a therapeutically effective amount of an antibody directed against said polypeptide.

This invention also comprises methods for the expression, production and/or functional analysis of specific polynucleotides and polypeptides. For this purpose, a polynucleotide covered by this invention should be defined as comprising a nucleotide sequence that has at least 80% identity over its entire length to any of the polynucleotide sequences described herein. More preferably, the identity is larger than 90%, and even more preferably, this identity is larger than 95%. A polypeptide covered by this invention should be defined as comprising at least 80% identity over its entire length to a polypeptide sequences described herein. More preferably, this identity is larger than 90%, and even more preferably, the identity is larger than 95%.

The methods therefore included in this invention cover the use of a DNA or RNA molecule comprising an expression system, wherein said expression system is capable of producing a polynucleotide or polypeptide encoded therefrom when said expression system is present in a compatible host cell. This host cell may be a eukaryotic or bacterial host cell, and it may be used for a process for producing a polynucleotide or polypeptide by transforming or transfecting it with an expression system such that the host cell, under appropriate culture conditions, produces the encoded polynucleotide or polypeptide.

This invention also covers methods for the identification and development of compounds, agonist or antagonists, which are capable of interfering with the expression or function of a polynucleotide or polypeptide described herein. Such methods may include the following steps:
(a) contacting a candidate compound with cells which express a polypeptide, or cell membranes expressing said polypeptide, or respond to said polypeptide; and
(b) observing the binding, or stimulation or inhibition of a functional response, or comparing the ability of the cells or cell membranes which were contacted with the candidate compound with the same cells or cell membranes which were not contacted with said polypeptide; or
(c) observing the cellular localization of the polypeptide after contacting it with the candidate compound with the cellular localization of the polypeptide without contacting it to the candidate compound; or
(d) contacting a candidate compound with a polypeptide and observe the activity or structural condition of a polypeptide and comparing it to the activity or structural condition of a polypeptide which is not contacted with the candidate compound.

Also the following steps may be used for the identification of such compounds:
(a) contacting a candidate compound with cells which express said polynucleotide, or respond to said polynucleotide; and
(b) observing the stimulation or inhibition of a functional response, or comparing the ability of the cells which were contacted with the candidate compound with the same cells which were not contacted with said polynucleotide.

The diagnostic and therapeutic methods of this invention may be useful for diseases selected from the group of estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis, sarcomas, Kaposi's sarcoma, osteosarcoma, head and neck cancer, small cell lung carcinoma, non-small cell lung carcinoma, leukemias, lymphomas, or other blood cell cancers, ichtiosis, acne, acne vulgaris, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity.

### Detailed description of the invention

The following examples further describe the invention:

### Example 1

### SEQ ID NO:1 (A8)

One rat cDNA clone, originally derived from the above described SSH analysis of the mammary tumor test system was used to establish the corresponding EST (Expressed Sequence Tag) cluster from rat EST databases. The nucleotide sequence identity within the cluster was over 96%. The consensus sequence of this cluster was used to run a blast (Basic Local Alignment Search Tool, http://www.ncbi.nlm.nih.gov/BLAST/) analysis against mouse gene sequence databases. A sequence identity of 89% was found with the mouse mRNA BC005755, which again showed a 89% identity on the nucleotide sequence level to the mRNAs of the human MEP50 gene sequence. The corresponding NCBI (National Center for Biotechnology Information) reference sequence (http://www.ncbi.nlm.nih.gov/RefSeq/) for this locus, NM_024102 has a length of 2428 nucleotides and codes for a protein of 342 amino acids. The gene MEP50 maps on chromosome 1.
MEP50 contains a G-protein beta WD-40 repeat according to a search with the database Pfam (Protein family alignment mutiple). Pfam is a large collection of protein multiple sequence alignments and profile hidden Markov models (Bateman, 2000, Nucleic Acids Res. 30, 276-280).
MEP50 also contains a Glycosyl hydrolases family 18 motif. MEP50 was shown to be part of the Methylosome (Friesen, 2002, J. Biol. Chem. 277, 8243-8247) that is involved in the assembly of snRNP. Interestingly MEP50 was also shown to interact with the phosphatase FCP1, the only Pol II Phosphatase isolated so far (Licciardo, 2003, Nucleic Acids Res. 31, 999-1005).

In figure 1, a summary of established data for SEQ ID NO:1 is presented.

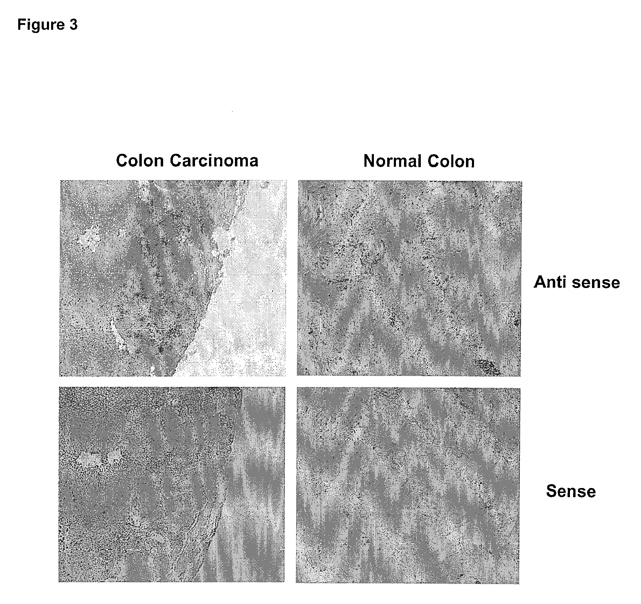

This sequence was shown to be differentially expressed in analysis of "In situ hybridization" (ISH) of matched human tumors (BioCat BA3, http://www.biocat.de), namely in cancers of the colon, stomach and breast, as exemplified in figure 3. Herein, data of ISH (In Situ Hybridization) experiments with Digoxygenin labelled RNA probes from the MEP50 locus (SEQ ID NO:1) are presented. RNA probes were generated with the DIG RNA labelling Kit from Roche according to the manufacturers instructions using a pOTB7 vector containing MEP50 (SEQ ID NO:1) sequences. Parraffin embedded tissue sections were deparaffinized, and postfixed in 4% paraformaldehyde. After incubation with proteinase K and washing, probes were denatured and hybridized to the slides at 65° over night. After several washes, the slides were subjected to a colorimetric assay using anti-digoxygenin antibodies (BM purple, Roche). Counterstain was done with H&E.

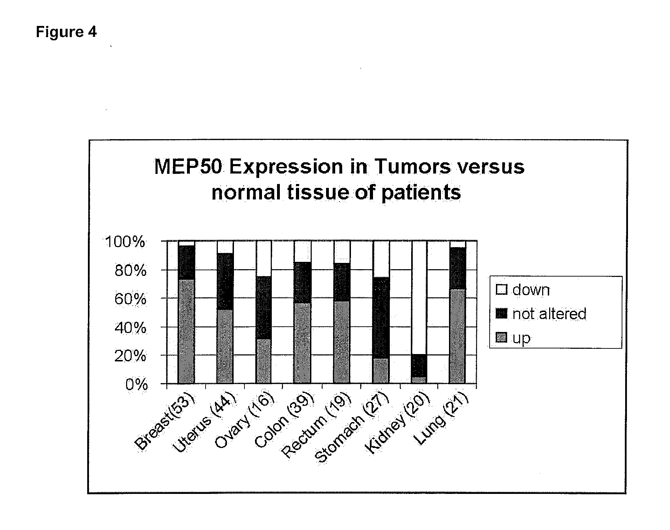

Tumor specific expression was further analyzed by hybridization experiments with Cancer Profiling Arrays (CA) from Clontech (http://www.bdbiosciences.com). The Cancer Profiling Arrays include normalized amplified cDNA from 241 tumor and corresponding normal tissues from individual patients, along with negative and positive controls, and cDNA from nine cancer cell lines. Here, overexpression was defined as upregulation of expression in the tumor probe versus expression in the normal probe of at least 1.5 fold. Percentage of upregulation in the tissues analysed is shown in figure 4. Herein, the cancer profiling expression analysis (CA) for SEQ ID NO:1 (MEP50) is presented. For this purpose, nylon filters carrying linear amplified cDNA from 241 tumor and corresponding normal tissues from individual patients (cancer filter arrays by Clontech) was hybridized with a radioactive labelled MEP50 (SEQ ID NO:1) cDNA. The signal of the tumor tissue was quantified by the phosphoimager analysis software AIDA (Fuji) and compared to the signal obtained by using corresponding hybridisation material of the normal tissue. The number of probe pairs per tissue is given in brackets. Definitions: A less than 0.7 fold expression of the sequence in the tumor sample is indicated as "DOWN", whereas "Up" means an at least more than 1.5 fold expression of the sequence in the tumor sample, each time compared to the expression in normal tissue samples. Percentages of Up and Down-regulations are shown in the columns. Numbers of tumor samples analysed are indicated in brackets next to the tumor tissue origin analysed (bottom). MEP50 shows significant upregulation (in more than 50% of analyzed pairs) in tissue samples derived from cancers of the breast, uterus, colon, rectum and lung.

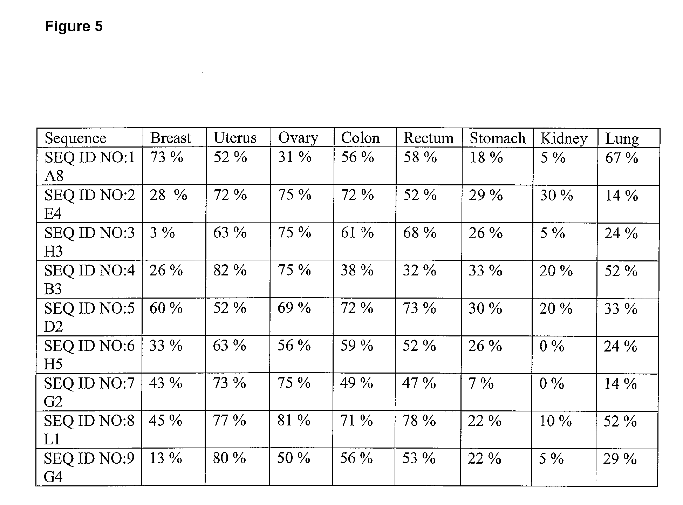

In figure 5, summary data for the cancer profiling expression analysis (CA) for SEQ ID NO:1-9 are presented according to the individual tumor tissue origin examined.

In order to functionally examine whether MEP50 could be causally involved in the process of tumor progression, MEP50 was transiently overexpressed or transiently downregulated by RNA interference in HEK-293T cells and subsequently potential resulting influences on tumor cell properties were assayed. Experiments shown in figures 6 and 7 demonstrate that overexpression of MEP50 leads to increased proliferation, its downregulation to decreased proliferation. These findings are further supported by analysis of HT29 colon carcinoma cells and T47D mammary carcinoma cells stably overexpressing MEP50. As shown in figure 8, MEP50 increases proliferation in both cell types. Thus, MEP50 is causally involved in regulating the proliferation capacity of tumor cells. MEP50 also affects the invasion potential of tumor cells. As shown in figure 9, HT29 colon carcinoma cells stably overexpressing MEP50 have a stronger capacity to invade into Matrigel (BD biosciences) which represents the basement membrane matrix.

In respect to these functional analysis, in detail the following tests have been performed:

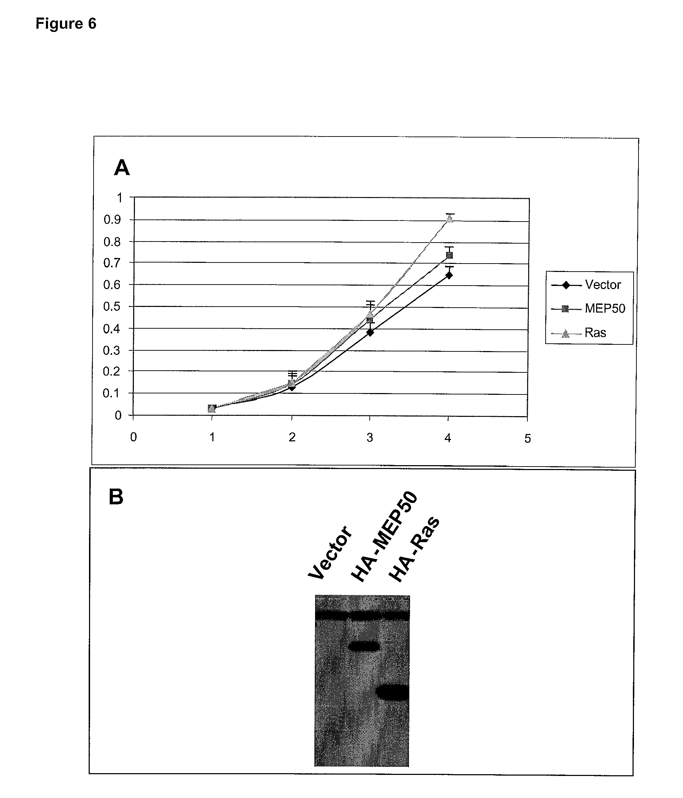

Figure 6: Data from proliferation assays with transiently transfected HEK-293T cells.
A: For these tests, MEP50 and Ras cDNAs were cloned into the mammalian expression vector pCDNA3.1 (Invitrogen). HEK-293T cells were then transfected with expression vectors for the indicated proteins using Lipofectamine (Invitrogen) according to the manufacturers instructions. 16h after transfection cells were seeded with 10,000 per well in triplicates in 96 well plates. From this time point on viable cells were determined every 24 h using the CellTiter Kit (Promega). The graphs represent the mean values of relative growth rates of three independent experiments. Note the increased growth rate upon expression of the Ras or MEP50 gene sequences.
B: Western Blot analysis testing the expression of the expressed proteins Ras and MEP50. For this purpose cells were lysed 24 h after transfection and lysates were subjected to gel electrophoresis and subsequent Western blotting with an anti-HA-antibody (12-CA-5). Note the clear expression of the proteins upon transfection of the expression contructs.

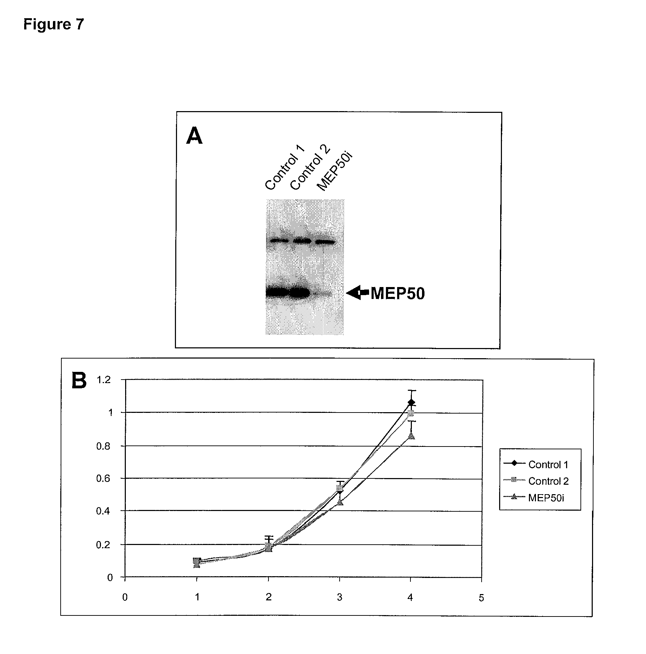

Figure 7: Proliferation assay using siRNA treated HEK-293T cells.
A: Analysis of the efficiency of the interference with the target protein expression, here tested on the protein level. HEK-293T cells were transiently transfected with an expression vector for MEP50 and the indicated siRNAs. 48 h after transfection cells were lysed and lysates were subjected to gelelectrophoresis and subsequent Western Blotting with an anti-HA-antibody (12-CA-5). Note that the expression of the target protein MEP50 could be strongly inhibited by using the siRNA targeting the MEP50 gene transcripts.
B: HEK-293T were transfected with the indicated siRNAs using Lipofectamine (Invitrogen) according to the manufacturers instructions. 16h after transfection cells were seeded with 10,000 cells per well in triplicates in 96 well plates. From this time point on viable cells were determined every 24 h using the non radioactive cell proliferation assay "Cell Titer 96" (Promega). The CellTiter 96 Assay is colorimetric method for determining the number of viable cells. It is composed of solutions of a novel tetrazolium compound [3-(4,5-dimethylthiazol-2-yl)-5-(3-carboxymethoxyphenyl)-2-(4-sulfophenyl)-2H]-tetrazolium, inner salt; MTS. MTS is bioreduced by cells into a formazan product that is soluble in tissue culture medium. The conversion of MTS into the aqueous soluble formazan product is accomplished by dehydrogenase enzymes found in metabolically active cells. The quantity of formazan product as measured by the amount of 490nm absorbance is directly proportional to the number of living cells in culture. The graphs represent mean values for absorbance at 490 nm of three independent experiments. Note the inhibition of proliferation upon downregulation of MEP50 expression using MEP50 specific siRNA molecules.

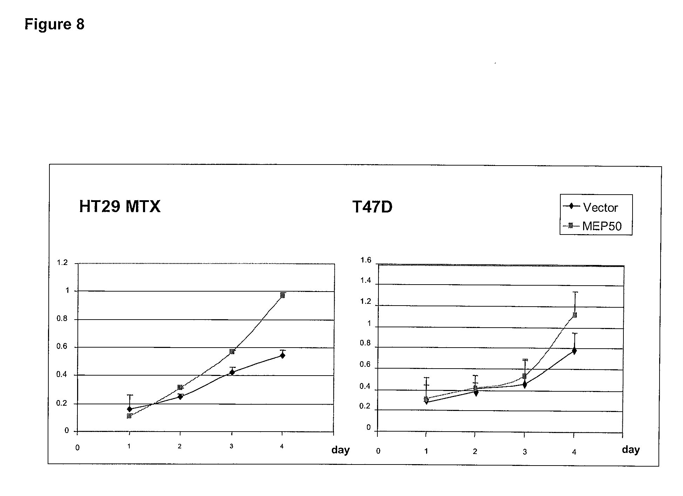

Figure 8: Proliferation assays using overexpression studies.
HT29 colon cancer cells and T47D breast cancer cells were stably transfected with either control vector pCDNA3.1 or a corresponding expression vector derived therof for MEP50. Stable mass cultures were selected using Neomycin. Cells were seeded with 10,000 cells per well in triplicates in 96 well plates. From this time point on viable cells were determined every 24 h using the CellTiter Kit (Promega). The graphs represent mean values for absorbance at 490 nm of three independent experiments. Note that the growth rate of both cell types is increased upon expression of MEP50.

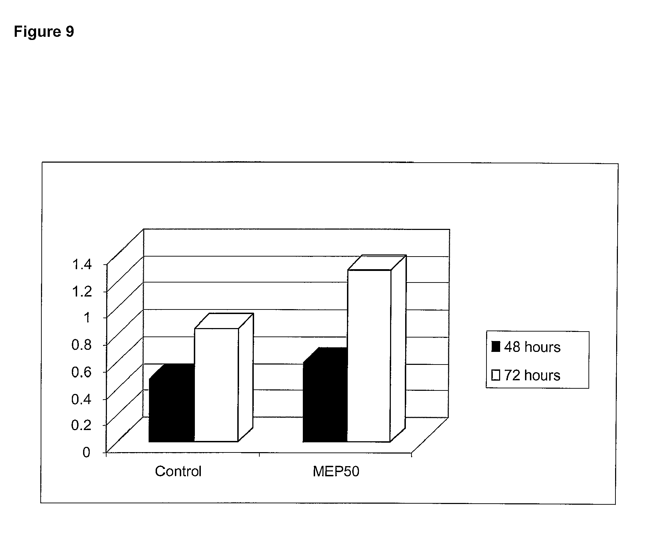

Figure 9: Invasion assay with stably transfected HT29 colon cancer cells. 10,0000 cells were seeded onto 2mg/ml Matrigel in the upper compartment of a transwell migration chamber (8 µm pores). The lower compartment contained medium with 10% serum. After 48 or 72 h cell density on the lower surface of the membrane was determined by staining with crystal violett and measuring the OD at 595 nm as a measurement of invasion through the Matrigel structure. Note that upon expression of the MEP50 gene the cells display an increased invasive character.

In summary, MEP50 shows upregulation in metastasizing tumor cells versus non metastasizing tumor cells, and also displays upregulated expression in various tumor tissues versus normal tissue samples. Moreover, MEP50 is functionally involved in processes involved in tumor progression like increased proliferation and invasion. Therefore, this sequence may particularly be useful for staging of human tumor diseases, as well as for decisions on prognosis and treatment modalities. Furthermore, the MEP50 gene and its gene products may be used as target structures to develop therapeutic anti-cancer drugs.

SEQ I D NO: 1 ( NM_024102)
cgt ccagt t t gagt ct aggt t ggagt t ggaaccgt ggagat gcggaaggaaaccccacccccccct agt gcccccggcggc
ccgggagt ggaat ct t cccccaaat gcgcccgcct gcat ggaacggcagt t ggaggct gcgcggt accggt ccgat gggg
cgct t ct cct cggggcct ccagcct gagt gggcgct gct gggccggct ccct ct ggct t t t t aaggacccct gt gccgcc
cccaacgaaggct t ct gct ccgccggagt ccaaacggaggct ggagt ggct gacct cact t gggt t ggggagagaggt at
t ct agt ggcct ccgat t caggt gct gt t gaat t gt gggaact agat gagaat gagacact t at t gt cagcaagt t ct gca
agt at gagcat gat gacat t gt gt ct acagt cagt gt ct t gagct ct ggcacacaagct gt cagt ggt agcaaagacat c
t gcat caaggt t t gggacct t gct cagcaggt ggt act gagt t cat accgagct cat gct gct caggt cact t gt gt t gc
t gcct ct cct cacaaggact ct gt gt t t ct t t cat gcagcgaggacaat agaat t t t act ct gggat acccgct gt ccca
agccagcat cacagat t ggct gcagt gcgcct ggct acct t cct acct cgct ggct t ggcat cct cagcaaagt gaagt c
t t t gt ct t t ggt gat gagaat gggacagt ct ccct t gt ggacaccaagagt acaagct gt gt cct gagct cagct gt aca
ct cccagt gt gt cact gggct ggt gt t ct ccccacacagt gt t ccct t cct ggcct ct ct cagt gaagact gct cact t g
ct gt gct ggact caagcct t t ct gagt t gt t t agaagccaagcccacagagact t t gt gagagat gcgact t ggt ccccg
ct caat cact ccct gct t accacagt gggct gggaccat caggt cgt ccaccacgt t gt gcccacagaacct ct cccagc
ccct ggacct gcaagt gt t act gagt agat t ggat t t aagacaaaaagcaagt cccccat gagt gt ccact t ct t t gccc
t gccct ct cagct t gt gagacaacacaggagcct t ct at agt at gt t gat at gct agat ct gt gccgt t aat aggcat cg
t ct ct cagcct gagggaggct ggat t ct gggt t cct gt agt cacagggaggaaaagct t t ct t aaaaat ggacat gt at g
t gcgt gt gagt gt gt gt gt agat t t at agt t t t t ggt agt ggcaggaat aaaaaaaat ccat cct acat ct t ccct aagc
act gcct ct ct ct caccccccaaaacaagt t gacgaaagggt t t t at gt agct gt ct at gaggaat t ggccgt gt ct ggg
t gggt t at gggat gt gggcat ccct gggt t ct t ggaagcagct ct t at gct act cat agagat gggat t gact t t at t t t
t t t at agt gct t aat t caccat t at gagaaat gct t ccagt cacaaaaat gcagcccagct cact ct gaggaagaagcag
gact t ggt acggt t t t acacaact cct t accat t aaact gaat cagaaat ccat t t t ct ggct gaat aaaaagt t t ggct
t gcct gt gt aat gcccact ccct t cccccct ggct ccct agt gat gggacat at at gagagagaagt gt t t t t ct at cat a
gacaccat aggggaaagt t t ggggat gaaggagagct t aaaggt gt t t caat t aagt t agaaaact gacacaggct gt t g
agaat t ct t t gccact t t t cccaccccaaaacagcat ggggcct gacat ct t ct gccct ggt cccct t t ct ct t gat gt g
gaaagt ct gaat gcagt at t t at agact t ct aaggt t t t aaaat ccagt at caagaagaaaat cagaaat act ggt t ggt
gaaat aaagagt t t aggcat t gt t ggcct gt ct t t t t t gaagcat gt gt gt t at gt gt agt t agat at at t t cact t at g
t gagt cat cat ggt gt t ggt ct t gt agcccat t at t t t t cct gt gct t ccccagct t cccaaagt agct agt t agaact t
aaggt aaat at t t at t ct t gggt t ggt ggagt ggat at t gccagt t aggagt cat ggat caat t act gat t at at t gaaa
gt aaat at aat caat t at gt act t t t gagct t t gcaggt t caat t t aggt aaaaat cacat t at gaaact gggaaagt ct
gaaggaat at gggcaaaat at t t ct cagt aaagct t ccat gct t caccct t gacat gat t accct t gagt aaaacat ggg
aat t t gt aaaaaaaaaaaaaaaaaaaa

SEQ ID NO: 10 - PROTEIN (NP_077007)
MRKETPPPLVPPAAREWNLPPNAPACMERQLEAARYRSDGALLLGASSLSGRCWAGSLWLFKDPCAAPNEGFCSAGVQTEAGV
ADLTWVGERGILVASDSGAVELWELDENETLIVSKFCKYEHDDIVSTVSVLSSGTQAVSGSKDICIKVWDLAQQVVLSSYRAH
AAQVTCVAASPHKDSVFLSCSEDNRILLWDTRCPKPASQIGCSAPGYLPTSLAWHPQQSEVFVFGDENGTVSLVDTKSTSCVL
SSAVHSQCVTGLVFSPHSVPFLASLSEDCSLAVLDSSLSELFRSQAHRDFVRDATWSPLNHSLLTTVGWDHQVVHHVVPTEPL
PAPGPASVTE

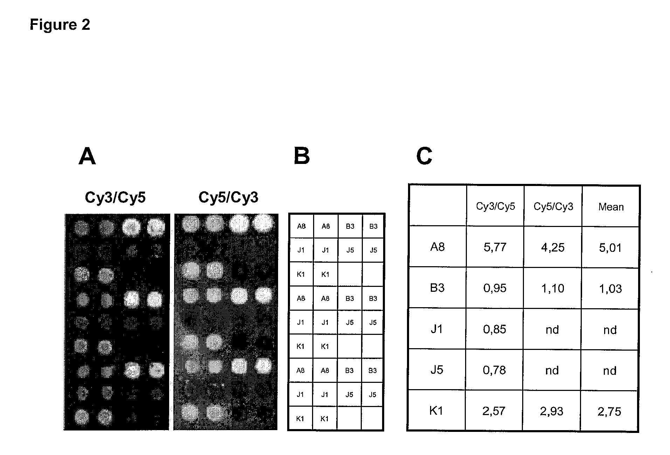

The combined data established for SEQ ID NO:1 together with the data for SEQ ID NO:2-9 and selected additional sequences are presented in summary in figure 1 which comprises a list of the cancer antigens identified, characterized and presented in this invention. Here, the identities of the cancer antigens of SEQ ID NO:1-9 are especially indicated.
Names and/or accession numbers (Acc. No.) of differentially expressed sequences are given. According to data derived from Microarray Analysis (gene expression analysis), in total 89 sequences were found to be differentially expressed in at least one pair of metastasizing versus non metastasizing cells (indicated as a "+" mark in the column Microarray). These Microarray Analysis experiments were performed as described in figure 2. Some of the sequences listed in the table have been shown to be differentially expressed (indicated as a "+" mark in the column ISH) also by performing "In situ Hybridization" (ISH) experiments with matched human normal and tumor tissue samples derived from at least three tissue types.
Several sequences were also analyzed in in Cancer profiling Arrays (CA): Here, overexpression of a given gene (indicated as a "+" mark in the column CA) was defined as upregulation of expression in the tumor probe versus the normal probe in at least 50% of analyzed pairs which were derived from at least 3 of 8 different tissues analyzed.
In addition, figure 1 also contains information on indications for functional involvement of the single sequences in metastatic processes. A positive "+" mark in this context indicates that a given cancer antigen gave rise to an at least 20% change of activity over control in at least one functional assay. For detailed information on functional assays see figure 6-9.
Nine sequences were estimated as positive ("+" mark in the column functional indications) for at least three out of four criteria measured for having a relevance in metastatic processes (i.e. measurements of the following tests: Analyses in Microarray, ISH, CA, functional tests). These sequences are highlighted and refer to SEQ ID NO:1-9. Detailed descriptions of these SEQ ID NO:1-9 are given in Examples 1-9. The column "ID" lists the internal identification number, "Sequence No" gives the number of the sequence used in the text.

In figure 2, raw Microarray analysis data from hybridization tests with cDNA from the endometrial cancer cell line HEC-1A versus the metastasizing endometrial cancer cell line AN3-CA (ATCC HTB-112 and -111) are presented, including in exemplified manner the analysis of the expression of SEQ ID NO:1, which is annotated as sequence A8 in figure 2.
Diagnostic tools in the form of cDNA chips were made by spotting 4 ng of each cDNA for the 89 genes listed in figure 1 onto glass slides. Each gene was spotted 6 times in duplets. In addition, 4 housekeeping genes were spotted (HPRT, β-Actin, α-Tubulin, Ubiquitin). For hybridisation purposes, 1.5 µg poly A⁺ RNA isolated from the cell lines listed in example 10 was reverse transcribed and labelled using the Cyscribe Kit (Amersham). In one half of the experiment RNA from the non metastasizing cells was labelled with Cy3, and RNA from the metastasizing cells with Cy5 (left side Figure 2A). In the other half of the experiment RNA from the non metastasizing cells was labelled with Cy5, and RNA from the metastasizing cells with Cy3 (right side Figure 2A). Probes were mixed and hybridized to the cDNA chips. Representative sections of the cDNA chips are shown in A. Gene sequences (cancer antigens) upregulated in the metastasizing cells light up red on the left side, and light up green on the right side. Yellow spots indicate unchanged expression. B: The spotting scheme for the sections of the cDNA chips shown in A is presented. C: Regulation factors for the expression of the five genes shown in A are given. Averages from 12 spots of the 635/532 nm signal in the column Cy3/Cy5, and of the 532/635 nm signal in the column Cy5/Cy3 are shown. "Mean" is the average of the Cy3/Cy5 and the Cy5/Cy3 value. Note: A regulation factor of, e.g., 5.01 as estimated as Mean value for the sequence annotated as A8, which represents SEQ ID NO:1, refers to a 5.01 fold overexpression of this sequence in the metastasising cells in comparison to the non metastasising tumor cells.

### Example 2

### SEQ ID NO:2 (E4)

Another rat cDNA clone, originally derived from the above described SSH analysis of the pancreatic tumor test system was used to establish the corresponding EST cluster from rat EST databases. Nucleotide sequence identity with an identified rat sequence cluster was over 96%. Three further clones derived from this pancreatic test system also matched to this gene sequence cluster with over 96% nucleotide sequence identity. The consensus sequence of this cluster was established by using the software DNAStar, SeqManll (http://www.dnastar.com/), and was subsequently used in blast analysis using the human genome sequence database BLAT (http://genome.ucsc.edu/cgi-bin/hgBlat?command=start). This way, a nucleotide sequence identity of 90% was identified with the human mRNA AK130372 representing the locus FAM49B (family with sequence similarity 49, member B), alias BM-009. The corresponding NCBI reference sequence for this locus, NM_016623 comprises a length of 2219 nucleotides and codes for a predicted protein of unknown function. According to the AceView application, different transcripts of this gene exist, altogether putatively encoding 19 different protein isoforms.
AceView represents an integrated view of the human genes as reconstructed by alignment of all publicly available mRNAs and ESTs on the genome sequence (http://www.ncbi.nih.gov/IEB/Research/Acembly/index.html?human).

The amino acid sequence of FAM49B was analyzed by PSORT, a computer program for the prediction of protein localization sites in cells. According to PSORT2 (http://psort.nibb.ac.jp) the proteins encoded by this RNA are most likely located in the cytoplasm. The amino acid sequence of FAM49B was also analyzed by Pfam search. According to this analysis this protein belongs to a family of several hypothetical eukaryotic proteins (DUF1394) of around 320 residues in length. The functions of this protein family are unknown. The gene is localized in the 8q24 region, an area found to be minimally overepresented in prostate cancer (Tsuchiya, 2000, Am. J. Pathol. 160, 1799-1806).
In figure 1, a summary of established data for SEQ ID NO:2 is presented.
This sequence was shown to be differentially expressed in Microarray Analysis comparing samples of metastasizing versus non metastasizing cells as exemplified for SEQ ID NO:1 in figure 2. Tumor specific expression was further analyzed by hybridization experiments with Cancer Profiling Arrays (CA) from Clontech (http://www.bdbiosciences.com). The estimated percentages of upregulation in the tissues analyzed is shown in figure 5. FAM49B shows significant upregulation (in more than 50% of analyzed pairs) in uterus, ovary, colon and rectum.

In order to functionally examine whether FAM49B could be causally involved in the process of tumor progression, it was transiently overexpressed or transiently downregulated by RNA interference in HEK-293T cells and subsequently potential resulting influences on tumor cell properties were assayed. For overexpression a sequence corresponding to the NCBI reference sequence (http://www.ncbi.nlm.nih.gov/RefSeq/) was used. Experiments as previously exemplified for SEQ ID NO:1 in figures 6-8 demonstrate, that overexpression of FAM49B leads to increased proliferation, whereas its downregulation results in decreased proliferation.
Furthermore, FAM49B also affects the invasion potential of tumor cells.

In summary, FAM49B shows upregulation in metastasizing tumor cells versus non metastasizing tumor cells, and also displays upregulated expression in various tumor tissues versus normal tissue samples. Moreover, FAM49B is functionally involved in processes involved in tumor progression like increased proliferation and invasion. Therefore, this sequence may particularly be useful for staging of human tumor diseases, as well as for decisions on prognosis and treatment modalities. Furthermore, the FAM49B gene and its gene products may be used as target structures to develop therapeutic anti-cancer drugs.

SEQ I D NO: 2 (NM_016623)
ggcaggt gt t gaggggct cccggt ccggct gccgccgct cccccgct ccggacccggggct cccccct agcgccgct gagg

agccgcct ct gcggct ccaggagggcgcaggagcgggact gagagcgcct ggaggct cgagcggagggt aat t cat t t gc
acacct gt t agcaagaaacagaagt t gaaggact ggaacaagt gaact aggaaagagggaacgccaat ccaaggat agaa
ggacaaggacagaat caccagcact ggct gaaggcct cct gt t t cct gcgct t t ct cct t t t cct gt gaaat ct ccgagg
agaagaaagaat gat ggacagt t t at cct t t cact gccacaaggcct gt t t act t ggcagt aggt cct t aagt t cct t gc
t t t t t t gct gct gt t t ggt gact ggaagaggcaccagagact ct cact ct ggggaggt t t gct ggcat gggt aat ct cat
t aaggt gct aaccagggacat agaccacaat gcagcacat t t t t t ct t ggact t t gaaagt acct t aacat ggggaat ct
t ct t aaagt t t t gacat gcacagacct t gagcaggggccaaat t t t t t cct t gat t t t gaaaat gcccagcct acagagt
ct gagaaggaaat t t at aat caggt gaat gt agt at t aaaagat gcagaaggcat ct t ggaggact t gcagt cat acaga
ggagct ggccacgaaat acgagaggcaat ccagcat ccagcagat gagaagt t gcaagagaaggcat ggggt gcagt t gt
t ccact agt aggcaaat t aaagaaat t t t acgaat t t t ct cagaggt t agaagcagcat t aagaggt ct t ct gggagcct
t aacaagt accccat at t ct cccacccagcat ct agagcgagagcaggct ct t gct aaacagt t t gcagaaat t ct t cat
t t cacact ccggt t t gat gaact caagat gacaaat cct gccat acagaat gat t t cagct at t at agaagaacat t gag
t cgt at gaggat t aacaat gt accggcagaaggagaaaat gaagt aaat aat gaat t ggcaaat cgaat gt ct t t gt t t t
at gct gaggcaact ccaat gct gaaaacct t gagt gat gccacaacaaaat t t gt at cagagaat aaaaat t t accaat a
gaaaat accacagat t gt t t aagcacaat ggct agt gt at gcagagt cat gct ggaaacaccggaat acagaagcagat t
t acaaat gaagagacagt gt cat t ct gct t gagggt aat ggt gggt gt cat aat act ct at gaccacgt acat ccagt gg
gagcat t t gct aaaact t ccaaaat t gat at gaaaggt t gt at caaagt t ct t aaggaccaacct cct aat agt gt ggaa
ggt ct t ct aaat gct ct caggt acacaacaaaacat t t gaat gat gagact acct ccaagcaaat t aaat ccat gct gca
at aacaat t ct ggaat aagcacct gct gt agacagaagacagt at t ct gcaat gact gagaat gcagt t t t t t agt gat t
gcaat t act at ct cat t t at t ct t gct t t t at t t ct t t cct ct gt t cct ct t ccct ct t t t t t aat cat gt t ct t aagac
t t ct t t t ct gt gccaaaat cagt aaagt t acact ct gaagggat at cat cct t t caaacgggccat ct aaggcagct aat
t at gcat t gcat t ggggt ct ct act gagaaaaat t ct gt gact t gaact aaat at t t t t aaat gt ggat t t t t t t t gaaa
ct aat at t t aat at t gct t ct cct gcat ggcaaaact gcct at t ct gct at t aaaaaccct caat gact t t at t t t ct a
ct gccgcct t t t t cat gt gcaaccaaaat gaaaat gt t t aaat t aact gt gt t gt acaaat ggt acccaacacaaact t t
t t t t aaat t agt aat act t t t gt t t aaagt t t t aagt t gcat t t t gact t t t t t t gt aaggat gt at gt t gt gt gt t t a
acct t t at t aact aacgt t aaaagct gt gat gt gt gcgt agaat at t acgt at gcat gt t cat gt ct aaagaat ggct gt
t gat gat aaaat aaaaat cagct t t cat t t t t ct aaaaaaaaaaaaaaaaaaaaaaaa

SEQ I D NO: 11 - PROTEI N ( NP_057707)
MGNLLKVLTCTDLEQGPNFFLDFENAQPTESEKEI YNQVNVVLKDAEGI LEDLQSYRGAGHEI REAI QHPADEKLQEKAWGAV
VPLVGKLKKFYEFSQRLEAALRGLLGALTSTPYSPTQHLEREQALAKQFAEI LHFTLRFDELKMTNPAI QNDFSYYRRTLSRM
RI NNVPAEGENEVNNELANRMSLFYAEATPMLKTLSDATTKFVSENKNLPI ENTTDCLSTMASVCRVMLETPEYRSRFTNEET
VSFCLRVMVGVI I LYDHVHPVGAFAKTSKI DMKGCI KVLKDQPPNSVEGLLNALRYTTKHLNDETTSKQI KSMLQ

### Example 3

### SEQ ID NO:3 (H3)

Another rat cDNA clone, originally derived from the above described SSH analysis of the pancreas tumor test system was used to establish the corresponding EST cluster from rat EST databases. Identity to the ESTs within this cluster was 98%. Identity within the cluster was over 96%. The consensus sequence of this cluster was used to blast against human genome sequence databases. An identity of 89% was found to the human mRNA NM_024085 representing the locus FLJ22169. The reference RNA has a length of 3816 nucleotides and codes for a predicted protein of unknown function with 839 amino acids. According to Pfam Search the predicted protein shares homology to Autophagy protein Apg9. In yeast, 15 Apg proteins coordinate the formation of autophagosomes. Autophagy is a bulk degradation process induced by starvation in eukaryotic cells. Apg9 plays a direct role in the formation of the cytoplasm to vacuole targeting and autophagic vesicles, possibly serving as a marker for a specialised compartment essential for these vesicle-mediated alternative targeting pathways. According to Psort2, this protein most likely localizes to the membrane. According to AceView, this gene produces, by alternative splicing, 9 different transcripts altogether encoding 9 different protein isoforms.
In figure 1, a summary of established data for SEQ ID NO:3 is presented.
This sequence was shown to be differentially expressed in Microarray Analysis comparing samples of metastasizing versus non metastasizing tumor cells as exemplified for SEQ ID NO:1 in figure 2. Tumor specific expression was further analyzed in hybridization experiments using Cancer Profiling Arrays (CA) from Clontech (http://www.bdbiosciences.com). Estimations of percentages of upregulation in the tissues analyzed is shown in figure 5. FLJ22169 shows significant upregulation of expression (in more than 50% of analyzed pairs) in tissues derived from cancers of the uterus, ovary, colon and rectum.

In order to functionally examine whether FLJ22169 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6 and 7, demonstrate that also overexpression of FLJ22169 leads to increased proliferation, its downregulation results in decreased proliferation. FLJ22169 also affects invasion potential of tumor cells in experiments performed according to those exemplified for SEQ ID NO:1 in figure 9.

In summary, FLJ22169 shows upregulation in metastasizing tumor cells versus non metastasizing tumor cells, and also displays upregulated expression in various tumor tissues versus normal tissue samples. Moreover, FLJ22169 is functionally involved in processes involved in tumor progression like increased proliferation and invasion. Therefore, this sequence may particularly be useful for staging of human tumor diseases, as well as for decisions on prognosis and treatment modalities. Furthermore, the FLJ22169 gene and its gene products may be used as target structures to develop therapeutic anti-cancer drugs.

SEQ I D NO: 3 ( NM_024085)
ggggt cgcgccgagccgagccgagccgagcggagccggcggagcct ct ggaat cacccgggt cgct gt t cct gaggt ggt

caaggt ggacagggggcggt ggt gat ggcgcagt t t gacact gaat accagcgcct agaggcct cct at agt gat t cacc
cccaggggaggaggacct gt t ggt gcacgt cgccgaggggagcaagt cacct t ggcaccgt at t gaaaacct t gacct ct
t ct t ct ct cgagt t t at aat ct gcaccagaagaat ggct t cacat gt at gct cat cggggagat ct t t gagct cat gcag
t t cct ct t t gt ggt t gcct t cact acct t cct ggt cagct gcgt ggact at gacat cct at t t gccaacaagat ggt gaa
ccacagt ct t caccct act gaacccgt caaggt cact ct gccagacgcct t t t t gcct gct caagt ct gt agt gccagga
t t caggaaaat ggct ccct t at caccat cct ggt cat t gct ggt gt ct t ct ggat ccaccggct t at caagt t cat ct at
aacat t t gct gct act gggagat ccact cct t ct acct gcacgct ct gcgcat ccct at gt ct gccct t ccgt at t gcac
gt ggcaagaagt gcaggcccggat cgt gcagacgcagaaggagcaccagat ct gcat ccacaaacgt gagct gacagaac
t ggacat ct accaccgcat cct ccgt t t ccagaact acat ggt ggcact ggt t aacaaat ccct cct gcct ct gcgct t c
cgcct gcct ggcct cggggaagct gt ct t ct t cacccgt ggt ct caagt acaact t t gagct gat cct ct t ct ggggacc
t ggct ct ct gt t t ct caat gaat ggagcct caaggccgagt acaaacgt ggggggcaacggct agagct ggcccagcgcc
t cagcaaccgcat cct gt ggat t ggcat cgct aact t cct gccgt gccccct cat cct cat at ggcaaat cct ct at gcc
t t ct t cagct at gct gaggt gct gaagcgggagccgggggccct gggagcacgct gct ggt cact ct at ggccgct gct a
cct ccgccact t caacgagct ggagcacgagct gcagt cccgcct caaccgt ggct acaagcccgcct ccaagt acat ga
at t gct t ct t gt cacct ct t t t gacact gct ggccaagaat ggagcct t ct t cgct ggct ccat cct ggct gt gct t at t
gccct caccat t t at gacgaagat gt gt t ggct gt ggaacat gt gct gaccaccgt cacact cct gggggt caccgt gac
cgt gt gcaggt cct t t at cccggaccagcacat ggt gt t ct gccct gagcagct gct ccgcgt gat cct cgct cacat cc
act acat gcct gaccact ggcagggt aat gcccaccgct cgcagacccgggacgagt t t gcccagct ct t ccagt acaag
gcagt gt t cat t t t ggaagagt t gt t gagccccat t gt cacacccct cat cct cat ct t ct gcct gcgcccacgggccct
ggagat t at agact t ct t ccgaaact t caccgt ggaggt cgt t ggt gt gggagat acct gct cct t t gct cagat ggat g
t t cgccagcat ggt cat ccccagt ggct at ct gct gggcagacagaggcct cagt gt accagcaagct gaggat ggaaag
acagagt t gt cact cat gcact t t gccat caccaaccct ggct ggcagccaccacgt gagagcacagcct t cct aggct t
cct caaggagcaggt t cagcgggat ggagcagct gct agcct cgcccaagggggt ct gct ccct gaaaat gccct ct t t a
cgt ct at ccagt cct t acaat ct gagt ct gagcccct gagcct t at cgcaaat gt ggt agct ggct cat cct gccggggc
cct ccact gcccagagacct gcagggct ccaggcacagggct gaagt cgcct ct gccct gcgct cct t ct ccccgct gca
acccgggcaggcgcccacaggccgggct cacagcaccat gacaggct ct ggggt ggat gccaggacagccagct ccggga
gcagcgt gt gggaaggacagct gcagagcct ggt gct gt cagaat at gcat ccacagagat gagcct gcat gccct ct at
at gcaccagct ccacaagcagcaggcccaggct gaacct gagcggcat gt at ggcaccgccgggagagt gat gagagt gg
agaaagcgcccct gat gaaggggagagggcgcccggcccccagt ct at ccct cgct ct gct agct at ccct gt gt ag
caccccggcct ggagct cct gagaccaccgccct gcat ggggct t ccagaggcgct acggt ggcat cacagat cct ggc
acagt gcccagggt t ccct ct cat t t ct ct cggct gcct ct t ggagggt gggcagaagat gggcagt cggcat caaggca
ccct gagcccgt gcccgaagagggct cggaggat gagct accccct caggt gcacaaggt at agacaaggct gagcaggg
t t cct gt ggcccaggat ggaggccaccgct gccct gccat cccgt ct gcct gccat gggacggct cct ct gagt gt t ccc
t ggccccat gt gt gt ggt gt t t gt gt gt ct gt gcct ggccaagggaggt gccaacact gggct t gccacagccccaggag
aggaat t t ggggcct aggaaccgagggcacacgggact ct agcct cat ccccaggacccccct t ggct cagagt gt ggt gc
t agaaact ggt ccccagcccagccccagt act gccacct t t acacct acccct gcaagt ccccagagggct gcccacgat
agaagct gccaagcagggagaacct gt gccaact gt ggagt ggggaggt t gggcct ggaccct caacccct gcaacct t c
cct agccccct caat agat gagcaggt caggct gt ggccct t acct cacccgcagt t ct cgcccagt gct gcagccggct
cacct ct ct ccgct t ct t gcacat cact ggcct gt gt gt gct gct t gct cct gt t ct gt t cgct t gct cccgt t ccgt t c
ggct t t t gct t t gcgt t agggt gaagaccct agcgt ccagct cccct caacgct at at t t t gacact aaaaaagaaggt t
t ct aaat t gt aggagcaggat ggaaat act t t gct gccct t gccat ct t t t aggat gggcccccaggagact gaggt ct t
cct gggccct cat t gct gct t at cgt acccccccat cacct gcacat gggacagaccgggct ggagggt gacct t ggct gt
gt acgt cccagcaaaagagct ct ggcccgcat ct cgct gt gccct gaagggggat gaagggcgat gcct cgcccgaggct
t t gggct gct gcact gcat gct gggact gct cct act ct ct gt cccacccct cacccagct gt ggt ccggct t t gggaga
gt ggt gaat t gcgct gcccgaact cggagcggagcagggt agggaccgt gt acagct t gat aaccct t aat aaaaaggga
gt t t gaccagaaaaaaaaaaaaaaaaaaaaaaaaaaaaaaaaaaaaaaaaaaaaaaaaaaaaaaaaaaaaaaaaaaaagaaa
aaaaaaaaaaaaaaaagaaaaaaaaaaaaaaaagaaaaaaaaaaaaaaaaaacct

SEQ I D NO: 12 - PROTEI N ( NP_076990)
MAQFDTEYQRLEASYSDSPPGEEDLLVHVAEGSKSPWHRI ENLDLFFSRVYNLHQKNGFTCMLI GEI FELMQFLFVVAFTTFL
VSCVDYDI LFANKMVNHSLHPTEPVKVTLPDAFLPAQVCSARI QENGSLI TI LVI AGVFWI HRLI KFI YNI CCYWEI HSFYLH

ALRIPMSALPYCTWQEVQARIVQTQKEHQICIHKRELTELDIYHRILRFQNYMVALVNKSLLPLRFRLPGLGEAVFFTRGLKY
NFELILFWGPGSLFLNEWSLKAEYKRGGQRLELAQRLSNRILWIGIANFLPCPLILIWQILYAFFSYAEVLKREPGALGARCW
SLYGRCYLRHFNELEHELQSRLNRGYKPASKYMNCFLSPLLTLLAKNGAFFAGSILAVLIALTIYDEDVLAVEHVLTTVTLLG
VTVTVCRSFIPDQHMVFCPEQLLRVILAHIHYMPDHWQGNAHRSQTRDEFAQLFQYKAVFILEELLSPIVTPLILIFCLRPRA
LEIIDFFRNFTVEVVGVGDTCSFAQMDVRQHGHPQWLSAGQTEASVYQQAEDGKTELSLMHFAITNPGWQPPRESTAFLGFLK
EQVQRDGAAASLAQGGLLPENALFTSIQSLQSESEPLSLIANVVAGSSCRGPPLPRDLQGSRHRAEVASALRSFSPLQPGQAP
TGRAHSTMTGSGVDARTASSGSSVWEGQLQSLVLSEYASTEMSLHALYMHQLHKQQAQAEPERHVWHRRESDESGESAPDEGG
EGARAPQSIPRSASYPCVAPRPGAPETTALHGGFQRRYGGITDPGTVPRVPSHFSRLPLGGWAEDGQSASRHPEPVPEEGSED
ELPPQVHKV

### Example 4

### SEQ ID NO:4 (B3)

Another rat cDNA clone, derived from the above described SSH analysis of the mammary tumor test system showed 99% identity to the rat mRNA CB717750. The corresponding rat EST cluster was used for a blast analysis against human genome databases. An identity of 90% was found on the nucleotide level to the human mRNA AK000178 representing the locus FLJ20171 which maps on chromosome 8. According to AceView, this locus produces, by alternative splicing, 13 different transcripts altogether encoding 13 different protein isoforms.
The corresponding NCBI Reference sequence NM_017697 comprises 2140 nucleotides and encodes a hypothetical protein of 358 amino acids. According to SMART analysis (Simple Modular Architecture Research Tool, http://smart.embl-heidelberg.de/) this protein contains a RNA recognition motif known as the eukaryotic putative RNA-binding region RNP-1 signature or RNA recognition motif (RRM). RRMs are found in a variety of RNA binding proteins, including heterogeneous nuclear ribonucleoproteins (hnRNPs), proteins implicated in regulation of alternative splicing, and protein components of small nuclear ribonucleoproteins (snRNPs). The motif also appears in a few single stranded DNA binding proteins. The RRM structure consists of four strands and two helices arranged in an alpha/beta sandwich, with a third helix present during RNA binding in some cases.
In figure 1, a summary of established data for SEQ ID NO:4 is presented.
This sequence was shown to be differentially expressed in Microarray Analysis comparing samples of metastasizing versus non metastasizing tumor cells as previously exemplified for SEQ ID NO:1 in figure 2. Tumor specific expression was further analyzed in hybridization experiments using Cancer Profiling Arrays (CA) from Clontech (http://www.bdbiosciences.com). Estimations of percentages of upregulation in the tissues analyzed is shown in figure 5. FLJ20171 shows significant upregulation (in more than 50% of analyzed pairs) in tissues derived from cancers of the uterus, ovary and lung.

In order to functionally examine whether FLJ20171 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6-8, demonstrate that also overexpression of FLJ20171 leads to increased proliferation, its downregulation results in decreased proliferation. FLJ20171 also affects invasion potential of tumor cells, as observed in experiments performed according to those exemplified for SEQ ID NO:1 in figure 9.

In summary, FLJ20171 shows upregulation in metastasizing tumor cells versus non metastasizing tumor cells, and also displays upregulated expression in various tumor tissues versus normal tissue samples. Moreover, FLJ20171 is functionally involved in processes involved in tumor progression like increased proliferation and invasion. Therefore, this sequence may particularly be useful for staging of human tumor diseases, as well as for decisions on prognosis and treatment modalities. Furthermore, the FLJ20171 gene and its gene products may be used as target structures to develop therapeutic anti-cancer drugs.

SEQ I D NO: 4 ( NM_017697)
gaat t caagaaat gt t gccct ggt t cacct gat at t gacaaact ggacgt t gccacaat gacagagt at t t aaat t t t ga
gaagagt agt t cagt ct ct cgat at ggagcct ct caagt t gaagat at ggggaat at aat t t t agcaat gat t cagagc
ct t at aat cacaggt t t t cagat ccagagagagt gaat t acaagt t t gaaagt ggaact t gcagcaagat ggaact t at t
gat gat aacaccgt agt cagggcacgaggt t t accat ggcagt ct t cagat caagat at t gcaagat t ct t caaaggact
caat at t gccaagggaggt gcagcact t t gt ct gaat gct cagggt cgaaggaacggagaagct ct ggt t aggt t t gt aa
gt gaggagcaccgagacct agcact acagaggcacaaacat cacat ggggacccggt at at t gaggt t t acaaagcaaca
ggt gaagat t t cct t aaaat t gct ggt ggt act t ccaat gaggt agcccagt t t ct ct ccaaggaaaat caagt cat t gt
t cgcat gcgggggct ccct t t cacggccacagct gaagaagt ggt ggcct t ct t t ggacagcat t gccct at t act gggg
gaaaggaaggcat cct ct t t gt cacct acccagat ggt aggccaacaggggacgct t t t gt cct ct t t gcct gt gaggaa
t at gcacagaat gcgt t gaggaagcat aaagact t gt t gggt aaaagat acat t gaact ct t caggagcacagcagct ga
agt t cagcaggt gct gaat cgat t ct cct cggcccct ct cat t ccact t ccaacccct cccat t at t ccagt act acct c
agcaat t t gt gcccccct acaaat gt t agagact gt at acgcct t cgaggt ct t ccct at gcagccacaat t gaggacat c
ct ggat t t cct gggggagt t cgccacagat at t cgt act cat ggggt t cacat ggt t t t gaat caccagggccgcccat c
aggagat gcct t t at ccagat gaagt ct gcggacagagcat t t at ggct gcacagaagt gt cat aaaaaaaaacat gaag
gacagat at gt t gaagt ct t t cagt gt t cagct gaggagat gaact t t gt gt t aat gggggcact t t aaat cgaaat gg
ct t at ccccaccgccat gt aagt t accat gcct gt ct cct ccct cct acacat t ccagct cct gct gcagt t at t cct a
cagaagct gccat t t accagccct ct gt gat t t t gaat ccacgagcact gcagccct ccacagcgt act acccagcaggc
act cagct ct t cat gaact acacagcgt act at cccagcccccccaggt t cgcct aat agt ct t ggct act t ccct acagc
t gct aat ct t agcggt gt ccct ccacagcct ggcacggt ggt cagaat gcagggcct ggcct acaat act ggagt t aagg
aaat t ct t aact t ct t ccaaggt t accagt gt t t gaaagat gt at ggt gat ct t gaaacct ccagacacaagaaaact t c
t agcaaat t caggggaagt t t gt ct acact caggct gcagt at t t t cagcaaact t gat t ggacaaacggcct gt gcct
t at ct t t t ggt ggagt gaaaaagt t t gagct agt gaagccaaat cgt aact t acagcaagcagcat gcagcat acct ggc
t ct t t gct gat t gcaaat aggcat t t aaaat gt gaat t t ggaat cagat gt ct ccat t act t ccagt t aaagt ggcat ca

t aggt gt t t cct aagt t t t aagt ct t ggat aaaaact ccaccagt gt ct accat ct ccaccat gaact ct gt t aaggaag
ct t cat t t t t gt at at t cccgct ct t t t ct ct t cat t t ccct gt ct t ct gcat aat cat gcct t ct t gct aagt aat t ca
agcat aagat ct t ggaat aat aaaat cacaat ct t aggagaaagaat aaaat t gt t at t t t cccagt ct ct t ggccat ga
t gat at ct t at gat t aaaaacaaat t aaat t t t aaaacacct gaaaaaaaaaaaaaaaa

SEQ ID NO: 13 - PROTEIN (NP_060167)
MTEYLNFEKSSSVSRYGASQVEDMGNIILAMISEPYNHRFSDPERVNYKFESGTCSKMELIDDNTVVRARGLPWQSSDQDIAR
FFKGLNIAKGGAALCLNAQGRRNGEALVRFVSEEHRDLALQRHKHHMGTRYIEVYKATGEDFLKIAGGTSNEVAQFLSKENQV
IVRMRGLPFTATAEEVVAFFGQHCPITGGKEGILFVTYPDGRPTGDAFVLFACEEYAQNALRKHKDLLGKRYIELFRSTAAEV
QQVLNRFSSAPLIPLPTPPIIPVLPQQFVPPTNVRDCIRLRGLPYAATIEDILDFLGEFATDIRTHGVHMVLNHQGRPSGDAF
IQMKSADRAFMAAQKCHKKKHEGQIC

### Example 5

### SEQ ID NO:5 (D2)

Another rat cDNA clone was used to establish the corresponding EST cluster from rat EST databases. Identity within the cluster was over 96%. The consensus sequence of this cluster was used for a blast analysis against human genome databases. An identity of 80% was found to the human mRNA NM_030815 representing the locus C20orf126 which maps on chromosome 20. The Ensembl Genome Browser (http://www.ensembl.org/Homo sapiens/) predicts that it produces one transcript with a length of 1290 bp. The coding sequence of the protein between the first in frame amino acid and the stop codon contains 176 residues. The first methionine corresponds to amino acid 44. The calculated molecular weight of the protein product is 15.5 kD.
Bioinformatic analysis according to PSORTII predicts that the subcellular localization of this protein is expected to be in the nucleus. Besides a nuclear localization signal, the predicted protein contains coiled coil domains. Such coiled coil structures (Psort Motiv, http://psort.nibb.ac.jp/ ) are found in some structural proteins, e.g. myosins, and in some DNA binding proteins as the so called leucine zipper. In this structure two α-helices bind each other forming a coil, in which this helices show a 3.5 residue periodicity which is slightly different from the typical value estimated at 3.6. Thus, the detection of coiled coil structure by searching for 7-residue periodicity is relatively more accurate than usual secondary structure prediction. Currently a classical detection algorithm developed by A. Lupas is used (Lupas, 1991, Science 252, 1162-1164). The function of C20orf126 is still unknown. Pfam analysis shows that this protein does not belong to any recognized protein family.
In figure 1, a summary of established data for SEQ ID NO:5 is presented.
This sequence was shown to be differentially expressed in Microarray Analysis comparing samples of metastasizing versus non metastasizing cells as previously exemplified for SEQ ID NO:1 in figure 2. Tumor specific expression was further analyzed in hybridization experiments using Cancer Profiling Arrays (CA) from Clontech (http://www.bdbiosciences.com). Estimations of percentages of upregulation in the tissues analyzed is shown in figure 5. C20orf126 shows significant upregulation (in more than 50% of analyzed pairs) in tissues derived from cancers of the breast, uterus, ovary, colon and rectum.

In order to functionally examine whether C20orf126 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6-8, demonstrate that also overexpression of C20orf126 leads to increased proliferation, its downregulation results in decreased proliferation. C20orf126 also affects invasion potential of tumor cells, as observed in experiments performed according to those exemplified for SEQ ID NO:1 in figure 9.

In summary, C20orf126 shows upregulation in metastasizing tumor cells versus non metastasizing tumor cells, and also displays upregulated expression in various tumor tissues versus normal tissue samples. Moreover, C20orf126 is functionally involved in processes involved in tumor progression like increased proliferation and invasion. Therefore, this sequence may particularly be useful for staging of human tumor diseases, as well as for decisions on prognosis and treatment modalities. Furthermore, the C20orf126 gene and its gene products may be used as target structures to develop therapeutic anti-cancer drugs.

SEQ I D NO: 5 ( NM_030815)
accgt t ct t t t aact gcgcaggcgcgccggaagcacct agagagcggcgcgt gcgcagcgggagt cgaagcggagat ccc
ggggt cgcgcgagagccgcaagcggagt t ggt gggcgct at gct at cacccgaggcagagcgagt gct gcggt acct t gt
agaagt ggaggagct cgccgaggaggt gct ggcggacaagcggcagat t gt ggacct ggacact aaaaggaat cagaat c
gagagggcct gagggccct gcagaaggat ct cagcct ct ct gaagat gt gat ggt t t gct t cgggaacat gt t t at caag
at gcct caccct gagacaaaggaaat gat t gaaaaagat caagat cat ct ggat aaagaaat agaaaaact gcggaagca
act t aaagt gaaggt caaccgcct t t t t gaggcccaaggcaaaccggagct gaagggt t t t aact t gaaccccct caacc
aggat gagct t aaagct ct caaggt cat ct t gaaaggat gagact caagaaccaagat gggggaccagcaacccccagg
gt cat ggaggacccaggaccct ccaacct t gacacct gt aaggacaggat ct gccct gt aaggggccagccgt caggaat
ct ggccat gaaaacct ct t gt agt gct t ggct act ct gt gat ggcaggagggaacct t cagcct gt ct ggct gct ggac
ct ggacaccagggct cggt ggacacaagat ct at t gacgggcct t ggt agccaccagt gggt gt gt ggggcagt ggct gt
gggggt gt aagaat gact gcaacaggcact t cccaacaat ggcct gct gt t cacat ggaccct gagcaaggaaggaggga
gggaggggcagagt ggagt gt cat t ccagcat t cct ct cagaagggagagaggt t t caggct ggt gccat gcgat t gga
at aaagcaggaggct cat gggt ggt t gct gaat gaagaacagaat ct t ggt gct t gt ggct caccacagccat ct gt gg
ggcaggcacacacacct cccgccagct ccaat t t t gcact t t t t ccct gct t gat t ccaagagt aggt gct gcct agcag
ccct t cgt ggccact ct t t act caggagggcct t gcagagt cct gcaccaggcct gggt gagt ggat gcgcct ct t acca

t at gacacgt gt caagat gccct t ccgcccccct ct gaaagt ggggcccggccagcact gct cgt t act gt ct gcct t cag
t ggt ct gaggt cccagt at gaact gccgt gaagt caaaact ct t at gt gt t cat t aagggct caat aaat gt t agct gaa
t gaat gaat agcaaaaaaaaaaaa

SEQ ID NO:14 - PROTEIN (NP_110442, C20orf126)
MLSPEAERVLRYLVEVEELAEEVLADKRQIVDLDTKRNQNREGLRALQKDLSLSEDVMVCFGNMFIKMPHPETKEMIEKDQDH
LDKEIEKLRKQLKVKVNRLFEAQGKPELKGFNLNPLNQDELKALKVILKG

### Example 6

### SEO ID NO:6 (H5)

Another rat cDNA clone, originally derived from the above described SSH analysis of the mammary tumor test system was used for a blast analysis against rat EST databases. Similarity was found to the EST BE101513 which the was used to establish the corresponding EST cluster from rat EST databases. Identity within the cluster was over 96%.
The consensus sequence of this cluster was used for blast analysis against the human genome browser BLAT (http://genome.ucsc.edu/cgi-bin/hgBlat?command=start). An identity of 90% was found to the human mRNA AK025697 representing the locus FBX045 which maps on chromosome 3. According to AceView, this gene produces, by alternative splicing, 3 different transcripts altogether encoding 3 different protein isoforms. The corresponding NCBI Reference sequence XM_117294 comprises 4159 nucleotides and encodes a hypothetical protein of 286 amino acids. Comparison to the InterPro Database, a database of protein families, domains and functional sites (http://www.ebi.ac.uk/interpro/index.html), a Cyclin like F box motif is identified in the product of this gene. The F-box domain was first described as a sequence motif found in cyclin-F that interacts with the protein SKP1. This relatively conserved structural motif is present in numerous proteins and serves as a link between a target protein and a ubiquitin-conjugating enzyme. According to InterPro, also the SPIa/RYanodine receptor SPRY motif is found in 2 isoforms from this gene. The SPRY domain is of unknown function.
In figure 1, a summary of established data for SEQ ID NO:6 is presented.
This sequence was shown to be differentially expressed in Microarray Analysis comparing samples of metastasizing versus non metastasizing tumor cells as previously exemplified for SEQ ID NO:1 in figure 2. Tumor specific expression was further analyzed in hybridization experiments using Cancer Profiling Arrays (CA) from Clontech (http://www.bdbiosciences.com). Estimations of percentages of upregulation in the tissues analyzed is shown in figure 5. FBX045 shows significant upregulation (in more than 50% of analyzed pairs) in tissues derived from cancers of the uterus, ovary, colon and rectum.

In order to functionally examine whether FBX045 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6-8, demonstrate that also overexpression of FBX045 leads to increased proliferation, its downregulation results in decreased proliferation. FBX045 also affects invasion potential of tumor cells, as observed in experiments performed according to those exemplified for SEQ ID NO:1 in figure 9.

In summary, FBX045 shows upregulation in metastasizing tumor cells versus non metastasizing tumor cells, and also displays upregulated expression in various tumor tissues versus normal tissue samples. Moreover, FBX045 is functionally involved in processes involved in tumor progression like increased proliferation and invasion. Therefore, this sequence may particularly be useful for staging of human tumor diseases, as well as for decisions on prognosis and treatment modalities. Furthermore, the FBX045 gene and its gene products may be used as target structures to develop therapeutic anti-cancer drugs.

SEQ I D NO: 6 ( XM_117294)
gt gcgccct t gct t cgt gccct caacccgcat ggcggagccgct ggcgcgccgcggagaggccgggcgagt cgggcggt t
t cggcgcccgcgct gagccgcggaggaggggcggaggacgcccct gcagccggt gcgt ct gccct cagt gaggcggggcg
cgcggcggacgcccccgggcaggggcgggagt ggt ggaggcgccggcggt t ggcact gacaggggcggt gagcgagccgc
t ccggt ct ccgggcgaggct t ggcct t ccgagcagagacggcgggaagcggcggcggcagcggcggccct agggccggct
ggt gaggcgat ggcggcgccggccccgggggct ggggcagcct cgggcggcgct ggct gt agcggcggcggcgcgggcgc
gggcgcgggct cgggct ct ggggccgcgggggccgggggccggct gcccagccgggt gct ggagt t ggt gt t ct ct t acc
t ggagct gt ccgagct gcggagct gcgccct ggt gt gcaagcact ggt accgct gcct gcacggcgat gagaacagcgag
gt gt ggcggagcct gt gcgcccgcagcct ggcagaagaggct ct gcgcacggacat cct gt gcaacct gcccagct acaa
ggccaagat acgt gct t t t caacat gcct t cagcact aat gact gct ccaggaat gt ct acat t aagaagaat ggct t t a
ct t t acat cgaaaccccat t gct cagagcact gat ggt gcaaggaccaagat t ggt t t cagt gagggccgccat gcat gg
gaagt gt ggt gggagggccct ct gggcact gt ggcagt gat t ggaat t gccacaaaacgggccccccat gcagt gccaagg
t t at gt ggcat t gct gggcagt gat gaccagagct ggggct ggaat ct ggt ggacaat aat ct act acat aat ggagaag
t caat ggcagt t t t ccacagt gcaacaacgcaccaaaat at cagat aggagaaagaat t cgagt cat ct t ggacat ggaa
gat aagact t t agct t t t gaacgt ggat at gagt t cct ggggggt t gct t t t agaggact t ccaaaggt ct gct t at accc
agcagt t t ct gct gt at at ggcaacacagaagt gact t t ggt t t acct t ggaaaacct t t ggacggat gacagt ggct t t
ct t gt gat gacagacagaat ggaggagagat ct gct t at gggaagt agaaccat gaagt gact gt cacacat gcat gt cc
aagaaacat cct gaaaacacat gaagt cgt aaact ggagaagcagct ct acagcagagat t at ct t cgt gt t t cct ct t t
ct act gggccagaaaaat cct cagggt t gcagt t ggt t gagt gggcagt t gacat at gcat gt t gcacccgat gt t gt ct
ct aagt t agcaat gt gt t at t t ccagct t t aaaggt gagat t gt agagat gct gt caaagggat aaggaaat agcaagat
t t t t aagt agt gt gt t t gt gaagact gat cccat t t t acaact gcct gt t ct t t ct ccagt cct t t t t t t t ccagccagc
t t gact at t agaaaagt at gaaact ggt t gggt t t t at t t aat at t t t t aat at at t gagaagcat ggt ct gcct ggact
gcact t ct ct aaaagt gagat at aaaat t gt gcagct at t t t aaaagt t gt at at aat at gt gt gt aaaaaaaaaaaact
gt aaaaaagaaaggacaaacaggt t gt t t t gt t ct agt t ct aat t t ct t aaaaaccact acat ggt t acaaaat t ggaat

aacat t t ggggacaact gggt t aact acaaagaagaggat t t t aagaggagat gt gt t gt at t gact cat t t t gt at t at
t t t t ggct t acagt t cccat agct gt t agagt ct ggt t t gt t t t t gt t t t t act ct caaaat cat agt aaagat ct ct ca
gt ct cct ggct aaagat t gaaggaaggcaaat ct at t t ct aat t at acat at at cagt aaggat gat ct caacat aat ag
t aat gt gt at ct t t t ggt at ccagt t t t at t t t t ggcct t ct aagaaagt gt ct cat aacacagaacat t gccat t t gct
ct t gt aggcct caaat at gaaagct at t agt cat agagcct aggaaaaaaagaat t gat t aat ggt cct t t t at t t t gt a
acct t at aaat gct gt agat at t at caaaaaaat t t t aat t t cat at t gt t t acat cat gcaact aat ct aagcct caaa
ct cgt t at t ggggct at aaagaaaacgt t t act t acccagct gaaacaggt t aagaat at t ct t aat ct cat t at agat a
at t gcccccat gggact t gaaat acaacacct t gt gct gaaaact t caggt t ggcaat at t t gaaggt t t cgt t gt agaa
gagt t t aacat t aact cct at t t t gact t acaaat ct t gt t t ct cat cact aaaat gct t t t gaat t aat aat ccaaccc
acat gagct gagagt t t t t ct t t t gt t agaaaagaaacagacat ct t t ct gt at gaaagt at aaat t gt at ggt t t t aga
t acat aagaat t gacaaaagcgagcgaaat ct t t gt act t ct gagt t ct t gct gt at gt at gt t t t gt t t t aaat ct gat
t agggacacccagcagct ggccgggat t ct t ggat t gct cct t gggagt t aagat t gt caat act cct gt gaagcaaggg
at t t cagccat agaacaaagat t t at t gt t gccacct gaaaagt t t acaagt at t t at t gt gt at t t gat acat t gct t g
aaaagat gaaat ct gt t aaagat t ct t t t cgat gt ccaggt t aagaagaaacct cct t gt at t gagt gaaat t at at gt t
aaat gt at t agagaat gt aggt ggt at agaaat t gat t t t t ct t ggt gt agaacaact cagt t cggcaaagt t t aaaat t
t gat t aaacaagagaagt ggt t caggt t gaagat ggact t gt t aggaagt gat caagt cct t t aagt act t gt t t ct t t t
t caggt t gt gat gt ggccat t ccgaat t t t gt t gagagt t t ggt t t at aat t gt ct ct t t t gt ct t gt t agt aaacat t c
at t t gcaacagt t t t gaaggt gct gagt ggaaaaccgaaacacat ggt t at t gcgt at t ggacct agaat gaaat aat t g
cct caat at t t aacaacaagccat t ct t at ct caaagat t aaat t cccgaat gt cccat t cgcaaat cat at gcaat t g
aagt gagcagcat gagcat ct gggt cat gagggcct t cat t t acgt aaat t t gt cact aaaacccagt agt agct ct aca
aaat ct t aaact gct gcagt gct caaggagat ggaat at ct t t gt cat t ggt gct gaggagagcat t t cggt agaagaca
gt t gcgcct gaagat t gagt gt aaat cat t caaaccagt ggt t ct cagt gt t ggct gt at acact t t gt agt cact t t gg
aat gt t ggaagacacat cgat gct t gggt t ccgt at gccaagat t ct gat gt t ggt ct ggaat at gagct ggt cat aagg
at t t t t aaaaact t t ct ggt cat t t caat at gct gccaaggt t gagaaccact gt t gt aaaat t cacct t gagt t t t ct c
at ct gcaaaat agaaaaaaaaaaat cct t gct ccct ccct t cact acct cacaaggat at t gagggt aaaggagaaaat a
at gggaaagt gct t gt gccgt ggat gaaaagt gct at t aaaagt caaaggagt gt t ct gt t t caat t cat agt at gat ca
gggaaagt gt aact gagt at act t t gt t gact t gggaaacct ggagcact t t ct t t ggt t ggt t aacgaagcat gcagat
gt ggaagcagacgt t act at t at ccct act at ggt ct t ct gt cat act gagacaggct gt t t t aat t acct ggt t t t aca
t aggaaagaagaaat at t aaggct t aaagt t t gt aat gat caat ggct cat aat t cat t aaat ct t t t cat acaaggaa

SEQ ID NO: 15 - PROTEIN (XP_117294)
MAAPAPGAGAASGGAGCSGGGAGAGAGSGSGAAGAGGRLPSRVLELVFSYLELSELRSCALVCKHWYRCLHGDENSEVWRSLC
ARSLAEEALRTDILCNLPSYKAKIRAFQHAFSTNDCSRNVYIKKNGFTLHRNPIAQSTDGARTKIGFSEGRHAWEVWWEGPLG
TVAVIGIATKRAPMQCQGYVALLGSDDQSWGWNLVDNNLLHNGEVNGSFPQCNNAPKYQIGERIRVILDMEDKTLAFERGYEF
LGVAFRGLPKVCLYPAVSAVYGNTEVTLVYLGKPLDG

### Example 7

### SEQ ID NO:7 (G2)

Another rat cDNA clone, originally derived from the above described SSH analysis of the mammary tumor test system was used for a blast analysis against rat EST databases. Identity of 99% was found to the rat mRNA C0568861. This sequence was used for a blast analysis against human genome databases. An identity of 84% was found to the human mRNA AK025571 representing the locus FLJ21918 which maps on chromosome 16. According to AceView, this gene produces, by alternative splicing, 7 different transcripts altogether encoding 8 different protein isoforms. The corresponding NCBI Reference sequence NM_024939 comprises 4021 nucleotides and encodes a hypothetical protein of 717 amino acids. According to InterPro, the RNA-binding region RNP-1 (RNA recognition motif) motif is found in 5 isoforms from this gene. Many eukaryotic proteins that are known or supposed to bind single-stranded RNA contain one or more copies of a putative RNA-binding domain of about 90 amino acids. This is known as the eukaryotic putative RNA-binding region RNP-1 signature or RNA recognition motif (RRM). RRMs are found in a variety of RNA binding proteins, including heterogeneous nuclear ribonucleoproteins (hnRNPs), proteins implicated in regulation of alternative splicing, and protein components of small nuclear ribonucleoproteins (snRNPs). The motif also appears in a few single stranded DNA binding proteins.
In figure 1, a summary of established data for SEQ ID NO:7 is presented.
This sequence was shown to be differentially expressed in Microarray Analysis comparing samples of metastasizing versus non metastasizing tumors cells as previously exemplified for SEQ ID NO:1 in figure 2. Tumor specific expression was further analyzed in hybridization experiments using Cancer Profiling Arrays (CA) from Clontech (http://www.bdbiosciences.com). Estimations of percentages of upregulation in the tissues analyzed is shown in figure 5. FLJ21918 shows significant upregulation (in more than 50% of analyzed pairs) in tissues derived from cancers of the uterus and ovary.

In order to functionally examine whether FLJ21918 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6-8, demonstrate that also overexpression of FLJ21918 leads to increased proliferation, its downregulation results in decreased proliferation. FLJ21918 also affects invasion potential of tumor cells, as observed in experiments performed according to those exemplified for SEQ ID NO:1 in figure 9.

In summary, FLJ21918 shows upregulation in metastasizing tumor cells versus non metastasizing tumor cells, and also displays upregulated expression in various tumor tissues versus normal tissue samples. Moreover, FLJ21918 is functionally involved in processes involved in tumor progression like increased proliferation and invasion. Therefore, this sequence may particularly be useful for staging of human tumor diseases, as well as for decisions on prognosis and treatment modalities. Furthermore, the FLJ21918 gene and its gene products may be used as target structures to develop therapeutic anti-cancer drugs.

SEQ I D NO: 7 ( NM_024939)
ggt agccgccccgccccgcggggcgccacgggcgggt ct t ggcagcgcccact gagccagccgggccgcaggt gccgccc
ccgat acacggt gt cccgcccaagct gat ccgcgt ct gcggt cggt cggt gcgt gcgt gcgcct cgt cggt ccgcgt gt c
t ggccgagagcccct t cct ct gcggccat gact ccgccgccgccgccgcccct cccccgggccct gaccccgcggccg
accccgccgcggacccct gcccct ggcccggat cact ggt cgt cct ct t cggggct acggcgggt gcgct gggacgggac
ct gggct cggacgagaccgact t aat cct cct agt t t ggcaagt ggt t gagccgcggagccgccaggt ggggacgct gca
caaat cgct ggt t cgt gccgaggcggccgcact gagt acgcagt gccgcgaggcgagcggcct gagcgccgacagcct gg
cgcgggcagagccgct ggacaaggt gct gcagcagt t ct cacagct ggt gaacggggat gt ggct t t gct gggcgggggc
ccct acat gct ct gcact gat gggcagcagct at t gcgacaggt cct gcaccccgaggcct ccaggaagaacct ggt gct
ccccgacat gt t ct t ct cct t ct at gacct ccgaagagaat t ccat at gcagcat ccaagcacct gccct gccagggacc
t cact gt ggccaccat ggcacagggt t t aggact ggagacagat gccacagaggat gact t t ggggt ct gggaagt caag
acaat ggt agct gt t at cct ccat ct act caaagagcccagcagt caat t gt t t t cgaagcccgaggt gat aaagcagaa
at acgagacggggcct t gcagcaaggct gat gt ggt ggacagt gagact gt ggt acgggct cgt gggt t gccgt ggcagt
cat cagaccaggacgt ggct cgct t ct t caaagggct caacgt ggccaggggt ggt gt agcact ct gcct caacgcccag
ggccgcagaaat ggcgaggccct cat ccgct t t gt ggacagcgagcagcgggacct agcgct gcagagacacaagcacca
cat gggcgt ccgct at at t gaggt gt at aaagcgacaggggaggagt t t gt aaagat t gcagggggcacat cact agagg
t ggct cgt t t ct t gt cacgggaagaccaagt gat cct gcggct gcggggact gccct t ct cggct gggccaacggacgt g
ct t ggct t cct ggggccagagt gcccagt gact gggggt accgaggggct gct ct t t gt gcgccat cct gat ggccggcc
gact ggt gat gcct t cgccct ct t t gct t gt gaggagct ggcacaggct gcact gcgcaggcacaagggcat gct gggt a
agcgat acat t gaact ct t ccggagcact gcagccgaagt gcagcaggt ct t gaaccgct at gcat ccggcccact cct t
cct acact gact gccccact gct gcccat cccct t cccact ggcacct gggact gggagggact gt gt acgcct ccgagg
cct gccct acacggccaccat t gaagacat cct gagct t t ct gggggaggcagcagct gacat t cggccccacggt gt ac
acat ggt gct caaccagcagggccggccat cgggcgat gcct t cat t cagat gacat cagcagagcgagccct agct gct
gct cagcgt t gccat aagaaggt gat gaaggagcgct acgt ggaggt ggt cccct gt t ccacagaggagat gagccgagt
gct gat ggggggcacct t gggccgcagt ggcat gt cccct ccacccct gcaagct gccct gcct ct caccacct acct aca
ccacct t ccaagccaccccaacgct cat t cccacggagacggcagct ct at acccct ct t cagcact gct cccagct gcc
agggt gcct gct gccccccacccct gt t gcct act at ccagggccagccact caact ct acct gaact acacagcct act a
cccaagccccccagt ct cccccaccact gt gggct acct cact acacccact gct gccct ggcct ct gct cccacct cag
t gt t gt cccagt caggagcct t ggt ccgcat gcagggt gt cccat acacggct ggt at gaaggat ct gct cagcgt ct t c
caggcct accagct acccgct gat gact acaccagt ct gat gcct gt t ggt gacccacct cgcact gt gt t acaagcccc
caaggaat gggt gt gt t t gt aggagagaaagccaggaggt aagagccagct gat at cct cggcgaacat gt ct ct cct ga
gt ccagaagaccagcaccct caacct ggt agct t ct t t ct ggct t gt caaagct ct cagaaggt acct agaggagcccaa
gccccagct ccat cct ccact t at t ct gcct gt t t cccccaaagacaat ggct ggaccct gcat gcagggct gggggt gg
aat ggggct aaccagct cct gat ggcct gagccaggcat ct t gact ggcacct ggagagccct t aagt ct gt cct ggct g
t ggcccat gccgacagat at cgt ggggct gacaggt ccacggcaggct t gct t t ct t t t at aaaat ggaagct ct ggt ac
ct t caat gt at gact cct gggagaat caagggt ccat ct gagcct ct gagt aaagat cccaat gt t ct acct ct ccct gt
ccct ct t gt aggggat agggaggcagagagagccagcccct accct cagagt at ct ggacct cagagaccat gt t gt gcc
aggggt ggt cccacct aaagat gct agcccct ct ccaggt gggcat aaggagt aacagat ggcaaaccacaaact at t t
t gat ggact gt gct gcagt at caccagaagacat t agggggcagt aggcccccacacaaaacct t caggct t gaat t t t a
aaggggaggact t t ct gccaact t t t ct t gt at gcct t gggaaagccagt t gccct gaacccagcagacaccat ggaat g
t cct t t gcacgcat t aaat ggt acagaact gaagcct cggaagcaat t ggaact cgat ct t ct ct t cct t aaat gaaaa
gt t at t gaccaaat ggact t t t t aaaagacacaggaccct t aact t t gccccaaagt gaggggct ccacaccaaccccag
gcggaggaacact cagacagat t aaggat act gt t gacct gt cact gt t t at t at t t cagcact aaaact gaggagcct c
aact gct ggct ct t ct t ccct t t gt at t t gt gt aaggagcact gcact cccat aaaaggt t t t aaaat acaaaat gt aca
agaacacacaat t ccaagt gct gt aaacat aact gagaaccagt t cct t t act aaacat ccat t t t at aaaacacaaggt
t t caat t t gagcccat ct gagcct t aaagat ccat t ct gaat accaaaaacagggct t cacagccaggcccagaagaggt

ct ggt gat aat ggct ggccct gggt ggggat agt t t acacccgggcagcagcaccacacat gaacccaaagacat gt t ct
t t t t aaagct gt t t t cagccat gt t t ct ct gt gcat ct ccagt aagcagaaggct acccat t ccat t cct caacccaaga
gct agcacagt t agagt aggagggggt gcgt act agcacgt gcccagt t gct cagt gct gct agt agaaat t gat t t gca
t agt ccaat ggat gt gt gct t t aacaccact at gt t gcacaaaaat t t aagt ct t t at ct acaaagccaaaaaat at t ga
ct ct t aacaccaaagct t t t acaaagct gat at aaaact gct t acat agt at acaaagct ct at t t t aaaat t t aat gt t
t at t t t aaat aggaaagcat t

SEQ ID NO: 16 - PROTEIN (NP_079215)
MTPPPPPPPPPGPDPAADPAADPCPWPGSLVVLFGATAGALGRDLGSDETDLILLVWQVVEPRSRQVGTLHKSLVRAEAAALS
TQCREASGLSADSLARAEPLDKVLQQFSQLVNGDVALLGGGPYMLCTDGQQLLRQVLHPEASRKNLVLPDMFFSFYDLRREFH
MQHPSTCPARDLTVATMAQGLGLETDATEDDFGVWEVKTMVAVILHLLKEPSSQLFSKPEVIKQKYETGPCSKADVVDSETVV
RARGLPWQSSDQDVARFFKGLNVARGGVALCLNAQGRRNGEALIRFVDSEQRDLALQRHKHHMGVRYIEVYKATGEEFVKIAG
GTSLEVARFLSREDQVILRLRGLPFSAGPTDVLGFLGPECPVTGGTEGLLFVRHPDGRPTGDAFALFACEELAQAALRRHKGM
LGKRYIELFRSTAAEVQQVLNRYASGPLLPTLTAPLLPIPFPLAPGTGRDCVRLRGLPYTATIEDILSFLGEAAADIRPHGVH
MVLNQQGRPSGDAFIQMTSAERALAAAQRCHKKVMKERYVEVVPCSTEEMSRVLMGGTLGRSGMSPPPCKLPCLSPPTYTTFQ
ATPTLIPTETAALYPSSALLPAARVPAAPTPVAYYPGPATQLYLNYTAYYPSPPVSPTTVGYLTTPTAALASAPTSVLSQSGA
LVRMQGVPYTAGMKDLLSVFQAYQLPADDYTSLMPVGDPPRTVLQAPKEWVCL

### Example 8

### SEQ ID NO:8 (L1)

Another rat cDNA clone, originally derived from the above described SSH analysis of the mammary tumor test system was used for a blast analysis against rat EST databases. 100% identity was found to the rat EST AW919679. This EST was used for a blast analysis against mouse genome databases. Identity of 90% was found to the mouse mRNA AK088107. The protein encoded by this RNA shows 90% identity on the amino acid level to the human hypothetical protein NP_620129 encoded by the locus C19orf22, alias MGC16353. The corresponding NCBI Reference sequence NM_138774 comprises 1810 nucleotides and encodes a hypothetical protein of 166 amino acids. According to AceView, it produces, by alternative splicing, 8 different transcripts altogether encoding 7 different protein isoforms. PSORT II analysis, trained on yeast data, predicts that the subcellular location of this partial protein is expected to be in the nucleus (56%). The following domain was found: PKAKGRK.
Pfam analysis shows that this protein does not belong to any recognized protein family.
In figure 1, a summary of established data for SEQ ID NO:8 is presented.
This sequence, C19orf22, was shown to be differentially expressed in Microarray Analysis comparing its expression in metastasizing versus non metastasizing tumor cells as previously exemplified for SEQ ID NO:1 in figure 2. Tumor specific expression was further analyzed in hybridization experiments using Cancer Profiling Arrays (CA) from Clontech (http://www.bdbiosciences.com). Estimated percentages of upregulation in the tissues analyzed is shown in figure 5. C19orf22 shows significant upregulation (in more than 50% of analyzed pairs) in tissues derived from cancer of the uterus, ovary, colon, rectum and lung.

In order to functionally examine whether C19orf22 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6-8, demonstrate that also overexpression of C19orf22 leads to increased proliferation, its downregulation results in decreased proliferation. C19orf22 also affects invasion potential of tumor cells, as observed in experiments performed according to those exemplified for SEQ ID NO:1 in figure 9.

In summary, C19orf22 shows upregulation in metastasizing tumor cells versus non metastasizing tumor cells, and also displays upregulated expression in various tumor tissues versus normal tissue samples. Moreover, C19orf22 is functionally involved in processes involved in tumor progression like increased proliferation and invasion. Therefore, this sequence may particularly be useful for staging of human tumor diseases, as well as for decisions on prognosis and treatment modalities. Furthermore, the C19orf22 gene and its gene products may be used as target structures to develop therapeutic anti-cancer drugs.

SEQ I D NO: 8 ( NM_138774)
gaaggccct gccgggcggcggcggcgacagcgt gcgagccat ggt cgcgct ggagaaccccgagt gcggcccggagg
cggcggagggcaccccgggcgggcggcggct gct gcccct t cccagct gcct gcct gccct agccagct cccaggt gaag
agact ct cggct t ccaggcggaaacagcact t cat caaccaggcagt gcggaact cagacct cgt gcccaaggccaaggg
gcggaagagcct ccagcgcct ggagaacacccagt acct cct gaccct gct ggagacagacgggggcct gcct ggcct gg
aggat ggggact t ggcacccccct gcat caccaggcat ct t t gccgaggcct gcaacaacgccacct at gt ggaggt ct gg
aacgat t t cat gaaccgct ccggggaggagcaggagcgggt t ct t cgct acct ggaggat gagggcaggagcaaggcgcg
gaggaggggccct ggccgt ggggaggaccggaggagagaggaccccgcct at acaccccgcgagt gct t ccagcgcat ca
gccggcgt ct gcgagccgt cct caagcgcagccgcat ccccat ggaaacgct ggagacct gggaggagcggct gct t cgg
t t ct t ct ccgt gt ccccccaggccgt gt acacagcaat gct agacaacagct t cgagaggct t ct gct gcacgct gt ct g
ccagt acat ggacct cat ct cggccagt gct gacct ggaggggaagcggcagat gaaggt cagt aat cggcacct ggat t
t cct gccgccggggct gct cct gt ccgcct acct ggagcagcacagct gat ggcggccccgcggagaccccgct gccacc
t cgcccagccat caagccct ccgat acct t cggct aaaat at ct t t cat at t t t t agaat t t gt cct cggaaacct t t t t
cgct t ggggt ggt ct ct ct cact ct gcccccct cct cacgcagct ct t ggcagt caacagacgct ggcggct ggggct gcc
cat gccat cccagct ccaagct t cccact ccgggact t gt gt t t gggt ggggagacct gacct gggcat gt t cct gt t t c
t t cat cgt t gagct t t t ct ggcccggt ct gaagct caagt gaggaggggggaggct gggt t t t t at cact t t t aat gaat t
t ggt gt gat t t gt t gt agat t t t t aaat t t ccct t t t ggagagaaaaaccaaaaaaact cgcccccact ggt aaaacat gg
gt ct t ggt cccagcccct gct cagcccct cccagt t t t t agct t gaat gagggt ggggt ct ct gggaccct gcccct cat
gccagaagcat ct t gt gt t gt at at gt gt gcgcgcgt gt gccct gagacccaggacagaagccacggt cct aagagccgg
t t t t at cct cgt cat t ct gcgt gt cct cccccacgccacct gt gt cggggct cagggt ct cct gct t t at at gagccccc
t t cct t t cct cccct cct t t at gct gggggt ccaggact t ccagccagaagcct ct gccct t gcact acct t gt ct gt ca
ccccat cccgt gt cccct cgt cccccagcct gact cct gcct gat agct cct gt gt ccccat gct ggt cct cct ggccca
ggct gcaggagccaggct gggggcct ccgcacccccct t gct gcgt gt gggt aat t gt gt t t t gggggaaagt ggggaat

t t aat aaat t t ct ggt gct ct ggcaaaaaaaaaaaaaaaaaaaaaaaaaaaaaa.

SEQ ID NO: 17 - PROTEIN (NP_620129)
MVANCGAAGTGGRRSCAASSVKRSASRRKHNAVRNSDVKAKGRKSRNTYTTDGGGDGDAASGAACNNATYVVWNDMNRSGRVR
YDGRSKARRRGGRGDRRRDAYTRCRSRRRAVKRSRMTTWRRSVSAVYTAMDNSRHAVCYMDSASADGKRMKVSNRHDGSAYHS

### Example 9

### SEQ ID NO:9 (G4)

Another rat cDNA clone, originally derived from the above described SSH analysis of the mammary tumor test system was used for a blast analysis against rat EST databases. 84% identity was found to the rat RNA BC030338 representing the locus LOC292139. The protein encoded by this locus shows 77% identity to the hypothetical human protein NP_060800 representing the locus KIAA1598. The corresponding NCBI reference mRNA for this locus NM_018330 comprises 3417 nucleotides and encodes a hypothetical protein of 456 amino acids which maps on chromosome 10. According to AceView, this gene produces, by alternative splicing, 11 different transcripts altogether encoding 11 different protein isoforms. PSORT II analysis predicts that the subcellular location of this protein is expected to be in the nucleus (60%). Pfam Search shows that the amino-terminus of the protein shares homology with the SMC domain of Chromosome segregation ATPases.
In figure 1, a summary of established data for SEQ ID NO:9 is presented.
This sequence was shown to be differentially expressed in Microarray Analysis comparing samples of metastasizing versus non metastasizing tumor cells as previously exemplified for SEQ ID NO:1 in figure 2. Tumor specific expression was further analyzed in hybridization experiments using Cancer Profiling Arrays (CA) from Clontech (http://www.bdbiosciences.com). Estimated percentages of upregulation in the tissues analyzed is shown in figure 5. KIAA1598 shows significant upregulated expression (in more than 50% of analyzed pairs) in tissues derived from cancers of the uterus, ovary, colon and rectum.
In order to functionally examine whether KIAA1598 could be causally involved in the progression of tumor progression, it was transiently overexpressed or transiently downregulated by RNA interference in HEK-293T cells and subsequently potential resulting influences on tumor cell properties were assayed. For its overexpression a sequence corresponding to the NCBI reference sequence (http://www.ncbi.nlm.nih.gov/RefSeq/) was used. Experiments as previously exemplified for SEQ ID NO:1 in figures 6-8, demonstrate that also overexpression of KIAA1598 leads to increased proliferation, its downregulation results in decreased proliferation. KIAA1598 also affects invasion potential of tumor cells, as observed in experiments performed according to those exemplified for SEQ ID NO:1 in figure 9.

In summary, KIAA1598 shows upregulation in metastasizing tumor cells versus non metastasizing tumor cells, and also displays upregulated expression in various tumor tissues versus normal tissue samples. Moreover, KIAA1598 is functionally involved in processes involved in tumor progression like increased proliferation and invasion. Therefore, this sequence may particularly be useful for staging of human tumor diseases, as well as for decisions on prognosis and treatment modalities. Furthermore, the KIAA1598 gene and its gene products may be used as target structures to develop therapeutic anti-cancer drugs.

SEQ I D NO: 9 (NM_018330)
cgaggct ggcat agcggct gccgacccgcct t cgt t cct ccaccccct gcacgggact gct gggcccgccccgccccgcc
t gcaggt gaagcggccgcagccgccgagt aggt gcgt ggggat gat ct cact cgcgcgct ccgcgccaggaggaggagga
gcgggagcggat ccaact t ccgggt agt ggagccgcaagccaccggcat ct t gct t t t ct t cccccct cct cct gt gt gc
cccgcgccgct ccct ct t t ccct t t t at t cccggccccacccgccaaaat gaacagct cggacgaagagaagcagct gca
gct cat t accagt ct gaaggagcaagcaat aggcgaat at gaagacct t agagcagagaaccagaaaacaaaggagaagt
gt gacaaaat t aggcaagaacgagat gaagccgt t aaaaaact ggaagaat t t cagaaaat t t ct cacat ggt cat agag
gaagt t aat t t cat gcagaaccat ct t gaaat agagaagact t gt cgagaaagt gct gaagct t t ggcaacaaagct aaa
t aaagaaaat aaaacgt t gaaaagaat cagcat gt t gt acat ggccaagct gggaccagat gt aat aact gaagagat aa
acat t gat gat gaagat t cgact acagacacagacggt gccgccgagact t gt gt ct cagt acagt gt cagaagcaaat t
aaagaact t cgagat caaat t gt at ct gt t caggaggaaaagaagat t t t agccat t gagct ggaaaat ct caagagcaa
act cgt agaagt aat t gaagaagt aaat aaagt t aaacaagaaaagact gt t t t aaat t cagaagt t ct t gaacagagaa
aagt ct t agaaaaat gcaat agagt gt ccat gt t agct gt agaagagt at gaggagat gcaagt aaacct ggagct ggag
aaggacct t cgaaagaaagcagagt cat t t gcacaagagat gt t cat t gagcaaaacaagct aaagagacaaagccacct
t ct gct gcagagct ccat ccct gat cagcagct t t t gaaagct t t agacgaaaat gcaaaact cacccagcaact t gaag
aagagagaat t cagcat caacaaaaggt caaagaat t agaagagcaact agaaaat gaaacact ccacaaagaaat acac
aacct caaacagcaact ggagct t ct agaggaagat aaaaaggaat t ggaat t gaaat at cagaat t ct gaagagaaagc
cagaaat t t aaagcact ct gt t gat gaact ccagaaacgagt gaaccagt ct gagaat t cagt acct ccaccacct cct c
ct ccaccaccact t cccccct ccacct cccaat cct at ccgat ccct cat gt ccat gat ccggaaacgat cccaccccagt
ggcagt ggt gct aagaaagaaaaggcaact caaccagaaacaact gaagaagt cacagat ct aaagaggcaagcagt t ga
agagat gat ggat agaat t aaaaagggagt t cat ct t agacccgt t aat cagacagccagaccgaagacaaagccagaat
ct t cgaaaggct gcgaaagt gcagt ggat gaact aaaaggaat act ggcct cccagt agcat t ggat gcaggaaaaaat a
cat t gacggt gaaaaacaagccgaaccagt t gt agt t t t agat cct gt t t ct acacat gaaccccaaaccaaagaccagg
t t gct gaaaaagat ccaact caacacaaggaggat gaaggcgaaat t caaccagaaaacaaagaagacagcat t gaaaac
gt gagagagacagacagct ccaact gct gat ccat aaaccagaagcct gat acgt t t ggaagt cct t t t caat aagcaca
t gat t agt gt t gt t at at t ggcaagggct gt agacat t ct gct ct ggt cact gt at t cagaat acaggt t ct t t t ct ggt
gt cact t t t gt aagt agcaact at aaacat aagt aagct gt t t agcaaaacacacat t cct agt aggt t t t ggt t t t t t g
at ct t t at aaagat gaggt t t t t t t cct agt t act gt at t aagt at gact t ct t t t agaaggt t acaaaaaaat t cagat
gt t gat acct t t t t aggaaat gt gcat accact cat caaat ggaat gct gaaagt t t gaggt gct t gt at at aat cggat
aaacaaaact gat caacccaat gt gat t t t aaaagcccccaaagaagct t ct gt t t t gggt ct gat cct ct t gat ggaga
aact gcagcagcat ggaaat t gt t gggt act gt ggcat acaagt t at t t t ct acagt agact gagat aaact gaaaact c
aggagct ggcat caaact cgt agt cccat agt cagt gt t aat t acacacat t gt t aact at t ggat gaaaaat acat gct
at t gat t gt gt ccaaagcct cccgaggacct ccgt ggggat gct ct ggt agcct gaat acagaact gaggt gaaagt cca
aacct t gaat t t t acagt agt aagt t ggt aaaccat gt gct ct gt gct at gagt t aat t at gt t t t cccaaat act aat g
t ggcacaagt accat at t t t at cagagt t ct t at gt acagt at ggt gaagat aagt gacaagcacacat t t t t ct t gct t
cact gct gt t ct at at t acacaggt t t gt t gt t gt t t t t t t t aaaaaagaat t aagcagt agt t agt ct ct aaaaat ac

aat gt t t caggct accacagt gaat aaat agaaat gt aat cagggat t aaaaaaaaaact t at gcagct t t t caaagt t g
at t gt t t caaaat t ggt gt t t at t t aaaat aagt ggt aat gt act t gaat gcact t t t t at gacaat gat t cagt aat gg
t aat t t t act at t aaagaaagt gaaaggt t t agt t t t gt t agcat ggct cagcat gt agct gt caggt gt t t t t cacct a
agggcaaaagaaaat gat agt aat aat t gcagt agt t gt at t gt at t gt at t t t t gcacgt gt ggt aagcat aggct t ga
agaggt gggt aggcaggt acat gt act t cct aaat t t t gagat aat t at ct t t ct gt aagt t cgt t at gct t gact gt t t
ccat gt t ct cccaat aat gat t t t at agt t act t at cact t t act cat ggagaat t aaaacgt aat gt t t t t caact gt a
t ct t t ct t t aact ggat aat act gct at at gat at gct t act acagact gcat t aat t cacgaaacgaat t ct gt t at gc
t gt aat t t gaact ct cct caccacaact t at t aaaaaggcaccaat agt t t cccat t

SEQ ID NO:18 - PROTEIN (NP_060800)
MNSSDEEKQLQLITSLKEQAIGEYEDLRAENQKTKEKCDKIRQERDEAVKKLEEFQKISHMVIEEVNFMQNHLEIEKTCRESA
EALATKLNKENKTLKRISMLYMAKLGPDVITEEINIDDEDSTTDTDGAAETCVSVQCQKQIKELRDQIVSVQEEKKILAIELE
NLKSKLVEVIEEVNKVKQEKTVLNSEVLEQRKVLEKCNRVSMLAVEEYEEMQVNLELEKDLRKKAESFAQEMFIEQNKLKRQS
HLLLQSSIPDQQLLKALDENAKLTQQLEEERIQHQQKVKELEEQLENETLHKEIHNLKQQLELLEEDKKELELKYQNSEEKAR
NLKHSVDELQKRVNQSENSVPPPPPPPPPLPPPPPNPIRSLMSMIRKRSHPSGSGAKKEKATQPETTEEVTDLKRQAVEEMMD
RIKKGVHLRPVNQTARPKTKPESSKGCESAVDELKGILASQ

### Example 10

All clones were used to perform blast analyses using gene sequence databases. Out of these investigations, in summary, 89 of 235 deduced human sequences were chosen and corresponding cDNAs were spotted with 4 ng per spot onto glass slides (Cornings CMT ULTRAGaps slides), to create a diagnostic, a so called cDNA chip. Subsequent hybridization experiments showed that all of these 89 sequences are differentially expressed in at least one of several pairs of metastasizing and non metastasizing cells, such as, e.g., in five pairs of primary tumor and metastasis samples from colon cancer patients.
In addition, the expression patterns of these 89 sequences in established cell lines displaying different metastasizing potentials were analysed. The following cell lines were utilized for this purpose:
- The non metastasizing colon cancer cell line SW480 and the metastasizing colon cancer cell line SW620 (ATCC CCL-227 and -228).
- The non metastasizing colon cancer cell line HT29mtx and the metastasizing colon cancer cell line HT29 (Lesuffleur, 1990, Cancer Res. 50, 6334-6343).
- The non metastasizing mammary cell line T47D and the metastasizing mammary cancer cell line MDA-MB-231 (ATCC HTB-133 and -26).
- The non metastasizing endometrial cancer cell line HEC-1A and the metastasizing endometrial cancer cell line AN3-CA (ATCC HTB-112 and -111).
- The non metastasizing prostate cancer cell line LNCap and metastasizing prostate cancer cell line DU145 (ATCC HTB-81 and CRL-1740).
- The non metastasizing pharynx carcinoma line FaDu and the Detroit-562 line established from a metastatic site of a pharynx carcinoma (ATCC HTB-43 and CCL-138).

Accession numbers of all sequences that showed differential expression at least in one of these systems in microarray analysis are listed in figure 1 which also contains information on differential expression of the single sequences established by "In situ hybridisation" (ISH) technology of matched human tumors (BioCat BA3, http://www.biocat.de). Three sequences were tested for their expression patterns on these slides and showed tumor specific expression in at least two tissue types. Tumor specific expression patterns were further analyzed in hybridization experiments using Cancer Profiling Arrays (CA) from Clontech (http://www.bdbiosciences.com). These Cancer Profiling Arrays include normalized amplified cDNAs from 241 tumor tissues and corresponding normal tissues from individual patients, along with negative and positive controls, and also cDNAs from nine cancer cell lines. In these experiments, overexpression of a given gene in these Cancer profiling Assays was defined as upregulation of expression in the tumor probe versus the normal probe in at least 50% of analyzed pairs which were analysed in at least 3 of 8 different tissues analysed. 25 of the 89 sequences listed in figure 1 were tested in the Cancer profiling Arrays; 9 of those showed tumor specific expression patterns according to the above mentioned criteria. Furthermore, figure 1 contains information on indications for functional involvement of the sequences listed in metastatic processes. A positive mark in this context was defined as displaying an at least 20% modification of activity over control values in at least one functional assay. For further detailed information on functional assays performed see figures 6-9.

An example of a gene-chip hybridization experiment utilizing cDNAs from the endometrial cancer cell line HEC-1A and the metastasizing endometrial cancer cell line AN3-CA (ATCC HTB-112 and -111) is shown in figure 2.

In summary, all sequences listed in figure 1 display metastasis specific expression patterns in hybridisation experiments. 9 of these sequences (designated SEQ ID NO:1-9) were tested positive for 2 further criteria of causal relevance and their involvement in the process of tumor progression.

These findings show, that this cDNA chip comprising the listed sequences of figure 1 can be used as a diagnostic and prognostic tool. It will enable the investigator to conclude about the presence of metastatic tumor cells in the body of a patient, and furthermore, might predict in future the therapeutic outcome of a given therapy, given that the therapy interferes with the presence or absence of one or several of the molecular cancer antigens presented in this invention and represented as cDNA on the corresponding diagnostic cDNA chip described above. In case a cancer antigen directly represents an anti-cancer target structure, than the therapeutic outcome might directly be measurable based on the activity or expression of this cancer antigen, e.g., if this cancer antigen is attacked therapeutically directly or indirectly by the therapeutic agent.
A therapeutic modulation of a cancer antigens function could be established by interfering with the expression of such a cancer antigen by e.g., including but not limited to, utilizing means of anti-sense RNA, RNAi or catalytic RNA technologies, or by various DNA or modified DNA oligonucleotide approaches.
Alternatively, antibodies directed against these cancer antigens could be suitable anti-cancer drugs, or drugs that interfere with activities, such as, but not limited to, enzymatic or structural activities, of these cancer antigens, or their existing localization specifications. Also, drugs which act on signalling pathways which are influenced by these cancer antigens could give rise to potent anti-cancer drugs.
In a particular embodiment of this invention, such therapeutic approaches could be suitable for the treatment of metastatic cancer disease, or for the prevention or suppression of metastatic tumor progression, and for the treatment, prevention and suppression of minimal residual tumor disease.

### Figures

Figure 1: List of cancer antigens identified, characterized and presented in this invention.
Figure 2: Raw Microarray analysis data from hybridization tests.
Figure 3: Data of ISH (In Situ Hybridization) experiments with Digoxygenin labelled RNA probes from the MEP 50 locus (SEQ ID NO:1) are presented.
Figure 4: The cancer profiling expression analysis (CA) for SEQ ID NO:1 (MEP50) is presented.
Figure 5: Summary data for the cancer profiling expression analysis (CA) for SEQ ID NO:1-9.
Figure 6: Data from proliferation assays (A) with transiently transfected HEK-293T cells.
Figure 7: Proliferation assay using siRNA treated HEK-293T cells.
Figure 8: Proliferation assays using overexpression studies.
Figure 9 : Invasion assay with stably transfected HT29 colon cancer cells.

## Claims

**1.** A method for diagnosing a disease or condition, or a susceptibility to a disease or condition, comprising the step of determining the expression, activity or mutations of at least one polynucleotide or expression product thereof in a first biological sample from a first subject, wherein said at least one polynucleotide comprises
(i) a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9 and the corresponding RNA sequences,
(ii) a sequence complementary to any one of the sequences under (i), or
(iii) a variant of any one of the sequences under (i) or (ii).

**2.** A method according to claim 1, wherein said at least one polynucleotide comprises a sequence encoding a polypeptide having a sequence selected from the group consisting of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO:18.

**3.** A method according to claim 1 or 2, wherein said expression product is a polypeptide comprising a sequence selected from the group consisting of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO:18.

**4.** A method according to any one of claims 1 to 3, comprising determining the expression or activity of said at least one polynucleotide or expression product thereof in said biological sample.

**5.** A method according to claim 4, wherein the step of determining the expression of at least one polynucleotide comprises determining the presence and/or amount of said at least one polynucleotide or expression product thereof in said biological sample.

**6.** A method according to any one of claims 1 to 3, wherein the step of determining the mutations consists of determining the presence or absence of one or more mutations in the nucleotide sequence of said at least one polynucleotide in said biological sample.

**7.** A method according to any one of claims 1 to 6, comprising the use of hybridization technology.

**8.** A method according to any one of claims 1 to 7, wherein the step of determining the expression of at least one polynucleotide in said sample comprises utilizing at least one recombinant polynucleotide.

**9.** A method according to claim 7 or 8, wherein a solid support on which at least one isolated polynucleotide is immobilized is contacted with said sample, and the isolated polynucleotide comprises a sequence as defined in claim 1.

**10.** A method according to claim 9, wherein at least 9 different isolated polynucleotides are immobilized on said solid support, and said 9 different isolated polynucleotides have the nucleotide sequences as shown in SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO:9, respectively.

**11.** A method according to claim 10, wherein at least 89 different isolated polynucleotides are immobilized on said solid support, and said 89 isolated polynucleotides have the nucleotide sequences as outlined in Figure 1.

**12.** A method according to any one of claims 1 to 6, comprising utilizing an antibody directed against a polypeptide selected from the group consisting of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO:18.

**13.** A method according to any one of claims 1 to 12, further comprising the step of comparing said expression or activity in said first sample with the expression or activity of said polynucleotide or expression product thereof in a second sample which was obtained from tissue which is not affected by said disease.

**14.** A method according to claim 13, wherein an increased expression or activity in said first sample compared to the expression or activity in said second sample is diagnostic of the disease.

**15.** A method according to any one of claims 1 to 14, wherein the disease is a tumor disease.

**14.** A method according to claim 13, which is a method for testing the presence of tumor cells in the subject's body.

**15.** A method according to claim 14, which is a method for testing, whether the subject's body contains tumor cells with an increased metastatic potential.

**16.** A method according to any one of claims 1 to 15, wherein the disease is selected from the group consisting of estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis, sarcomas, Kaposi's sarcoma, osteosarcoma, head and neck cancer, small cell lung carcinoma, non-small cell lung carcinoma, leukemias, lymphomas, or other blood cell cancers, ichtiosis, acne, acne vulgaris, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and obesity.

**17.** A method according to any one of claims 1 to 16, in which a prognostic conclusion can be made about the subject's disease.

**18.** A method according to any one of claims 1 to 16, in which a prognostic conclusion can be made about the subject's response to a therapeutic treatment by monitoring of the clinical effectiveness of the treatment.

**19.** A method for identifying compounds which modulate the expression or activity of any of the polynucleotides or expression products thereof as defined in any one of claims 1 to 3, comprising
(a) contacting a candidate compound with cells which express said polynucleotide or a polypeptide encoded thereby, or with cell membranes comprising said polypeptide, or respond to said polypeptide; and
(b) determining the effect of said candidate compound on the expression, activity, cellular localization or structural condition of said polynucleotide or polypeptide; or determining a functional response of said cells.

**20.** A method according to claim 19, wherein the step of determining the effect comprises comparing said expression, activity, cellular localization or structural condition of said polynucleotide or polypeptide with the expression, activity, cellular localization or structural condition of said polynucleotide or polypeptide in cells which were not contacted with the candidate compound.

**21.** A method according to claim 20, wherein the candidate compound is selected if the expression of said polynucleotide or polypeptide in the cells which were contacted with the candidate compound is lower than in the cells which were not contacted with the candidate compound.

**22.** A method according to any one of claims 19 to 21, further comprising comparing the viability of the cells which were contacted with the candidate compound and the viability of cells which were not contacted with the candidate compound.

**23.** A compound identified by a method according to any one of claims 19 to 22, wherein said compound is a compound which antagonizes or agonizes any one of the polynuleotides or expression products thereof as defined in any one of claims 1 to 3.

**24.** A compound according to claim 23 which is an antisense nucleic acid capable of suppressing the expression of any one of the polynucleotides or expression products thereof as defined in any one of claims 1 to 3.

**25.** A solid support on which at least one isolated polynucleotide is immobilized, wherein said isolated polynucleotide has
(i) a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, and fragments thereof,
(ii) a sequence complementary to any one of the sequences under (i), or
(iii) a sequence which is an allelic variant of any one of the sequences under (i) or (ii).

**26.** A solid support according to claim 25, wherein at least 9 different isolated polynucleotides are immobilized on said solid support, and said 9 different isolated polynucleotides have the nucleotide sequences as shown in SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO:9, respectively; or the corresponding complementary sequences.

**27.** A solid support according to claim 26, wherein at least 89 different isolated polynucleotides are immobilized on said solid support, and said 89 isolated polynucleotides have the nucleotide sequences as outlined in Figure 1.

**28.** The use of a polynucleotide or polypeptide as defined in any one of claims 1 to 3, or of a compound according to claim 23 or 24, in the manufacture of a medicament for the treatment or prevention of a disease associated with increased activity or expression of a polynucleotide or polypeptide as defined in any one of claims 1 to 3.

**29.** The use according to claims 28, wherein said disease is selected from the group consisting of estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis, sarcomas, Kaposi's sarcoma, osteosarcoma, head and neck cancer, small cell lung carcinoma, non-small cell lung carcinoma, leukemias, lymphomas, or other blood cell cancers, ichtiosis, acne, acne vulgaris, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and obesity.

**30.** The use according to claim 28 or 29, wherein said treatment comprises
(a) administering to a subject a therapeutically effective amount of a compound which causes a decrease in the expression of said polynucleotide; and/or
(b) administering to the subject a therapeutically effective amount of an antagonist to said polypeptide; and/or
(c) administering to the subject a therapeutically effective amount of an agonist to said polypeptide; and/or
(d) administering to the subject a nucleic acid molecule that inhibits the expression of the nucleotide sequence encoding said polypeptide; and/or
(e) administering to the subject a polynucleotide as defined in any one of claims 1 to 3; or a nucleotide sequence complementary to said nucleotide sequence in a form so as to effect production of said thereof encoded polypeptide activity in vivo; and/or
(f) administering to the subject a therapeutically effective amount of a polypeptide that competes with said polypeptide for its ligand, substrate, or receptor; and/or
(g) administering to the subject a therapeutically effective amount of an antibody directed against said polypeptide.

**31.** The use according to any one of claims 28 to 30 **characterized in that** the progression of a subject's disease to metastatic tumor progression is suppressed by said treatment.

**32.** The use according to any one of claims 28 to 31, wherein said medicament is for the treatment, prevention or suppression of minimal residual tumor disease.
